(19) Europäisches Patentamt
European Patent Office
Office européen des brevets

(11) EP 1 032 424 B1

(12) # EUROPEAN PATENT SPECIFICATION

(45) Date of publication and mention of the grant of the patent:
**12.09.2001 Bulletin 2001/37**

(21) Application number: **98949193.1**

(22) Date of filing: **02.11.1998**

(51) Int Cl.[7]: **A61K 45/06**

(86) International application number:
**PCT/IB98/01752**

(87) International publication number:
**WO 99/26659 (03.06.1999 Gazette 1999/22)**

(54) **COMBINATION OF AN ALDOSE REDUCTASE INHIBITOR AND A GLYCOGEN PHOSPHORYLASE INHIBITOR**

ZUSAMMENSETZUNGEN WELCHE ALDOSEREDUCTASEHEMMER UND GLYCOGEN PHOSPHORYLASE HEMMER ENTHALTEN

COMBINAISON D'UN INHIBITEUR DE REDUCTASE D'ALDOSE ET D'UN INHIBITEUR DE PHOSPHORYLASE DE GLYCOGENE

(84) Designated Contracting States:
**AT BE CH CY DE DK ES FI FR GB GR IE IT LI LU NL PT SE**
Designated Extension States:
**AL LT LV MK RO SI**

(30) Priority: **21.11.1997 US 66365 P**

(43) Date of publication of application:
**06.09.2000 Bulletin 2000/36**

(73) Proprietor: **Pfizer Products Inc.**
**Groton, Connecticut 06340 (US)**

(72) Inventors:
- **MYLARI, Banavara, Lakshman**
**Waterford, CT 06385 (US)**
- **HOOVER, Dennis, Jay**
**Groton, CT 06340 (US)**
- **HULIN, Bernard**
**Essex, CT 06426 (US)**
- **TREADWAY, Judith, Lee**
**Groton, CT 06340 (US)**

(74) Representative: **Hayles, James Richard**
**Pfizer Limited,**
**Patents Department,**
**Ramsgate Road**
**Sandwich Kent CT13 9NJ (GB)**

(56) References cited:
**EP-A- 0 624 369**
**WO-A-96/39384**
**WO-A-96/39385**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 032 424 B1

**Description**

BACKGROUND OF INVENTION

This invention relates to pharmaceutical combination of an aldose reductase inhibitor and a glycogen phosphorylase inhibitor, kits containing such combinations and the use of such combinations to treat diabetes, hyperglycemia, hypercholesterolemia, hypertension, hyperinsulinemia, hyperlipidemia, atherosclerosis and tissue ischemia in mammals.

In spite of the early discovery of insulin and its subsequent widespread use in the treatment of diabetes, and the later discovery of and use of sulfonylureas (e.g. Chlorpropamide™ (Pfizer), Tolbutamide™ (Upjohn), Acetohexamide™ (E.I. Lilly), Tolazamide™ (Upjohn)), biguanides (e.g. Phenformin™ (Ciba Geigy), Metformin™ (G. D. Searle)), alpha-glucosidase inhibitors (e.g., Precose™ (Bayer)) and insulin sensitizers (e.g., Rezulin™ (Parke Davis)) as oral hypoglycemic agents, there is a continuing need for treatments of diabetes. The use of insulin, necessary in about 10 % of diabetic patients in which synthetic hypoglycemic agents are not effective (Type I diabetes, insulin dependent diabetes mellitus), requires multiple daily doses, usually by self injection. Determination of the proper dosage of insulin requires frequent estimations of the sugar in urine or blood. The administration of an excess dose of insulin causes hypoglycemia, with effects ranging from mild abnormalities in blood glucose to coma, or ever death. Treatment of non-insulin dependent diabetes mellitus (Type II diabetes, NIDDM) usually consists of a combination of diet, exercise, oral agents, e.g. sulfonylureas, and in more severe cases, insulin. However, the clinically available hypoglycemics can have other side effects which limit their use. In any event, where one of these agents may fail in an individual case, another may succeed. A continuing need for hypoglycemic agents, which may have fewer side effects or succeed where others fail, is clearly evident

Aldose reductase inhibitors constitute a class of compounds which have become widely known for their utility in preventing and treating conditions arising from complications of diabetes such as diabetic neuropathy and nephropathy. Such compounds are well known to those skilled in the art and are readily identified by standard biological tests.

For example, the compound zopolrestat, 1-phthalazineacetic acid, 3,4-dihydro-4-oxo-3-[[5-(trifluoromethyl)-2-benzothiazolyl]methyl]-, is known, for example from commonly assigned U.S. patent 4,939,140 to Larson et al., together with a number of compounds related thereto, to have utility as aldose reductase inhibitors. Zopolrestat has the structure

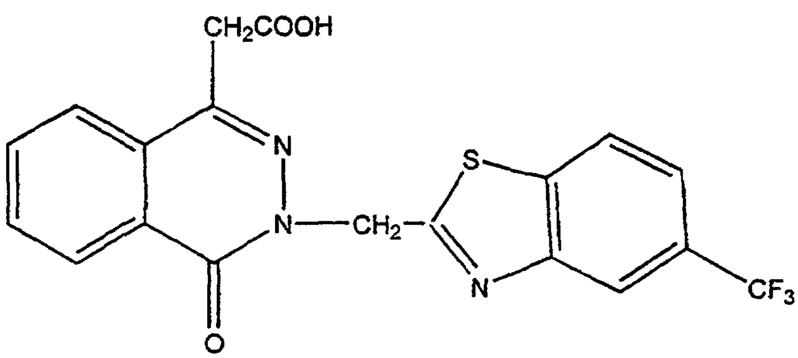

and, as an aldose reductase inhibitor, is useful in the treatment of the above-mentioned complications arising from diabetes mellitus.

Certain aldose reductase inhibitors have been taught for use in lowering lipid levels in mammals. See, for example, U. S. patent 4,492,706 (the disclosure of which is hereby incorporated by reference) to Kallai-sanfacon and EP 0 310 931 A2 (Ethyl Corporation).

Commonly assigned U. S. patent 5,064,830 (the disclosure of which is hereby incorporated by reference) to Going discloses the use of chain oxophthalazinyl acetic acids, including zopolrestat, for lowering of blood uric acid levels.

Commonly assigned U.S. patent application No. 08/059,688 discloses the use of certain aldose reductase inhibitors, including zopolrestat, for lowering blood lipid levels in humans. The disclosure notes that therapeutic utilities derive from the treatment of diseases caused by an increased level of triglycerides in the blood, such diseases include cardiovascular disorders such as thrombosis, arteriosclerosis, myocardial infarction, and angina pectoris.

Atherosclerosis, a disease of the arteries, is recognized to be the leading cause of death in the United States and Western Europe. The pathological sequence leading to atherosclerosis and occlusive heart disease is well known. The earliest stage in this sequence is the formation of "fatty streaks" in the carotid, coronary and cerebral arteries and

in the aorta. These lesions are yellow in color due to the presence of lipid deposits found principally within smooth-muscle cells and in macrophages of the intima layer of the arteries and aorta. Further, it is postulated that most of the cholesterol found within the fatty streaks, in turn, give rise to development of the "fibrous plaque", which consists of accumulated intimal smooth muscle cells laden with lipid and surrounded by extra-cellular lipid, collagen, elastin and proteoglycans. The cells plus matrix form a fibrous cap that covers a deeper deposit of cell debris and more extra cellular lipid. The lipid is primarily free and esterified cholesterol. The fibrous plaque forms slowly, and is likely in time to become calcified and necrotic, advancing to the "complicated lesion" which accounts for the arterial occlusion and tendency toward mural thrombosis and arterial muscle spasm that characterize advanced atherosclerosis.

Epidemiological evidence has firmly established hyperlipidemia as a primary risk factor in causing cardiovascular disease (CVD) due to atherosclerosis. In recent years, leaders of the medical profession have placed renewed emphasis on lowering plasma cholesterol levels, and low density lipoprotein cholesterol in particular, as an essential step in prevention of CVD. The upper limits of "normal" are now known to be significantly lower than heretofore appreciated. As a result, large segments of Western populations are now realized to be at particular high risk. Such independent risk factors include glucose intolerance, left ventricular hypertrophy, hypertension, and being of the male sex. Cardiovascular disease is especially prevalent among diabetic subjects, at least in part because of the existence of multiple independent risk factors in this population. Successful treatment of hyperlipidemia in the general population, and in diabetic subjects in particular, is therefore of exceptional medical importance.

Hypertension (or high blood pressure) is a condition which occurs in the human population as a secondary symptom to various other disorders such as renal artery stenosis, pheochromocytoma or endocrine disorders. However, hypertension is also evidenced in many patients in whom the causative agent or disorder is unknown. While such "essential" hypertension is often associated with disorders such as obesity, diabetes and hypertriglyceridemia, the relationship between these disorders has not been elucidated. Additionally, many patients display the symptoms of high blood pressure in the complete absence of any other signs of disease or disorder.

It is known that hypertension can directly lead to heart failure, renal failure and stroke (brain hemorrhaging). These conditions are capable of causing short-term death in a patient Hypertension can also contribute to the development of atherosclerosis and coronary disease. These conditions gradually weaken a patient and can led to long-term death.

The exact cause of essential hypertension is unknown, though a number of factors are believed to contribute to the onset of the disease. Among such factors are stress, uncontrolled emotions, unregulated hormone release (the renin, angiotensin, aldosterone system), excessive salt and water due to kidney malfunction, wall thickening and hypertrophy of the vasculature resulting in constricted blood vessels and genetic factors.

The treatment of essential hypertension has been undertaken bearing the foregoing factors in mind. Thus, a broad range of beta-blockers, vasoconstrictors, angiotensin converting enzyme inhibitors and the like have been developed and marketed as antihypertensives. The treatment of hypertension utilizing these compounds has proven beneficial in the prevention of short-interval deaths such as heart failure, renal failure and brain hemorrhaging. However, the development of atherosclerosis or heart disease due to hypertension over a long period of time remains a problem. This implies that although high blood pressure is being reduced, the underlying cause of essential hypertension is not responding to this treatment

Hypertension has been associated with elevated blood insulin levels, a condition known as hyperinsulinemia. Insulin, a peptide hormone whose primary actions are to promote glucose utilization, protein synthesis and the formation and storage of neutral lipids, also acts to promote vascular cell growth and increase renal sodium retention, among other things. These latter functions can be accomplished without affecting glucose levels and are known causes of hypertension. Peripheral vasculature growth, for example, can cause constriction of peripheral capillaries; while sodium retention increases blood volume. Thus, the lowering of insulin levels in hyperinsulinemics can prevent abnormal vascular growth and renal sodium retention caused by high insulin levels and thereby alleviate hypertension.

Cardiac hypertrophy is a significant risk factor in the development of sudden death, myocardial infarction, and congestive heart failure. These cardiac events are due, at least in part, to increased susceptibility to myocardial injury after ischemia and reperfusion which can occur in out-patient as well as perioperative settings. There is an unmet medical need to prevent or minimize adverse myocardial perioperative outcomes, particularly perioperative myocardial infarction. Both non-cardiac and cardiac surgery are associated with substantial risks for myocardial infarction or death. Some 7 million patients undergoing non-cardiac surgery are considered to be at risk, with incidences of perioperative death and serious cardiac complications as high as 20-25% in some series. In addition, of the 400,000 patients undergoing coronary by-pass surgery annually, perioperative myocardial infarction is estimated to occur in 5% and death in 1-2%. There is currently no marketed drug therapy in this area which reduces damage to cardiac tissue from perioperative myocardial ischemia or enhances cardiac resistance to ischemic episodes. Such a therapy is anticipated to be life-saving and reduce hospitalizations, enhance quality of life and reduce overall health care costs of high risk patients.

Hepatic glucose production is an important target for NIDDM therapy. The liver is the major regulator of plasma

glucose levels in the post absorptive (fasted) state, and the rate of hepatic glucose production in NIDDM patients is significantly elevated relative to normal individuals. Likewise, in the postprandial (fed) state, where the liver has a proportionately smaller role in the total plasma glucose supply, hepatin glucose production is abnormally high in NIDDM patients.

Glycogenolysis is an important target for interruption of hepatic glucose production. The liver produces glucose by glycogenolysis (breakdown of the glucose polymer glycogen) and gluconeogenesis (synthesis of glucose from 2- and 3-carbon precursors). Several lines of evidence indicate that glycogenolysis may make an important contribution to hepatic glucose output in NIDDM. First, in normal post absorptive humans, up to 75% of hepatic glucose production is estimated to result from glycogenolysis. Second, patients having liver glycogen storage diseases, including Hers' disease (glycogen phosphorylase deficiency), display episodic hypoglycemia. These observations suggest that glycogenolysis may be a significant process for hepatic glucose production.

Glycogenolysis is catalyzed in liver, muscle, and brain by tissue-specific isoforms of the enzyme glycogen phosphorylase. This enzyme cleaves the glycogen macromolecule to release glucose-1-phosphate and a new shortened glycogen macromolecule. Two types of glycogen phosphorylase inhibitors have been reported to date: glucose and glucose analogs [Martin, J.L. et al. *Biochemistry* 1991, *30*, 10101] and caffeine and other purine analogs [Kasvinsky, P.J. et al. *J. Biol. Chem.* 1978, 253, 3343-3351 and 9102-9106]. These compounds, and glycogen phosphorylase inhibitors in general, have been postulated to be of potential use for the treatment of NIDDM by decreasing hepatic glucose production and lowering glycemia. [Blundell, T.B. et al. *Diabetologia* 1992, 35, Suppl. 2, 569-576 and Martin et al. Biochemistry 1991, 30, 10101].

The mechanism(s) responsible for the myocardial injury observed after ischemia and reperfusion is not fully understood. It has been reported (M. F. Allard, et al. Am. J. Physiol. 267, H66-H74, 1994) that "pre ischemic glycogen reduction...is associated with improved post ischemic left ventricular functional recovery in hypertrophied rat hearts".

Thus, although there are a variety of hyperglycemia, hypercholesterolemia, hypertension, hyperinsulinemia, hyperlipidemia, atherosclerosis and ischemic therapies there is a continuing need and a continuing search in this field of art for alternative therapies.

## SUMMARY OF THE INVENTION

This invention is directed to pharmaceutical compositions comprising aldose reductase inhibitors and glycogen phosphorylase inhibitors and to the use of such compositions in the manufacture of a medicament for the treatment of insulin resistant conditions, including diabetes in mammals (e.g., humans either male or female) or to the use of such compositions in the manufacture of a medicament for reducing tissue damage (e.g., substantially preventing tissue damage, inducing tissue protection) resulting from ischemia.

The combinations comprise therapeutically effective amounts of an aldose reductase inhibitor and a glycogen phosphorylase inhibitor.

A preferred amount of aldose reductase inhibitor is about 0.1 mg/kg to about 20 mg/kg and a preferred amount of glycogen phosphorylase inhibitor is about 0.1 mg/kg to about 15 mg/kg.

An especially preferred aldose reductase inhibitor is 1-phthalazineacetic acid, 3,4-dihydro-4-oxo-3-[[5-trifluoromethyl)-2-benzothiazolyl]methyl]-.

Preferred glycogen phosphorylase inhibitors include compounds having the Formula I

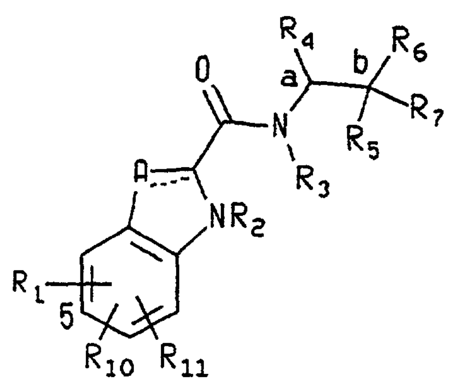

Formula I

and the pharmaceutically acceptable salts and prodrugs thereof wherein

the dotted line (---) is an optional bond;
A is -C(H)=, -C(($C_1$-$C_4$)alkyl)= or -C(halo)= when the dotted line (---) is a bond, or A is methylene or -CH(($C_1$-$C_4$) alkyl)- when the dotted line (---) is not a bond;
$R_1$, $R_{10}$ or $R_{11}$ are each independently H, halo, 4-, 6- or 7-nitro, cyano, ($C_1$-$C_4$)alkyl, ($C_1$-$C_4$)alkoxy, fluoromethyl, difluoromethyl or trifluoromethyl;
$R_2$ is H;
$R_3$ is H or ($C_1$-$C_5$)alkyl;
$R_4$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, phenyl($C_1$-$C_4$)alkoxy($C_1$-$C_4$)alkyl, thien-2- or -3-yl($C_1$-$C_4$)alkyl or fur-2- or -3-yl($C_1$-$C_4$)alkyl wherein said $R_4$ rings are mono-, di- or tri-substituted independently on carbon with H, halo, ($C_1$-$C_4$)alkyl, ($C_1$-$C_4$) alkoxy, trifluoromethyl, hydroxy, amino or cyano; or
$R_4$ is pyrid-2-, -3- or -4-yl($C_1$-$C_4$)alkyl, thiazol-2-, -4- or -5-yl($C_1$-$C_4$)alkyl, imidazol -1-, -2-, -4- or -5-yl($C_1$-$C_4$)alkyl, pyrrol-2- or -3-yl($C_1$-$C_4$)alkyl, oxazol-2-, -4- or -5-yl-($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl-($C_1$-$C_4$)alkyl, pyrimidin-2-, -4-, -5- or -6-yl($C_1$-$C_4$)alkyl, pyrazin-2- or -3-yl($C_1$-$C_4$)alkyl or 1,3,5-triazin-2-yl($C_1$-$C_4$)alkyl, wherein said preceding $R_4$ heterocycles are optionally mono-or di-substituted independently with halo, trifluoromethyl, ($C_1$-$C_4$)alkyl, ($C_1$-$C_4$) alkoxy, amino or hydroxy and said mono-or di-substituents are bonded to carbon;
$R_5$ is H, hydroxy, fluoro, ($C_1$-$C_5$)alkyl, ($C_1$-$C_5$)alkoxy, ($C_1$-$C_6$)alkanoyl, amino($C_1$-$C_4$)alkoxy, mono-N- or di-N,N-($C_1$-$C_4$)alkylamino($C_1$-$C_4$)alkoxy, carboxy($C_1$-$C_4$)alkoxy, ($C_1$-$C_5$)alkoxy-carbonyl($C_1$-$C_4$)alkoxy, benzyloxycarbonyl($C_1$-$C_4$)alkoxy, or carbonyloxy wherein said carbonyloxy is carbon-carbon linked with phenyl, thiazolyl, imidazolyl, 1H-indolyl, furyl, pyrrolyl, oxazolyl, pyrazolyl, isoxazolyl, isothiazolyl, pyridazinyl, pyrimidinyl, pyrazinyl or 1,3,5-triazinyl and wherein said preceding $R_5$ rings are optionally mono-substituted with halo, ($C_1$-$C_4$)alkyl, ($C_1$-$C_4$)alkoxy, hydroxy, amino or trifluoromethyl and said mono-substituents are bonded to carbon;
$R_7$ is H, fluoro or ($C_1$-$C_5$)alkyl; or
$R_5$ and $R_7$ can be taken together to be oxo;
$R_6$ is carboxy, ($C_1$-$C_8$)alkoxycarbonyl, C(O)N$R_8$$R_9$ or C(O)$R_{12}$,
wherein
$R_8$ is H, ($C_1$-$C_3$)alkyl, hydroxy or ($C_1$-$C_3$)alkoxy; and
$R_9$ is H, ($C_1$-$C_8$)alkyl, hydroxy, ($C_1$-$C_8$)alkoxy, methylene-perfluorinated($C_1$-$C_8$)alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding $R_9$ rings are carbon-nitrogen linked; or
$R_9$ is mono-, di- or tri-substituted ($C_1$-$C_5$)alkyl, wherein said substituents are independently H, hydroxy, amino, mono-N- or di-N,N-($C_1$-$C_5$)alkylamino; or
$R_9$ is mono- or di-substituted ($C_1$-$C_5$)alkyl, wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl or 1,3,5-triazinyl
wherein the nonaromatic nitrogen-containing $R_9$ rings are optionally mono-substituted on nitrogen with ($C_1$-$C_6$) alkyl, benzyl, benzoyl or ($C_1$-$C_6$)alkoxycarbonyl and wherein the $R_9$ rings are optionally mono-substituted on carbon with halo, ($C_1$-$C_4$)alkyl, ($C_1$-$C_4$)alkoxy, hydroxy, amino, or mono-N- and di-N,N ($C_1$-$C_5$)alkylamino provided that no quatemized nitrogen is included and there are no nitrogen-oxygen, nitrogen-nitrogen or nitrogen-halo bonds;
$R_{12}$ is piperazin-1-yl, 4-($C_1$-$C_4$)alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxo-thiazolidin-3-yl, 1,1-dioxo-thiazolidin-3-yl, 2-($C_1$-$C_6$) alkoxycarbonylpyrrolidin-1-yl, oxazolidin-3-yl or 2(R)-hydroxymethylpyrrolidin-1-yl; or
$R_{12}$ is 3- and/or 4-mono-or di-substituted oxazetidin-2-yl, 2-, 4-, and/or 5-mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di- substituted 1,1-dioxothiazolidin-3-yl, 3- and/or 4-, mono- or di-substituted pyrrolidin-1-yl, 3-, 4- and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4-and/or 5-, mono- and/ or di-substituted isothiazolidin-2-yl wherein said $R_{12}$ substituents are independently H, halo, ($C_1$-$C_5$)-alkyl, hydroxy, amino, mono-N- or di-N,N-($C_1$-$C_5$)alkylamino, formyl, oxo, hydroxyimino, ($C_1$-$C_5$)alkoxy, carboxy, carbamoyl, mono-N-or di-N,N-($C_1$-$C_4$)alkylcarbamoyl, ($C_1$-$C_4$)alkoxyimino, ($C_1$-$C_4$)alkoxymethoxy, ($C_1$-$C_6$)alkoxycarbonyl, carboxy($C_1$-$C_5$)alkyl or hydroxy($C_1$-$C_5$)alkyl;
with the proviso that if $R_4$ is H, methyl, ethyl or n-propyl $R_5$ is OH;
with the proviso that if $R_5$ and $R_7$ are H, then $R_4$ is not H, methyl, ethyl, n-propyl, hydroxy($C_1$-$C_3$)alkyl or ($C_1$-$C_3$)

alkoxy($C_1$-$C_3$)alkyl and $R_6$ is C(O)$NR_8R_9$, C(O)$R_{12}$ or ($C_1$-$C_4$)alkoxycarbonyl.

A first group of preferred compounds of Formula I consists of those compounds wherein

$R_1$ is 5-H, 5-halo, 5-methyl or 5-cyano;
$R_{10}$ and $R_{11}$ are each independently H or halo;
A is -C(H)=;
$R_2$ and $R_3$ are H;
$R_4$ is phenyl($C_1$-$C_2$)alkyl wherein said phenyl groups are mono-, di- or tri-substituted independently with H or halo or mono- or di- substituted independently with H, halo, ($C_1$-$C_4$)alkyl, ($C_1$-$C_4$)alkoxy, trifluoromethyl, hydroxy, amino or cyano; or
$R_4$ is thien-2- or -3-yl($C_1$-$C_2$)alkyl, pyrid-2-, -3- or -4-yl($C_1$-$C_2$)alkyl, thiazol-2-,-4- or -5-yl($C_1$-$C_2$)alkyl, imidazol -1-, -2-, -4- or -5-yl($C_1$-$C_2$)alkyl, fur-2- or -3-yl($C_1$-$C_2$)alkyl, pyrrol-2- or -3-yl($C_1$-$C_2$)alkyl, oxazol-2-, -4- or -5-yl-($C_1$-$C_2$) alkyl, pyrazol-3-,-4- or -5-yl($C_1$-$C_2$)alkyl, isoxazol-3-, -4- or -5-yl($C_1$-$C_2$)alkyl wherein said preceding $R_4$ heterocycles are optionally mono- or di-substituted independently with halo, trifluoromethyl, ($C_1$-$C_4$)alkyl, ($C_1$-$C_4$)alkoxy, amino or hydroxy and said mono- or di-substituents are bonded to carbon;
$R_5$ is hydroxy;
$R_6$ is C(O)$NR_8R_9$ or C(O)$R_{12}$; and
$R_7$ is H.

Within the above first group of preferred compounds of Formula I is a first group of especially preferred compounds wherein

the carbon atom a has (S) stereochemistry;
the carbon atom b has (R) stereochemistry;
$R_4$ is phenyl($C_1$-$C_2$)alkyl, thien-2-yl-($C_1$-$C_2$)alkyl, thien-3-yl-($C_1$-$C_2$)alkyl, fur-2-yl-($C_1$-$C_2$)alkyl or fur-3-yl-($C_1$-$C_2$) alkyl wherein said rings are mono- or di- substituted independently with H or fluoro;
$R_6$ is C(O)$NR_8R_9$;
$R_8$ is ($C_1$-$C_3$)alkyl, hydroxy or ($C_1$-$C_3$)alkoxy; and
$R_9$ is H, ($C_1$-$C_8$)alkyl, hydroxy, hydroxy($C_1$-$C_6$)alkyl, ($C_1$-$C_8$)alkoxy, pyridyl, morpholinyl, piperazinyl, pyrrolidinyl, piperidinyl, imidazolyl or thiazolyl or ($C_1$-$C_4$)alkyl mono-substituted with pyridyl, morpholinyl, piperazinyl, pyrrolidinyl, piperidinyl, imidazolyl or thiazolyl.

Within the above first group of especially preferred compounds are the particularly preferred compounds

5-Chloro-1H-indole-2-carboxylic acid [(1S)-((R)-hydroxy-dimethylcarbamoylmethyl)-2-phenyl-ethyl]-amide,
5,6-Dichloro-1H-indole-2-carboxylic acid {(1S)-[(R)-hydroxy-(methoxy-methylcarbamoyl)-methyl]-2-phenyl-ethyl}-amide,
5-Chloro-1H-indole-2-carboxylic acid {(1S)-[(R)-hydroxy-(methoxy-methylcarbamoyl)-methyl]-2-phenyl-ethyl}-amide,
5-Chloro-1H-indole-2-carboxylic acid ((1S)-{(R)-hydroxy-[(2-hydroxy-ethyl)-methyl-carbamoyl]-methyl}-2-phenyl-ethyl)-amide,
5-Chloro-1 H-indole-2-carboxylic acid {(1S)-[(R)-hydroxy-(methyl-pyridin-2-yl-carbamoyl)-methyl]-2-phenyl-ethyl}-amide or
5-Chloro-1H-indole-2-carboxylic acid ((1S)-{(R)-hydroxy-[methyl-(2-pyridin-2-yl-ethyl)-carbamoyl]-methyl}-2-phenyl-ethyl)-amide.

Within the above first group of especially preferred compounds are the compounds wherein

a.

$R_1$ is 5-chloro;
$R_{10}$ and $R_{11}$ are H;
$R_4$ is benzyl;
$R_8$ is methyl; and
$R_9$ is methyl;

b.

$R_1$ is 5-chloro;
$R_{11}$ is H;
$R_{10}$ is 6-chloro;
$R_4$ is benzyl;
$R_8$ is methyl; and
$R_9$ is methoxy;

C.

$R_1$ is 5-chloro;
$R_{10}$ and $R_{11}$ are H;
$R_4$ is benzyl;
$R_8$ is methyl; and
$R_9$ is methoxy;

d.

$R_1$ is 5-chloro;
$R_{10}$ and $R_{11}$ are H;
$R_4$ is benzyl;
$R_8$ is methyl; and
$R_9$ is 2-(hydroxy)ethyl;

e.

$R_1$ is 5-chloro;
$R_{10}$ and $R_{11}$ are H;
$R_4$ is benzyl;
$R_8$ is methyl; and
$R_9$ is pyridin-2-yl; and

f.

$R_1$ is 5-chloro;
$R_{10}$ and $R_{11}$ are H;
$R_4$ is benzyl;
$R_8$ is methyl; and
$R_9$ is 2-(pyridin-2-yl)ethyl.

Within the above first group of preferred compounds of Formula I is a second group of especially preferred compounds wherein

the carbon atom a is (S) stereochemistry;
the carbon atom b is (R) stereochemistry;
$R_4$ is phenyl($C_1$-$C_2$)alkyl, thien-2-yl-($C_1$-$C_2$)alkyl, thien-3-yl-($C_1$-$C_2$)alkyl, fur-2-yl-($C_1$-$C_2$)alkyl or fur-3-yl-($C_1$-$C_2$)alkyl wherein said rings are mono- or di- substituted independently with H or fluoro;
$R_6$ is C(O)$R_{12}$; and
$R_{12}$ is morpholino, 4-($C_1$-$C_4$)alkylpiperazin-1-yl, 3-substituted azetidin-1-yl, 3-and/or 4-, mono- or di-substituted pyrrolidin-1-yl, 4- and/or 5- mono- or di-substituted isoxazolidin-2-yl, 4- and/or 5-, mono- or di-substituted 1,2-oxazinan-2-yl wherein said substituents are each independently H, halo, hydroxy, amino, mono-N- or di-N,N-($C_1$-$C_6$)alkylamino, oxo, hydroxyimino or alkoxy.

Within the above second group of especially preferred compounds are the particularly preferred compounds

5-Chloro-1 H-indole-2-carboxylic acid [(1 S)-benzyl-(2R)-hydroxy-3-(4-methylpiperazin-1-yl)-3-oxo-propyl]-amide hydrochloride,
5-Chloro-1H-indole-2-carboxylic acid [(1S)-benzyl-(2R)-hydroxy-3-(3-hydroxyazetidin-1-yl)-3-oxo-propyl]-amide,
5-Chloro-1H-indole-2-carboxylic acid ((1S)-benzyl-(2R)-hydroxy-3-isoxazolidin-2-yl-3-oxo-propyl)-amide,

5-Chloro-1H-indole-2-carboxylic add ((1S)-benzyl-(2R)-hydroxy-3-[1,2]oxazinan-2-yl-3-oxo-propyl)-amide,
5-Chloro-1H-indole-2-carboxylic acid [(1S)-benzyl-(2R)-hydroxy-3-((3S)-hydroxy-pyrrolidin-1-yl)-3-oxo-propyl]-amide,
5-Chloro-1H-indole-2-carboxylic acid [(1S)-benzyl-3-((3S,4S)-dihydroxy-pyrrolidin-1-yl)-(2R)-hydroxy-3-oxo-propyl]-amide,
5-Chloro-1H-indole-2-carboxylic acid [(1S)-benzyl-3-((3R,4S)-dihydroxypyrrolidin-1-yl)-(2R)-hydroxy-3-oxo-propyl]-amide or
5-Chloro-1 H-indole-2-carboxylic acid ((1S)-benzyl-(2R)-hydroxy-3-morpholin-4-yl-3-oxo-propyl)-amide.

Within the above second group of especially preferred compounds are the compounds wherein

a.

$R_1$ is 5-chloro;
$R_{10}$ and $R_{11}$ are H;
$R_4$ is benzyl; and
$R_{12}$ is 4-methylpiperazin-1-yl;

b.

$R_1$ is 5-chloro;
$R_{10}$ and $R_{11}$ are H;
$R_4$ is benzyl; and
$R_{12}$ is 3-hydroxyazetidin-1-yl;

C.

$R_1$ is 5-chloro;
$R_{10}$ and $R_{11}$ are H;
$R_4$ is benzyl; and
$R_{12}$ is isoxazolidin-2-yl;

d.

$R_1$ is 5-chloro;
$R_{10}$ and $R_{11}$ are H;
$R_4$ is benzyl; and
$R_{12}$ is (1,2)-oxazinan-2-yl;

e.

$R_1$ is 5-chloro;
$R_{10}$ and $R_{11}$ are H;
$R_4$ is benzyl; and
$R_{12}$ is 3(S)-hydroxypyrrolidin-1-yl;

f.

$R_1$ is 5-chloro;
$R_{10}$ and $R_{11}$ are H;
$R_4$ is benzyl; and
$R_{12}$ is (3S,4S)-dihydroxypyrrolidin-1-yl;

g.

$R_1$ is 5-chloro;
$R_{10}$ and $R_{11}$ are H;
$R_4$ is benzyl; and

$R_{12}$ is (3R,4S)-dihydroxypyrrolidin-1-yl; and

h.

$R_1$ is 5-chloro;
$R_{10}$ and $R_{11}$ are H;
$R_4$ is benzyl; and
$R_{12}$ is morpholino.

A second group of preferred compounds of Formula I consists of those compounds wherein

$R_1$ is H, halo, methyl or cyano;
$R_{10}$ and $R_{11}$ are each independently H or halo;
A is -C(H)=;
$R_2$ and $R_3$ are H;
$R_4$ is phenyl($C_1$-$C_2$)alkyl wherein said phenyl groups are mono-, di- or tri-substituted independently with H or halo or mono- or di- substituted independently with H, halo, ($C_1$-$C_4$)alkyl, ($C_1$-$C_4$)alkoxy, trifluoromethyl, hydroxy, amino or cyano; or
$R_4$ is thien-2- or -3-yl($C_1$-$C_2$)alkyl, pyrid-2-, -3- or -4-yl($C_1$-$C_2$)alkyl, thiazol-2-,-4- or -5-yl($C_1$-$C_2$)alkyl, imidazol -1-, -2-, -4- or -5-yl($C_1$-$C_2$)alkyl, fur-2- or -3-yl($C_1$-$C_2$)alkyl, pyrrol-2- or -3-yl($C_1$-$C_2$)alkyl, oxazol-2-, -4- or -5-yl-($C_1$-$C_2$) alkyl, pyrazol-3-,-4- or -5-yl($C_1$-$C_2$)alkyl, isoxazol-3-, -4- or -5-yl($C_1$-$C_2$)alkyl wherein said preceding $R_4$ heterocyctes are optionally mono- or di-substituted independently with halo, trifluoromethyl, ($C_1$-$C_4$)alkyl, ($C_1$-$C_4$)alkoxy, amino or hydroxy and said mono- or di-substituents are bonded to carbon;
$R_5$ is hydroxy;
$R_6$ is carboxy or ($C_1$-$C_8$)alkoxycarbonyl; and
$R_7$ is H, fluoro or ($C_1$-$C_6$)alkyl.

Within the second group of preferred compounds of Formula I is a group of especially preferred compounds wherein

the carbon atom a is (S) stereochemistry;
the carbon atom b is (R) stereochemistry;
$R_4$ is phenyl($C_1$-$C_2$)alkyl thien-2-yl-($C_1$-$C_2$)alkyl, thien-3-yl-($C_1$-$C_2$)alkyl, fur-2-yl-($C_1$-$C_2$)alkyl or fur-3-yl-($C_1$-$C_2$) alkyl wherein said rings are mono- or di- substituted independently with H or fluoro;
$R_{10}$ and $R_{11}$ are H;
$R_6$ is carboxy; and
$R_7$ is H.

Preferred within the immediately preceding group is a compound wherein

$R_1$ is 5-chloro;
$R_{10}$ and $R_{11}$ are H; and
$R_4$ is benzyl.

A third group of preferred compounds of Formula I consists of those compounds wherein

$R_1$ is H, halo, methyl or cyano;
$R_{10}$ and $R_{11}$ are each independently H or halo;
A is -C(H)=;
$R_2$ and $R_3$ are H;
$R_4$ is phenyl($C_1$-$C_2$)alkyl wherein said phenyl groups are mono-, di- or tri-substituted independently with H or halo or mono- or di- substituted independently with H, halo, ($C_1$-$C_4$)alkyl, ($C_1$-$C_4$)alkoxy, trifluoromethyl, hydroxy, amino or cyano; or
$R_4$ is thien-2- or -3-yl($C_1$-$C_2$)alkyl, pyrid-2-, -3- or -4-yl($C_1$-$C_2$)alkyl, thiazol-2-,-4- or -5-yl($C_1$-$C_2$)alkyl, imidazol -1-, -2-, -4- or -5-yl($C_1$-$C_2$)alkyl, fur-2- or -3-yl($C_1$-$C_2$)alkyl, pyrrol-2- or -3-yl($C_1$-$C_2$)alkyl, oxazol-2-, -4- or -5-yl-($C_1$-$C_2$) alkyl, pyrazol-3-,-4- or -5-yl($C_1$-$C_2$)alkyl, isoxazol-3-, -4- or -5-yl($C_1$-$C_2$)alkyl wherein said preceding $R_4$ heterocycles are optionally mono- or di-substituted independently with halo, trifluoromethyl, ($C_1$-$C_4$)alkyl, ($C_1$-$C_4$)alkoxy, amino or hydroxy and said mono- or di-substituents are bonded to carbon;

$R_5$ is fluoro, $(C_1-C_4)$alkyl, $(C_1-C_5)$alkoxy, amino$(C_1-C_4)$alkoxy, mono-N- or di-N,N-$(C_1-C_4)$alkylamino$(C_1-C_4)$alkoxy, carboxy$(C_1-C_4)$alkoxy, $(C_1-C_5)$alkoxycarbonyl$(C_1-C_4)$alkoxy, benzyloxycarbonyl$(C_1-C_4)$alkoxy;
$R_6$ is carboxy or $(C_1-C_8)$alkoxycarbonyl; and
$R_7$ is H, fluoro or $(C_1-C_6)$alkyl.

A fourth group of preferred compounds of Formula I consists of those compounds wherein

$R_1$ is H, halo, methyl or cyano;
$R_{10}$ and $R_{11}$ are each independently H or halo;
A is -C(H)=;
$R_2$ and $R_3$ are H;
$R_4$ is phenyl$(C_1-C_2)$alkyl wherein said phenyl groups are mono-, di- or tri-substituted independently with H or halo or mono- or di- substituted independently with H, halo, $(C_1-C_4)$alkyl, $(C_1-C_4)$alkoxy, trifluoromethyl, hydroxy, amino or cyano; or
$R_4$ is thien-2- or -3-yl$(C_1-C_2)$alkyl, pyrid-2-, -3- or -4-yl$(C_1-C_2)$alkyl, thiazol-2-,-4- or -5-yl$(C_1-C_2)$alkyl, imidazol -1-, -2-, -4- or -5-yl$(C_1-C_2)$alkyl, fur-2- or -3-yl$(C_1-C_2)$alkyl, pyrrol-2- or -3-yl$(C_1-C_2)$alkyl, oxazol-2-, -4- or -5-yl-$(C_1-C_2)$alkyl, pyrazol-3-,-4- or -5-yl$(C_1-C_2)$alkyl, isoxazol-3-, -4- or -5-yl$(C_1-C_2)$alkyl wherein said preceding $R_4$ heterocycles are optionally mono- or di-substituted independently with halo, trifluoromethyl, $(C_1-C_4)$alkyl, $(C_1-C_4)$alkoxy, amino or hydroxy and said mono- or di-substituents are bonded to carbon;
$R_5$ is fluoro, $(C_1-C_4)$alkyl, $(C_1-C_5)$alkoxy, amino$(C_1-C_4)$alkoxy, mono-N- or di-N,N-$(C_1-C_4)$alkylamino$(C_1-C_4)$alkoxy, carboxy$(C_1-C_4)$alkoxy, $(C_1-C_5)$alkoxycarbonyl$(C_1-C_4)$alkoxy, benzyloxycarbonyl$(C_1-C_4)$alkoxy;
$R_6$ is $C(O)NR_8R_9$ or $C(O)R_{12}$; and
$R_7$ is H, fluoro or $(C_1-C_6)$alkyl.

Preferred glycogen phosphorylase inhibitors include compounds having the Formula IA

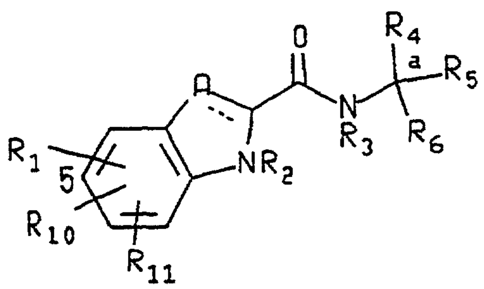

Formula IA

and the pharmaceutically acceptable salts and prodrugs thereof wherein

the dotted line (—) is an optional bond;
A is -C(H)=, -C($(C_1-C_4)$alkyl)=, -C(halo)= or -N=, when the dotted line (—) is a bond, or A is methylene or -CH($(C_1-C_4)$alkyl)-, when the dotted line (—) is not a bond;
$R_1$, $R_{10}$ or $R_{11}$ are each independently H, halo, cyano, 4-, 6-, or 7-nitro, $(C_1-C_4)$alkyl, $(C_1-C_4)$alkoxy, fluoromethyl, difluoromethyl or trifluoromethyl;
$R_2$ is H;
$R_3$ is H or $(C_1-C_5)$alkyl;
$R_4$ is H, methyl, ethyl, n-propyl, hydroxy$(C_1-C_3)$alkyl, $(C_1-C_3)$alkoxy$(C_1-C_3)$alkyl, phenyl$(C_1-C_4)$alkyl, phenylhydroxy$(C_1-C_4)$alkyl, (phenyl)($(C_1-C_4)$-alkoxy)$(C_1-C_4)$alkyl, thien-2- or -3-yl$(C_1-C_4)$alkyl or fur-2- or -3-yl$(C_1-C_4)$alkyl wherein said $R_4$ rings are mono-, di- or tri-substituted independently on carbon with H, halo, $(C_1-C_4)$alkyl, $(C_1-C_4)$alkoxy, trifluoromethyl, hydroxy, amino, cyano or 4,5-dihydro-1H-imidazol-2-yl; or
$R_4$ is pyrid-2-, -3- or -4-yl$(C_1-C_4)$alkyl, thiazol-2-, -4- or -5-yl$(C_1-C_4)$alkyl, imidazol-2-, -4- or -5-yl$(C_1-C_4)$alkyl, pyrrol-2- or -3-yl$(C_1-C_4)$alkyl, oxazol-2-, -4- or -5-yl$(C_1-C_4)$alkyl, pyrazol-3-, -4- or -5-yl$(C_1-C_4)$alkyl, isoxazol-3-, -4- or -5-yl$(C_1-C_4)$alkyl, isothiazol-3-, -4- or -5-yl$(C_1-C_4)$alkyl, pyridazin-3- or -4-yl$(C_1-C_4)$alkyl, pyrirnidin-2-, -4-, -5- or -6-yl$(C_1-C_4)$alkyl, pyrazin-2- or -3-yl$(C_1-C_4)$alkyl, 1,3,5-triazin-2-yl$(C_1-C_4)$alkyl or indol-2-$(C_1-C_4)$alkyl, wherein said preceding $R_4$ heterocycles are optionally mono-or di-substituted independently with halo, trifluoromethyl, $(C_1-C_4)$

alkyl, $(C_1-C_4)$alkoxy, amino, hydroxy or cyano and said substituents are bonded to carbon; or
$R_4$ is $R_{15}$-carbonyloxymethyl, wherein said $R_{15}$ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding $R_{15}$ rings are optionally mono- or di-substituted independently with halo, amino, hydroxy, $(C_1-C_4)$alkyl, $(C_1-C_4)$alkoxy or trifluoromethyl and said mono- or di-substituents are bonded to carbon;
$R_5$ is H;
$R_6$ is carboxy, $(C_1-C_8)$alkoxycarbonyl, benzyloxycarbonyl, $C(O)NR_8R_9$ or $C(O)R_{12}$

wherein

$R_8$ is H, $(C_1-C_6)$alkyl, cyclo$(C_3-C_6)$alkyl, cyclo$(C_3-C_6)$alkyl$(C_1-C_5)$alkyl, hydroxy or $(C_1-C_8)$alkoxy; and
$R_9$ is H, cyclo$(C_3-C_8)$alkyl, cyclo$(C_3-C_8)$alkyl$(C_1-C_5)$alkyl, cyclo$(C_4-C_7)$alkenyl, cyclo$(C_3-C_7)$alkyl$(C_1-C_5)$alkoxy, cyclo$(C_3-C_7)$alkyloxy, hydroxy, methylene-perfluorinated$(C_1-C_8)$alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked; or
$R_9$ is $(C_1-C_6)$alkyl or $(C_1-C_8)$alkoxy wherein said $(C_1-C_6)$alkyl or $(C_1-C_8)$alkoxy is optionally monosubstituted with cyclo$(C_4-C_7)$alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, thiomorpholinyl, 1-oxothiomorpholinyl, 1,1-dioxothiomorpholinyl, pyridazinyl, pyrimidinyl, pyrazinyl, piperazinyl, 1,3,5-triazinyl or indolyl and wherein said $(C_1-C_6)$alkyl or $(C_1-C_8)$alkoxy are optionally additionally independently mono- or di-substituted with halo, hydroxy, $(C_1-C_5)$alkoxy, amino, mono-N- or di-N,N-$(C_1-C_5)$alkylamino, cyano, carboxy, or $(C_1-C_4)$alkoxycarbonyl; and
wherein the $R_9$ rings are optionally mono- or di-substituted independently on carbon with halo, $(C_1-C_4)$alkyl, $(C_1-C_4)$alkoxy, hydroxy, hydroxy$(C_1-C_4)$alkyl, amino$(C_1-C_4)$alkyl, mono-N- or di-N,N-$(C_1-C_4)$alkylamino$(C_1-C_4)$alkyl, $(C_1-C_4)$alkoxy$(C_1-C_4)$alkyl, amino, mono-N- or di-N,N-$(C_1-C_4)$alkylamino, cyano, carboxy, $(C_1-C_5)$alkoxycarbonyl, carbamoyl, formyl or trifluoromethyl and said $R_9$ rings may optionally be additionally mono- or di-substituted independently with $(C_1-C_5)$alkyl or halo;

with the proviso that no quatemized nitrogen on any $R_9$ heterocycle is included;

$R_{12}$ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydrobenzo[d][1,2]oxazin-3-yl, 3,4-dihydro-benzo[e][1,2]-oxazin-2-yl, 3H-benzo[d]isoxazol-2-yl, 3H-benzo[c]isoxazol-1-yl or azepan-1-yl,
wherein said $R_{12}$ rings are optionally mono-, di- or tri-substituted independently with halo, $(C_1-C_5)$alkyl, $(C_1-C_5)$alkoxy, hydroxy, amino, mono-N- or di-N,N-$(C_1-C_5)$alkylamino, formyl, carboxy, carbamoyl, mono-N- or di-N,N-$(C_1-C_5)$alkylcarbamoyl, $(C_1-C_6)$alkoxy$(C_1-C_3)$alkoxy, $(C_1-C_5)$alkoxycarbonyl, benzyloxycarbonyl, $(C_1-C_5)$alkoxycarbonyl$(C_1-C_5)$alkyl, $(C_1-C_4)$alkoxycarbonylamino, carboxy$(C_1-C_5)$alkyl, carbamoyl$(C_1-C_5)$alkyl, mono-N- or di-N,N-$(C_1-C_5)$alkylcarbamoyl$(C_1-C_5)$alkyl, hydroxy$(C_1-C_5)$alkyl, $(C_1-C_4)$alkoxy$(C_1-C_4)$alkyl, amino$(C_1-C_4)$alkyl, mono-N- or di-N,N-$(C_1-C_4)$alkylamino$(C_1-C_4)$alkyl, oxo, hydroxyimino or $(C_1-C_6)$alkoxyimino and wherein no more than two substituents are selected from oxo, hydroxyimino or $(C_1-C_6)$alkoxyimino and oxo, hydroxyimino or $(C_1-C_6)$alkoxyimino are on nonaromatic carbon; and
wherein said $R_{12}$ rings are optionally additionally mono- or di-substituted independently with $(C_1-C_5)$alkyl or halo;
with the proviso that when $R_6$ is $(C_1-C_5)$alkoxycarbonyl or benzyloxycarbonyl then $R_1$ is 5-halo, 5-$(C_1-C_4)$alkyl or 5-cyano and $R_4$ is (phenyl)(hydroxy)$(C_1-C_4)$alkyl, (phenyl)($(C_1-C_4)$alkoxy)$(C_1-C_4)$alkyl, hydroxymethyl or Ar$(C_1-C_2)$alkyl, wherein Ar is thien-2- or -3-yl, fur-2- or -3-yl or phenyl wherein said Ar is optionally mono- or di-substituted independently with halo; with the provisos that when $R_4$ is benzyl and $R_5$ is methyl, $R_{12}$ is not 4-hydroxy-piperidin-1-yl or when $R_4$ is benzyl and $R_5$ is methyl $R_6$ is not $C(O)N(CH_3)_2$;
with the proviso that when $R_1$ and $R_{10}$ and $R_{11}$ are H, $R_4$ is not imidazol-4-ylmethyl, 2-phenylethyl or 2-hydroxy-2-phenylethyl;
with the proviso that when both $R_8$ and $R_9$ are n-pentyl, $R_1$ is 5-chloro, 5-bromo, 5-cyano, 5$(C_1-C_5)$alkyl, 5$(C_1-C_5)$alkoxy or trifluoromethyl;
with the proviso that when $R_{12}$ is 3,4-dihydroisoquinol-2-yl, said 3,4-dihydroisoquinol-2-yl is not substituted with

carboxy(($C_1$-$C_4$)alkyl;
with the proviso that when $R_8$ is H and $R_9$ is ($C_1$-$C_6$)alkyl, $R_9$ is not substituted with carboxy or ($C_1$-$C_4$)alkoxycarbonyl on the carbon which is attached to the nitrogen atom N of $NHR_9$; and
with the proviso that when $R_6$ is carboxy and $R_1$, $R_{10}$, $R_{11}$ and $R_5$ are all H, then $R_4$ is not benzyl, H, (phenyl)(hydroxy)methyl, methyl, ethyl or n-propyl.

A first group of preferred compounds of Formula IA consists of those compounds wherein

$R_1$ is 5-H, 5-halo, 5-methyl, 5-cyano or 5-trifluoromethyl;
$R_{10}$ and $R_{11}$ are each independently H or halo;
A is -C(H)=;
$R_2$ and $R_3$ are H;
$R_4$ is H, methyl, phenyl($C_1$-$C_2$)alkyl, wherein said phenyl groups are mono- or di-substituted independently with H, halo, ($C_1$-$C_4$)alkyl, ($C_1$-$C_4$)alkoxy, trifluoromethyl, hydroxy, amino or cyano and wherein said $R_4$ groups are optionally additionally mono-substituted with halo; or
$R_4$ is thien-2- or -3-yl($C_1$-$C_2$)alkyl, pyrid-2-, -3- or -4-yl($C_1$-$C_2$)alkyl, thiazol-2-,-4- or -5-yl($C_1$-$C_2$)alkyl, imidazol-2-, -4- or -5-yl($C_1$-$C_2$)alkyl, fur-2- or -3-yl($C_1$-$C_2$)alkyl, pyrrol-2- or -3-yl($C_1$-$C_2$)alkyl, oxazol-2-, -4- or -5-yl($C_1$-$C_2$)alkyl, pyrazol-3-, -4- or -5-yl($C_1$-$C_2$)alkyl, isoxazol-3-, -4- or -5-yl($C_1$-$C_2$)alkyl, isothiazol-3-, -4- or -5-yl($C_1$-$C_2$)alkyl, pyridazin-3- or -4-yl($C_1$-$C_2$)alkyl, pyrimidin-2-, -4-, -5- or -6-yl($C_1$-$C_2$)alkyl, pyrazin-2- or -3-yl($C_1$-$C_2$)alkyl or 1,3,5-triazin-2-yl($C_1$-$C_2$)alkyl wherein said preceding $R_4$ heterocycles are optionally mono- or di-substituted independently with halo, trifluoromethyl, ($C_1$-$C_4$)alkyl, ($C_1$-$C_4$)alkoxy, amino or hydroxy and said mono- or di-substituents are bonded to carbon;
$R_5$ is H; and
$R_6$ is C(O)$NR_8R_9$ or C(O)$R_{12}$.

Within the above first group of preferred compounds of Formula IA is a first group of especially preferred compounds wherein

$R_4$ is H, phenyl($C_1$-$C_2$)alkyl, thien-2- or -3-yl($C_1$-$C_2$)alkyl, fur-2- or -3-yl($C_1$-$C_2$)alkyl wherein said $R_4$ rings are mono- or di-substituted independently with H or fluoro;
$R_6$ is C(O)$R_{12}$; and
$R_{12}$ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, isoxazolidin-2-yl, isothiazolidin-2-yl, 1,2-oxazetidin-2-yl, oxazolidin-3-yl, 1,3-dihydroisoindol-2-yl, or azepan-1-yl,
wherein said $R_{12}$ rings are optionally mono- or di-substituted independently with halo, ($C_1$-$C_5$)alkyl, ($C_1$-$C_5$)alkoxy, hydroxy, amino, mono-N-or di-N,N-($C_1$-$C_5$)alkylamino, formyl, carboxy, carbamoyl, mono-N- or di-N,N-($C_1$-$C_5$)alkylcarbamoyl, ($C_1$-$C_5$)alkoxycarbonyl, hydroxy($C_1$-$C_5$)alkyl, amino($C_1$-$C_4$)alkyl, mono-N- or di-N,N-($C_1$-$C_4$)alkylamino($C_1$-$C_4$)alkyl, oxo, hydroxyimino or ($C_1$-$C_6$)alkoxyimino with the proviso that only the $R_{12}$ heterocycles thiazolidin-3-yl, pyrrolidin-1-yl, piperidin-1-yl, piperazin-1-yl, piperazin-4-yl, azetidin-1-yl, 1,2-oxazinan-2-yl, isoxazolidin-2-yl, or oxazolidin-3-yl are optionally mono- or di-substituted with oxo, hydroxyimino, or ($C_1$-$C_6$)alkoxyimino; and
wherein said $R_{12}$ rings are optionally additionally mono- or di-substituted independently with ($C_1$-$C_5$)alkyl.

Within the above group of especially preferred compounds are the compounds

5-Chloro-1H-indole-2-carboxylic acid [(1S)-benzyl-2-(3-hydroxyiminopyrrolidin-1-yl)-2-oxo-ethyl]-amide,
5-Chloro-1H-indole-2-carboxylic acid [2-(cis-3,4-dihydroxy-pyrrolidin-1-yl)-2-oxo-ethyl]-amide,
5-Chloro-1 H-indole-2-carboxylic acid [2-((3S,4S)-dihydroxy-pyrrolidin-1-yl)-2-oxo-ethyl]-amide,
5-Chloro-1H-indole-2-carboxylic acid [(1S)-benzyl-3-((cis)-dihydroxypyrrolidin-1-yl)-(2R)-hydroxy-3-oxopropyl]-amide,
5-Chloro-1H-indole-2-carboxylic acid [(1S)-benzyl-2-(cis-3,4-dihydroxy-pyrrolidin-1-yl)-2-oxo-ethyl]-amide,
5-Chloro-1H-indole-2-carboxylic acid [2-(1,1-dioxo-thiazolidin-3-yl)-2-oxo-ethyl]-amide,
5-Chloro-1H-indole-2-carboxylic acid (2-oxo-2-thiazolidin-3-yl-ethyl)-amide,
5-Chloro-1H-indole-2-carboxylic acid [(1S)-(4-fluoro-benzyl)-2-(4-hydroxy-piperidin-1-yl)-2-oxo-ethyl]-amide,
5-Chloro-1H-indole-2-carboxylic acid [(1S)-benzyl-2-((3RS)-hydroxy-piperidin-1-yl)-2-oxo-ethyl]-amide,
5-Chloro-1H-indole-2-carboxylic acid [2-oxo-2-((1RS)-oxo-1-thiazolidin-3-yl)-ethyl]-amide,
5-Chloro-1H-indole-2-carboxylic acid [(1S)-(2-fluoro-benzyl)-2-(4-hydroxy-piperidin-1-yl)-2-oxo-ethyl]-amide,

5-Chloro-1H-indole-2-carboxylic acid [(1S)-benzyl-2((3S,4S)-dihydroxy-pyrrolidin-1-yl)-2-oxo-ethyl]-amide,
5-Chloro-1H-indole-2-carboxyiic acid [(1S)-benzyl-2-(3-hydroxy-azetidin-1-yl)-2-oxo-ethyl]-amide,
5-Chloro-1H-indole-2-carboxylic acid [(1S)-benzyl-2-(3-hydroxyimino-azetidin-1-yl)-2-oxo-ethyl]-amide or
5-Chloro-1H-indole-2-carboxylic acid [(1S)-benzyl-2-(4-hydroxyimino-piperidin-1-yl)-2-oxo-ethyl]-amide.

Within the above group of especially preferred compounds is a first group of particularly preferred compounds wherein

$R_4$ is H; and
$R_{12}$ is thiazolidin-3-yl, 1-oxo-thiazolidin-3-yl, 1,1-dioxo-thiazolidin-3-yl or oxazolidin-3-yl or said $R_{12}$ substituents optionally mono- or di-substituted independently with carboxy, $(C_1-C_5)$alkoxycarbonyl, hydroxy$(C_1-C_3)$alkyl, amino$(C_1-C_3)$alkyl, mono-N- or di-N,N-$(C_1-C_3)$alkylamino$(C_1-C_3)$alkyl or
$R_{12}$ is mono- or di-substituted pyrrolidin-1-yl wherein said substituents are independently carboxy, $(C_1-C_5)$alkoxycarbonyl, $(C_1-C_5)$alkoxy, hydroxy, hydroxy$(C_1-C_3)$alkyl, amino, amino$(C_1-C_3)$alkyl, mono-N- or di-N,N-$(C_1-C_3)$alkylamino$(C_1-C_3)$alkyl or mono-N- or di-N,N-$(C_1-C_4)$alkylamino; and
the $R_{12}$ rings are optionally additionally independently disubstituted with $(C_1-C_5)$alkyl.

Preferred compounds within the immediately preceding group of particularly preferred compounds are compounds wherein

a.

$R_1$ is 5-chloro;
$R_{10}$ and $R_{11}$ are H; and
$R_{12}$ is cis-3,4-dihydroxy-pyrrolidin-1-yl;

b.

$R_1$ is 5-chloro;
$R_{10}$ and $R_{11}$ are H; and
$R_{12}$ is (3S,4S)-dihydroxy-pyrrolidin-1-yl;

C.

$R_1$ is 5-chloro;
$R_{10}$ and $R_{11}$ are H; and
$R_{12}$ is 1,1-dioxo-thiazolidin-3-yl;

d.

$R_1$ is 5-chloro;
$R_{10}$ and $R_{11}$ are H; and
$R_{12}$ is thiazolidin-3-yl; and

e.

$R_1$ is 5-chloro;
$R_{10}$ and $R_{11}$ are H; and
$R_{12}$ is 1-oxo-thiazolidin-3-yl.

Within the above group of especially preferred compounds is a second group of particularly preferred compounds wherein

$R_4$ is phenylmethyl, thien-2- or -3-ylmethyl wherein said $R_4$ rings are optionally mono- or di-substituted with fluoro; and
$R_{12}$ is thiazolidin-3-yl, 1-oxo-thiazolidin-3-yl, 1,1-dioxo-thiazolidin-3-yl or oxazolidin-3-yl or said $R_{12}$ substituents optionally mono- or di-substituted independently with carboxy or $(C_1-C_5)$alkoxycarbonyl, hydroxy$(C_1-C_3)$alkyl, amino$(C_1-C_3)$alkyl or mono-N- or di-N,N-$(C_1-C_3)$alkylamino$(C_1-C_3)$alkyl

or $R_{12}$ is mono- or di-substituted azetidin-1-yl or mono- or di-substituted pyrrolidin-1-yl or mono- or di-substituted piperidin-1-yl wherein said substituents are independently carboxy, $(C_1\text{-}C_5)$alkoxycarbonyl, hydroxy$(C_1\text{-}C_3)$alkyl, amino$(C_1\text{-}C_3)$alkyl,, mono-N- or di-N,N-$(C_1\text{-}C_3)$alkylamino$(C_1\text{-}C_3)$alkyl, hydroxy, $(C_1\text{-}C_5)$alkoxy, amino, mono-N- or di-N,N-$(C_1\text{-}C_5)$alkylamino, oxo, hydroxyimino or $(C_1\text{-}C_5)$alkoxyimino; and
the $R_{12}$ rings are optionally additionally mono- or di-substituted independently with $(C_1\text{-}C_5)$alkyl.

Preferred compounds within the immediately preceding group of particularly preferred compounds are compounds wherein

a.

$R_1$ is 5-chloro;
$R_{10}$ and $R_{11}$ are H;
$R_4$ is 4-fluorobenzyl;
$R_{12}$ is 4-hydroxypiperidin-1-yl; and
the stereochemistry of carbon (a) is (S);

b.

$R_1$ is 5-chloro;
$R_{10}$ and $R_{11}$ are H;
$R_4$ is benzyl;
$R_{12}$ is 3-hydroxypiperidin-1-yl; and
the stereochemistry of carbon (a) is (S);

C.

$R_1$ is 5-chloro;
$R_{10}$ and $R_{11}$ are H;
$R_4$ is benzyl;
$R_{12}$ is cis-3,4-dihydroxy-pyrrolidin-1-yl; and
the stereochemistry of carbon (a) is S;

d.

$R_1$ is 5-chloro;
$R_{10}$ and $R_{11}$ are H; $R_4$ is benzyl;
$R_{12}$ is 3-hydroxyimino-pyrrolidin-1-yl; and
the stereochemistry of carbon (a) is (S);

e.

$R_1$ is 5-chioro;
$R_{10}$ and $R_{11}$ are H;
$R_4$ is 2-fluorobenzyl;
$R_{12}$ is 4-hydroxypiperidin-1-yl; and
the stereochemistry of carbon (a) is (S);

f.

$R_1$ is 5-chloro;
$R_{10}$ and $R_{11}$ are H;
$R_4$ is benzyl;
$R_{12}$ is (3S,4S)-dihydroxy-pyrrolidin-1-yl; and
the stereochemistry of carbon (a) is (S);

g.

$R_1$ is 5-chloro;
$R_{10}$ and $R_{11}$ are H;
$R_4$ is benzyl;
$R_{12}$ is 3-hydroxy-azetidin-1-yl; and
the stereochemistry of carbon (a) is (S);

h.

$R_1$ is 5-chloro;
$R_{10}$ and $R_{11}$ are H;
$R_4$ is benzyl;
$R_{12}$ is 3-hydroxyimino-azetidin-1-yl; and
the stereochemistry of carbon (a) is (S); and

i.

$R_1$ is 5-chloro;
$R_{10}$ and $R_{11}$ are H;
$R_4$ is benzyl;
$R_{12}$ is 4-hydroxyimino-piperidin-1-yl; and
the stereochemistry of carbon (a) is (S).

A second group of especially preferred compounds within the first group of preferred compounds are the compounds wherein

$R_4$ is H, phenyl($C_1$-$C_2$)alkyl, thien-2- or -3-yl($C_1$-$C_2$)alkyl, fur-2- or -3-yl($C_1$-$C_2$)alkyl wherein said $R_4$ rings are mono- or di-substituted independently with H or fluoro;
$R_6$ is C(O)$NR_8R_9$;and
$R_8$ is H, ($C_1$-$C_5$)alkyl, hydroxy or ($C_1$-$C_4$)alkoxy; and
$R_9$ is H, cyclo($C_4$-$C_6$)alkyl, cyclo($C_3$-$C_6$)alkyl($C_1$-$C_5$)alkyl, methylene-perfluorinated($C_1$-$C_3$)alkyl, pyridyl, pyrrolidinyl, oxazolyl, thiazolyl, imidazolyl, piperidinyl, benzothiazolyl or thiochromanyl; or
$R_9$ is ($C_1$-$C_5$)alkyl wherein said ($C_1$-$C_5$)alkyl is optionally substituted with cyclo($C_4$-$C_6$)alkenyl, phenyl, thienyl, pyridyl, pyrrolidinyl, oxazolyl, thiazolyl, imidazolyl, pyrazolyl, piperidinyl, morpholinyl, thiomorpholinyl, 1-oxothiomorpholinyl, or 1,1-dioxothiomorpholinyl and wherein said ($C_1$-$C_5$)alkyl or ($C_1$-$C_4$)alkoxy is optionally additionally independently mono- or di-substituted with halo, hydroxy, ($C_1$-$C_5$)alkoxy, amino, mono-N- or di-N,N-($C_1$-$C_5$)alkylamino, cyano, carboxy, or ($C_1$-$C_4$)alkoxycarbonyl; and
wherein the $R_9$ rings are optionally mono- or di-substituted independently on carbon with halo, ($C_1$-$C_4$)alkyl, ($C_1$-$C_4$)alkoxy, hydroxy, amino, mono-N- or di-N,N-($C_1$-$C_4$)alkylamino, carbamoyl, ($C_1$-$C_5$)alkoxycarbonyl or carbamoyl.

Within the immediately preceding second group of especially preferred compounds are the compounds wherein

a.

$R_1$ is 5-chloro;
$R_{10}$ and $R_{11}$ are H;
$R_4$ is benzyl;
$R_8$ is methyl; and
$R_9$ is 3-(dimethylamino)propyl;

b.

the stereochemistry of carbon (a) is (S);
$R_1$ is 5-chloro;
$R_{10}$ and $R_{11}$ are H;
$R_4$ is benzyl;
$R_8$ is methyl; and

$R_9$ is 3-pyridyl;

c.

the stereochemistry of carbon (a) is (S);
$R_1$ is 5-chloro;
$R_{10}$ and $R_{11}$ are H;
$R_4$ is benzyl;
$R_8$ is methyl; and
$R_9$ is 2-hydroxyethyl; and

d.

the stereochemistry of carbon (a) is (S);
$R_1$ is 5-fluoro;
$R_{10}$ and $R_{11}$ are H;
$R_4$ is 4-fluorophenylmethyl;
$R_8$ is methyl; and
$R_9$ is 2-morpholinoethyl.

A third group of especially preferred compounds within the first group of preferred compounds are the compounds wherein

$R_4$ is H, phenyl($C_1$-$C_2$)alkyl, thien-2- or -3-yl($C_1$-$C_2$)alkyl, fur-2- or -3-yl($C_1$-$C_2$)alkyl wherein said $R_4$ rings are mono- or di-substituted independently with H or fluoro;
$R_6$ is C(O)N$R_8R_9$;and
$R_8$ is H, ($C_1$-$C_5$)alkyl, hydroxy or ($C_1$-$C_4$)alkoxy; and
$R_9$ is ($C_1$-$C_4$)alkoxy wherein said ($C_1$-$C_4$)alkoxy is optionally substituted with cyclo($C_4$-$C_6$)alkenyl, phenyl, thienyl, pyridyl, pyrrolidinyl, oxazolyl; thiazolyl, imidazolyl, pyrazolyl, piperidinyl, morpholinyl, thiomorpholinyl, 1-oxothiomorpholinyl, or 1,1-dioxothiomorpholinyl and wherein said ($C_1$-$C_5$)alkyl or ($C_1$-$C_4$)alkoxy is optionally additionally independently mono- or di-substituted with halo, hydroxy, ($C_1$-$C_5$)alkoxy, amino, mono-N- or di-N, N-($C_1$-$C_5$)alkylamino, cyano, carboxy, or ($C_1$-$C_4$)alkoxycarbonyl; and
wherein the $R_9$ rings are optionally mono- or di-substituted independently on carbon with halo, ($C_1$-$C_4$)alkyl, ($C_1$-$C_4$)alkoxy, hydroxy, amino, mono-N- or di-N,N-($C_1$-$C_4$)alkylamino, carbamoyl, ($C_1$-$C_5$)alkoxycarbonyl or carbamoyl.

Within the immediately preceding third group of especially preferred compounds are the compounds wherein

a.

$R_1$ is 5-chloro;
$R_{10}$ and $R_{11}$ are H;
$R_4$ is benzyl;
$R_8$ is methyl; and
$R_9$ is 2-hydroxyethoxy;

b.

the stereochemistry of carbon (a) is (S);
$R_1$ is 5-chloro;
$R_{10}$ and $R_{11}$ are H;
$R_4$ is 4-fluorophenylmethyl;
$R_8$ is methyl; and
$R_9$ is methoxy;

C.

the stereochemistry of carbon (a) is (S);

$R_1$ is 5-chloro;
$R_{10}$ and $R_{11}$ are H;
$R_4$ is benzyt;
$R_8$ is methyl; and
$R_9$ is methoxy;

A second group of preferred compounds of Formula IA are those compounds wherein

$R_1$ is 5-halo, 5-methyl, 5-cyano or trifluoromethyl;
$R_{10}$ and $R_{11}$ are each independently H or halo;
A is -C(H)=;
$R_2$ and $R_3$ are H;
$R_4$ is H, phenyl($C_1$-$C_2$)alkyl, thien-2- or -3-yl($C_1$-$C_2$)alkyl, fur-2- or -3-yl($C_1$-$C_2$)alkyl wherein said rings are mono- or di-substituted independently with H or fluoro;
$R_5$ is H; and
$R_6$ is ($C_1$-$C_5$)alkoxycarbonyl.

A third group of preferred compounds of Formula IA are those compounds wherein

$R_1$ is 5-halo, 5-methyl, 5-cyano or trifluoromethyl;
$R_{10}$ and $R_{11}$ are each independently H or halo;
A is -C(H)=;
$R_2$ and $R_3$ are H;
$R_4$ is H, methyl or phenyl($C_1$-$C_2$)alkyl, wherein said phenyl groups are mono-or di-substituted independently with H, halo, ($C_1$-$C_4$)alkyl, ($C_1$-$C_4$)alkoxy, trifluoromethyl, hydroxy, amino or cyano and wherein said phenyl groups are additionally mono- or di-substituted independently H or halo; or
$R_4$ is thien-2- or -3-yl($C_1$-$C_2$)alkyl, pyrid-2-, -3- or -4-yl($C_1$-$C_2$)alkyl, thiazol-2-,-4- or -5-yl($C_1$-$C_2$)alkyl, imidazol-2-, -4- or -5-yl($C_1$-$C_2$)alkyl, fur-2- or -3-yl($C_1$-$C_2$)alkyl, pyrrol-2- or -3-yl($C_1$-$C_2$)alkyl, oxazol-2-, -4- or -5-yl($C_1$-$C_2$)alkyl, pyrazol-3-, -4- or -5-yl($C_1$-$C_2$)alkyl, isoxazol-3-, -4- or -5-yl($C_1$-$C_2$)alkyl, isothiazol-3-, -4- or -5-yl($C_1$-$C_2$)alkyl, pyridazin-3- or -4-yl($C_1$-$C_2$)alkyl, pyrimidin-2-, -4-, -5- or -6-yl($C_1$-$C_2$)alkyl, pyrazin-2- or -3-yl($C_1$-$C_2$)alkyl or 1,3,5-triazin-2-yl($C_1$-$C_2$)alkyl wherein said preceding $R_4$ heterocycles are optionally mono- or di-substituted independently with halo, trifluoromethyl, ($C_1$-$C_4$)alkyl, ($C_1$-$C_4$)alkoxy, amino or hydroxy and said mono- or di-substituents are bonded to carbon;
$R_5$ is H; and
$R_6$ is carboxy.

Within the third group of preferred compounds is a first group of especially preferred compounds wherein

$R_{10}$ and $R_{11}$ are H; and
$R_4$ is H.

Particularly preferred within the immediately preceding especially preferred group is a compound wherein $R_1$ is 5-chloro.

Another aspect of this invention is the use of

a. a first compound, said first compound being an aldose reductase inhibitor; and
b. a second compound, said second compound being a glycogen phosphorylase inhibitor in the manufacture of a medicament for treating a mammal having an insulin resistant condition.

Preferred insulin resistant conditions taken individually or as a group include diabetes, hyperinsulinemia, impaired glucose tolerance, hyperglycemia and/or hyperlipidemia following meals, type II diabetes, altered body composition, reduction of lean body mass, obesity (especially abdominal visceral obesity), hypertension, dyslipidemia (e.g., increased free fatty acid, triglyceride, VLDL cholesterol, and LDL cholesterol levels, and reduced HDL cholesterol level), atherosclerosis, tissue ischemia and cardiovascular diseases, syndrome X (also referred to as Metabolic syndrome"), pregnancy, conditions of infection, uremia, hyperandrogenism, hypercortisolemia or other conditions of adrenocortical hormone excess, acromegaly growth hormone excess or polycystic ovarian disease.

Especially preferred insulin resistant conditions taken individually or as a group include dyslipidemia, tissue ischemia, obesity, polycystic ovarian disease, syndrome X and hypertension.

In particular diabetes is especially preferred.

A preferred aldose reductase inhibitor is 1-phthalazineacetic acid, 3,4-dihydro-4-oxo-3-[[5-trifluoromethyl)-2-benzothiazolyl]methyl]- or a pharmaceutically acceptable salt thereof.

A preferred glycogen phosphorylase inhibitor is

5-chloro-1H-indole-2-carboxylic add [(1S)-benzyl-(2R)-hydroxy-3-((3S)-hydroxypyrrolidin-1-yl)-3-oxopropyl]-amide;

5-chloro-1H-indole-2-carboxylic acid [(1S)-benzyl-3-((3S,4S)-dihydroxypyrrolidin-1-yl) -(2R)-hydroxy- 3-oxopropyl]-amide;

5-chloro-1H-indole-2-carboxylic acid [(1S)-((R)-hydroxy-dimethylcarbamoyl-methyl)-2-phenyl-ethyl]-amide;

5-chloro-1H-indole-2-carboxylic add [(1S)-((R)-hydroxy-methoxy-methyl-carbamoyl)-methyl)-2-phenyl-ethyl]-amide;

5-chloro-1H-indole-2-carboxylic acid [(1S)-((R)-hydroxy-[(2-hydroxy-ethyl)-methyl-carbamoyl]-methyl)-2-phenyl-ethyl]-amide;

5-chloro-1H-indole-2-carboxylic acid [(1S)-benzyl-2-(3-hydroxyimino-pyrrolidin-1-yl)-2-oxo-ethyl]-amide;

5-chloro-1H-indole-2-carboxylic acid [2-(cis-3,4-dihydroxy-pyrrolidin-1-yl)-2-oxo-ethyl]-amide;

5-chloro-1H-indole-2-carboxylic acid [(1S)-benzyl-3-((cis)-dihydroxypyrrolidin-1-yl)-(2R)-hydroxy- 3-oxopropyl]-amide;

5-chloro-1H-indole-2-carboxylic acid [2-((3S,4S)- dihydroxy-pyrrolidin-1-yl)-2-oxo-ethyl]-amide;

5-chloro-1H-indole-2-carboxylic acid [(1S)-benzyl-3-((cis)-dihydroxypyrrolidin-1-yl)-(2R)-hydroxy-3-oxopropyl]-amide;

5-chloro-1 H-indole-2-carboxylic acid [(1S)-benzyl-2-(cis-3,4-dihydroxy-pyrrolidin-1-yl)-2-oxo-ethyl]-amide;

5-chloro-1H-indole-2-carboxylic acid [2-(1,1-dioxo-thiazolidin-3-yl)-2-oxo-ethyl]-amide;

5-chloro-1 H-indole-2-carboxylic acid [(1S)-(4-fluoro-benzyl)-2-(4-hydroxy-piperidin-1-yl)-2-oxo-ethyl]-amide;

5-chloro-1H-indole-2-carboxylic acid [(1S)-benzyl-2-((3RS)-hydroxy-piperidin-1-yl)-2-oxo-ethyl]-amide;

5-chloro-1 H-indole-2-carboxylic acid [2-oxo-2-((1RS)-oxo-thiazolidin-3-yl)-ethyl]-amide; or

5-chloro-1H-indole-2-carboxylic acid [(1S)-benzyl-2-(3-hydroxy-azetidin-1-yl)-2-oxo-ethyl]-amide.

A particularly preferred mammal is a female or male human.

Another preferred aspect of this method is wherein the first compound and the second compound are administered substantially simultaneously.

Another aspect of this invention is a synergistic pharmaceutical composition for achieving an insulin sensitizing effect in a mammal comprising

a. an amount of a first compound, said first compound being an aldose reductase inhibitor, and
b. an amount of a second compound, said second compound being a glycogen phosphorylase inhibitor

wherein the amount of the first compound alone and the amount of the second compound alone is insufficient to achieve the insulin sensitizing effect if administered alone and wherein the combined effect of the amounts of the first and second compounds is greater than the sum of the insulin sensitizing effects achievable with the individual amounts of the first and second compound, and a pharmaceutically acceptable diluent or carrier.

Another aspect of this invention is a kit comprising:

a. a therapeutically effective amount of an aldose reductase inhibitor and a pharmaceutically acceptable carrier in a first unit dosage form;

b. a therapeutically effective amount of a glycogen phosphorylase inhibitor and a pharmaceutically acceptable carrier in a second unit dosage form; and

c. container means for containing said first and second dosage forms.

Yet another aspect of this invention is the use of

a. a first compound, said first compound being an aldose reductase inhibitor; and

b. a second compound, said second compound being a glycogen phosphorylase inhibitor, in the manufacture of a medicament for achieving an insulin sensitizing effect in a mammal which presents insulin resistant conditions,

wherein the amount of the first compound alone and the amount of the second compound alone is insufficient to achieve said insulin sensitizing effect and wherein the combined effect of the amounts of the first and second compounds is greater than the sum of the insulin sensitizing effects achievable with the individual amounts of the first and second compound.

Another aspect of this invention is directed to the use of

a. an aldose reductase inhibitor; and

b. a glycogen phosphorylase inhibitor in the manufacture of a medicament for reducing tissue damage resulting from or which could result from schemia.

Preferred ischemic tissues taken individually or as a group are wherein the ischemic tissue is cardiac, brain, liver, kidney, lung, gut, skeletal muscle, spleen, pancreas, nerve, spinal cord, retina tissue, the vasculature, or intestinal tissue.

An especially preferred ischemic tissue is cardiac tissue.

Preferably, the combination of this invention is being administered prophylactically.

The ischemic damage treatable according to this invention may occur during organ transplantation.

Preferably, the combination of this invention is being administered prior to cardiac surgery.

The term insulin resistance conditions refers to conditions (an insulin resistant syndrome or state) in which the sensitivity and/or responsiveness to insulin in organs, tissues, or cells in the body of a mammal is reduced compared to the normal (or insulin sensitive) state. This resistance results in multiple abnormalities in glucose, protein, and lipid metabolism, electrolyte and ion imbalances, and growth, for example, by organs, tissues, and cells, which can manifest in one or more of the following (but not limited to): hyperinsulinemia, impaired glucose tolerance (IGT), hyperglycemia and/or hyperlipidemia following meals, type II diabetes, altered body composition, reduction of lean body mass, obesity (especially abdominal visceral obesity), hypertension, dyslipidemia (e.g. increased free fatty acid, triglyceride, VLDL cholesterol, and LDL cholesterol levels, and reduced HDL cholesterol level), atherosclerosis, tissue ischemia, and cardiovascular diseases (Kopelman and Albon, 1997; DeFronzo and Ferrannini, 1991; Reaven, 1991; Malmstrom, et al., 1997). As a result of the insulin resistant state, a greater amount of insulin is required to approach or achieve the same biological action of insulin in organs, tissues, and cells compared to the normal (or insulin sensitive) state, which leads to an increased demand in the pancreas to secrete more insulin (worsened hyperinsulinemia), and in the most extreme circumstance, pancreatic failure and insulin insufficiency leading to a type I diabetic condition. Insulin resistant states can, for example, include obesity, syndrome X (also referred to as "Metabolic syndrome"), pregnancy, or conditions of infection, uremia, hyperandrogenism, hypercortisolemia or other conditions of adrenocortical hormone excess, acromegaly or growth hormone excess, polycystic ovaries, or can be associated with older age or specific ethnic groups (Kopelman and Albon, 1997).

The term insulin sensitizing effect refers to a state wherein patient's tissues are made to give a normal or better than normal biologic response to a given amount of insulin.

The term "reduction" is intended to include partial prevention or prevention which, although greater than that which would result from taking no drug or from taking placebo, is less than 100% in addition to substantially total prevention.

The term "damage resulting from [...] ischemia" as employed herein refers to conditions directly associated with reduced blood flow to tissue, for example due to a dot or obstruction of blood vessels which supply blood to the subject tissue and which result, inter alia, in lowered oxygen transport to such tissue, impaired tissue performance, tissue dysfunction and necrosis. Alternatively, where blood flow or organ perfusion may be quantitatively adequate, the oxygen carrying capacity of the blood or organ perfusion medium may be reduced, e.g., in hypoxic environment, such that oxygen supply to the tissue is lowered, and impaired tissue performance, tissue dysfunction, and tissue necrosis ensues.

The term aldose reductase inhibitor refers to compounds which inhibit the bioconversion of glucose to sorbitol catalyzed by the enzyme aldose reductase.

The term glycogen phosphorylase inhibitor refers to any substance or agent or any combination of substances and/or agents which reduces, retards, or eliminates the enzymatic action of glycogen phosphorylase. The currently known enzymatic action of glycogen phosphorylase is the degradation of glycogen by catalysis of the reversible reaction of a glycogen macromolecule and inorganic phosphate to glucose-1-phosphate and a glycogen macromolecule which is one glucosyl residue shorter than the original glycogen macromolecule (forward direction of glycogenolysis).

The term "treating", "treat" or "treatment" as used herein includes preventative (e.g., prophylactic) and palliative treatment.

By "pharmaceutically acceptable" it is meant the carrier, diluent, excipients, and/or salt must be compatible with the other ingredients of the formulation, and not deleterious to the recipient thereof.

The expression "prodrug" refers to compounds that are drug precursors which following administration, release the drug *in vivo* via some chemical or physiological process (e.g., a prodrug on being brought to the physiological pH or through enzyme action is converted to the desired drug form). Exemplary prodrugs upon cleavage release the corresponding free acid.

By alkylene is meant saturated hydrocarbon (straight chain or branched ) wherein a hydrogen atom is removed from each of the terminal carbons. Exemplary of such groups (assuming the designated length encompasses the particular example) are methylene, ethylene, propylene, butylene, pentylene, hexylene, heptylene).

By halo is meant chloro, bromo, iodo, or fluoro.

By alkyl is meant straight chain saturated hydrocarbon or branched saturated hydrocarbon. Exemplary of such alkyl groups (assuming the designated length encompasses the particular example) are methyl, ethyl, propyl, isopropyl, butyl, sec-butyl, tertiary butyl, pentyl, isopentyl, neopentyl, tertiary pentyl, 1-methylbutyl, 2-methylbutyl, 3-methylbutyl, hexyl, isohexyl, heptyl and octyl.

By alkoxy is meant straight chain saturated alkyl or branched saturated alkyl bonded through an oxy. Exemplary of such alkoxy groups (assuming the designated length encompasses the particular example) are methoxy, ethoxy, propoxy, isopropoxy, butoxy, isobutoxy, tertiary butoxy, pentoxy, isopentoxy, neopentoxy, tertiary pentoxy, hexoxy, isohexoxy, heptoxy and octoxy.

As used herein the term mono-N- or di-N,N-($C_1$-$C_x$)alkyl... refers to the ($C_1$-$C_x$)alkyl moiety taken independently when it is di-N,N-($C_1$-$C_x$)alkyl...(x refers to integers).

It is to be understood that if a carbocydic or heterocyclic moiety may be bonded or otherwise attached to a designated substrate, through differing ring atoms without denoting a specific point of attachment, then all possible points are intended, whether through a carbon atom or, for example, a trivalent nitrogen atom. For example, the term "pyridyl" means, for example, 2-, 3-, or 4-pyridyl, the term "thienyl" means, for example, 2-, or 3-thienyl, and so forth.

The expression "pharmaceutically-acceptable salt" refers to nontoxic anionic salts containing anions such as (but not limited to) chloride, bromide, iodide, sulfate, bisulfate, phosphate, acetate, maleate, fumarate, oxalate, lactate, tartrate, citrate, gluconate, methanesulfonate and 4-toluene-sulfonate. The expression also refers to nontoxic cationic salts such as (but not limited to) sodium, potassium, calcium, magnesium, ammonium or protonated benzathine (N, N'-dibenzylethylenediamine), choline, ethanolamine, diethanolamine, ethylenediamine, meglamine (N-methyl-glucamine), benethamine (N-benzylphenethylamine), piperazine or tromethamine (2-amino-2-hydroxymethyl-1,3-propanediol).

As used herein, the expressions "reaction-inert solvent" and "inert solvent" refers to a solvent or mixture of solvents which does not interact with starting materials, reagents, intermediates or products in a manner which adversely affects the yield of the desired product

The parenthetical negative or positive sign used herein in the nomenclature denotes the direction plane polarized light is rotated by the particular stereoisomer.

The chemist of ordinary skill will recognize that certain compounds of this invention will contain one or more atoms which may be in a particular stereochemical or geometric configuration, giving rise to stereoisomers and configurational isomers. All such isomers and mixtures thereof are included in this invention. Hydrates and solvents of the compounds of this invention are also included.

DTT means dithiothreitol. DMSO means dimethyl sulfoxide. EDTA means ethylenediamine tetraacetic acid.

Other features and advantages will be apparent from the specification and claims which describe the invention.

## DETAILED DESCRIPTION OF THE INVENTION

In general the compounds of this invention can be made by processes which include processes known in the chemical arts, particularly in light of the description contained herein. Certain processes for the manufacture of the compounds of this invention are provided as further features of the invention and are illustrated by the following reaction schemes. Other processes may be described in the experimental section.

Any aldose reductase inhibitor may be used as a compound (active agent) of this invention. The term aldose reductase inhibitor refers to compounds which inhibit the bioconversion of glucose to sorbitol catalyzed by the enzyme aldose reductase. Such inhibition is readily determined by those skilled in the art according to standard assays (J. Malone, Diabetes, 29:861-864, 1980. "Red Cell Sorbitol, an Indicator of Diabetic Control"). A variety of aldose reductase inhibitors are described and referenced below, however, other aldose reductase inhibitors will be known to those skilled in the art.

Common chemical USAN names or other designation are in parentheses where applicable, together with reference to appropriate patent literature disclosing the compound.

The activity of an aldose reductase inhibitor in a tissue can be determined by testing the amount of aldose reductase inhibitor that is required to lower tissue sorbitol (i.e., by inhibiting the further production of sorbitol consequent to blocking aldose reductase) or lower tissue fructose (by inhibiting the production of sorbitol consequent to blocking aldose reductase and consequently the production of fructose). While not wishing to be bound by any particular theory or mechanism, it is believed that an aldose reductase inhibitor, by inhibiting aldose reductase, prevents or reduces ischemic damage as described hereinafter.

Accordingly, examples of aldose reductase inhibitors useful in the compositions and methods of this invention include:

1. 3-(4-bromo-2-fluorobenzyl)-3,4-dihydro-4-oxo-1-phthalazineacetic acid (ponalrestat, US 4,251,528);
2. N[[(5-trifluoromethyl)-6-methoxy-1-naphthalenyl]thioxomethyl}-N-methylglycine (tolrestat, US 4,600,724);
3. 5-[(Z,E)-β-methylcinnamylidene]-4-oxo-2-thioxo-3-thiazolideneacetic acid (epalrestat, US 4,464,382, US 4,791,126, US 4,831,045);
4. 3-(4-bromo-2-fluorobenzyl)-7-chloro-3,4-dihydro-2,4-dioxo-1(2H)-quinazolineacetic add (zenarestat, US 4,734,419, and 4,883,800);
5. 2R,4R-6,7-dichloro-4-hydroxy-2-methylchroman-4-acetic acid (US 4,883,410);
6. 2R,4R-6,7-dichloro-6-fluoro-4-hydroxy-2-methylchroman-4-acetic acid (US 4,883,410);
7. 3,4-dihydro-2,8-diisopropyl-3-oxo-2H-1,4-benzoxazine-4-acetic acid (US 4,771,050);
8. 3,4-dihydro-3-oxo-4-[(4,5,7-trifluoro-2-benzothiazolyl)methyl]-2H-1,4-benzothiazine-2-acetic acid (SPR-210, U.S. 5,252,572);
9. N-[3,5-dimethyl-4-[(nitromethyl)sulfonyl]phenyl]-2-methyl-benzeneacetamide (ZD5522, U.S. 5,270,342 and U.S. 5,430,060);
10. (S)-6-fluorospiro[chroman-4,4'-imidazolidine]-2,5'-dione (sorbinil, US 4,130,714);
11. d-2-methyl-6-fluoro-spiro(chroman-4',4'-imidazolidine)-2',5'-dione (US 4,540,704);
12. 2-fluoro-spiro(9H-fluorene-9,4'imidazolidine)2',5'-dione (US 4,438,272);
13. 2,7-di-fluoro-spiro(9H-fluorene-9,4'imidazolidine)2',5'-dione (US 4,436,745, US 4,438,272);
14. 2,7-di-fluoro-5-methoxy-spiro(9H-fluorene-9,4' imidazolidine)2',5'-dione (US 4,436,745, US 4,438,272);
15. 7-fluoro-spiro(5H-indenol[1,2-b]pyridine-5,3'-pyrrolidine)2,5'-dione (US 4,436,745, US 4,438,272);
16. d-cis-6'-chloro-2',3'-dihydro-2'-methyl-spiro-(imidazolidine-4,4'-4'-H-pyrano(2,3-b)pyridine)-2,5-dione (US 4,980,357);
17. spiro[imidazolidine-4,5'(6H)-quinoline]2,5-dione-3'-chloro-7,'8'-dihydro-7'-methyl-(5'-cis)(US 5,066,659);
18. (2S,4S)-6-fluoro-2',5'-dioxospiro(chroman-4,4'-imidazolidine)-2-carboxamide (US 5,447,946); and
19. 2-[(4-bromo-2-fluorophenyl)methyl]-6-fluorospiro[isoquinoline-4(1H),3'-pyrrolidine]-1,2',3,5'(2H)-tetrone (ARI-509, US 5,037,831).

Other aldose reductase inhibitors include compounds having formula II

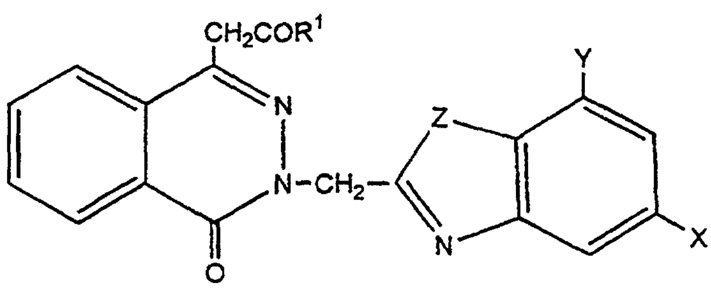

(II)

or a pharmaceutically acceptable salt thereof, wherein

Z is O or S;
$R^1$ is hydroxy or a group capable of being removed in vivo to produce a compound of formula II wherein $R^1$ is OH; and
X and Y are the same or different and are selected from hydrogen, trifluoromethyl, fluoro, and chloro.

A preferred subgroup within the above group of aldose reductase inhibitors includes numbered compounds 1, 2, 3, 4, 5, 6, 9, 10, and 17, and the following compounds of Formula II :

20. 3,4-dihydro-3-(5-fluorobenzothiazol-2-ylmethyl)-4-oxophthalazin-1-yl-acetic acid [$R^1$=hydroxy; X=F; Y=H];
21. 3-(5,7-difluorobenzothiazol-2-ylmethyl)-3,4-dihydro-4-oxophthalazin-1-ylacetic acid [$R^1$=hydroxy; X=Y=F];
22. 3-(5-chlorobenzothiazol-2-ylmethyl)-3,4-dihydro-4-oxophthalazin-1-ylacetic acid [$R^1$=hydroxy; X=Cl; Y=H];
23. 3-(5,7-dichlorobenzothiazol-2-ylmethyl)-3,4-dihydro-4-oxophthalazin-1-ylacetic acid [$R^1$=hydroxy; X=Y=Cl];
24. 3,4-dihydro-4-oxo-3-(5-trifluoromethylbenzoxazol-2-ylmethyl)phthalazin-1-ylacetic acid [$R^1$=hydroxy; X=$CF_3$; Y=H];
25. 3,4-dihydro-3-(5-fluorobenzoxazol-2-ylmethyl)-4-oxophthalazin-1-yl-acetic acid [$R^1$=hydroxy; X=F; Y=H];
26. 3-(5,7-difluorobenzoxazol-2-ylmethyl)-3,4-dihydro-4-oxophthalazin-1-ylacetic acid [$R^1$=hydroxy; X=Y=F];
27. 3-(5-chlorobenzoxazol-2-ylmethyl)-3,4-dihydro-4-oxophthalazin-1-ylacetic acid [$R^1$=hydroxy; X=Cl; Y=H];
28. 3-(5,7-dichlorobenzoxazol-2-ylmethyl)-3,4-dihydro-4-oxophthalazin-1-ylacetic acid [$R^1$=hydroxy; X=Y=Cl]; and
29. zopolrestat; 1-phthalazineacetic acid, 3,4-dihydro-4-oxo-3-[[5-(trifluoromethyl)-2-benzothiazolyl]methyl]-[$R^1$=hydroxy; X=trifluoromethyl; Y=H].

In compounds 20-23, and 29 Z is S. In compounds 24-28, Z is O.

Of the above subgroup, compounds 20-29 are more preferred with 29 especially preferred.

The aldose reductase inhibitor compounds of this invention are readily available or can be easily synthesized by those skilled in the art using conventional methods of organic synthesis, particularly in view of the pertinent patent specification descriptions.

Any glycogen phosphorylase inhibitor may be used as the second compound of this invention. The term glycogen phosphorylase inhibitor refers to any substance or agent or any combination of substances and/or agents which reduces, retards, or eliminates the enzymatic action of glycogen phosphorylase. The currently known enzymatic action of glycogen phosphorylase is the degradation of glycogen by catalysis of the reversible reaction of a glycogen macromolecule and inorganic phosphate to glucose-1-phosphate and a glycogen macromolecule which is one glucosyl residue shorter than the original glycogen macromolecule (forward direction of glycogenolysis). Such actions are readily determined by those skilled in the art according to standard assays (e.g., as described hereinafter). A variety of these compounds are included in the following published PCT patent applications: PCT application publication WO 96/39384 and WO96/39385. However, other glycogen phosphorylase inhibitors will be known to those skilled in the art.

In general the compounds of Formula I and IA can be made by processes which include processes known in the chemical arts, particularly in light of the description contained herein. Certain processes for the manufacture of Formula I and IA compounds are provided as further features of the invention and are illustrated by the following reaction schemes.

## SCHEME I

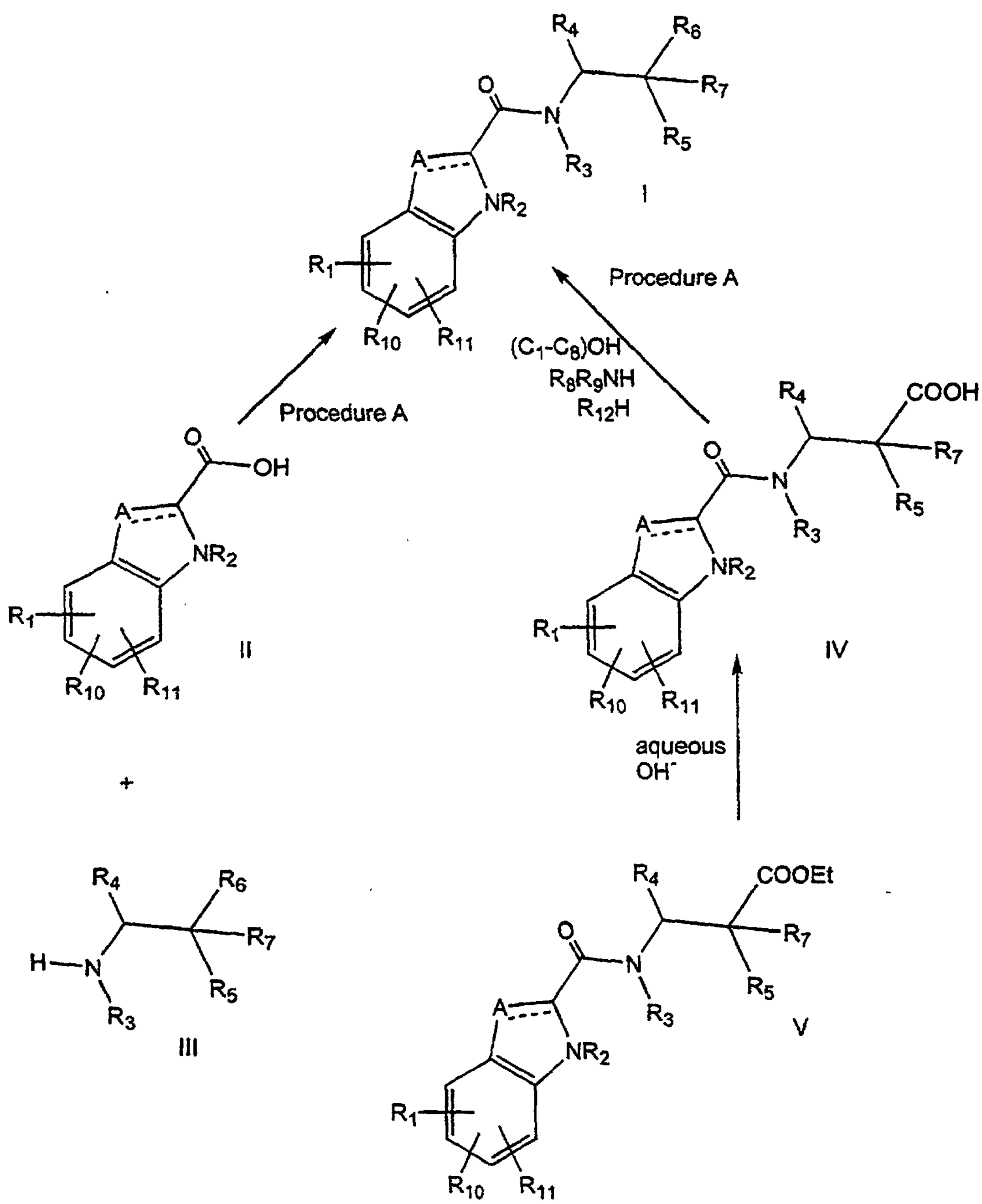
Procedure A
(C1-C8)OH
R8R9NH
R12H
Procedure A
aqueous
OH-
I
II
III
IV
V
+

## SCHEME II

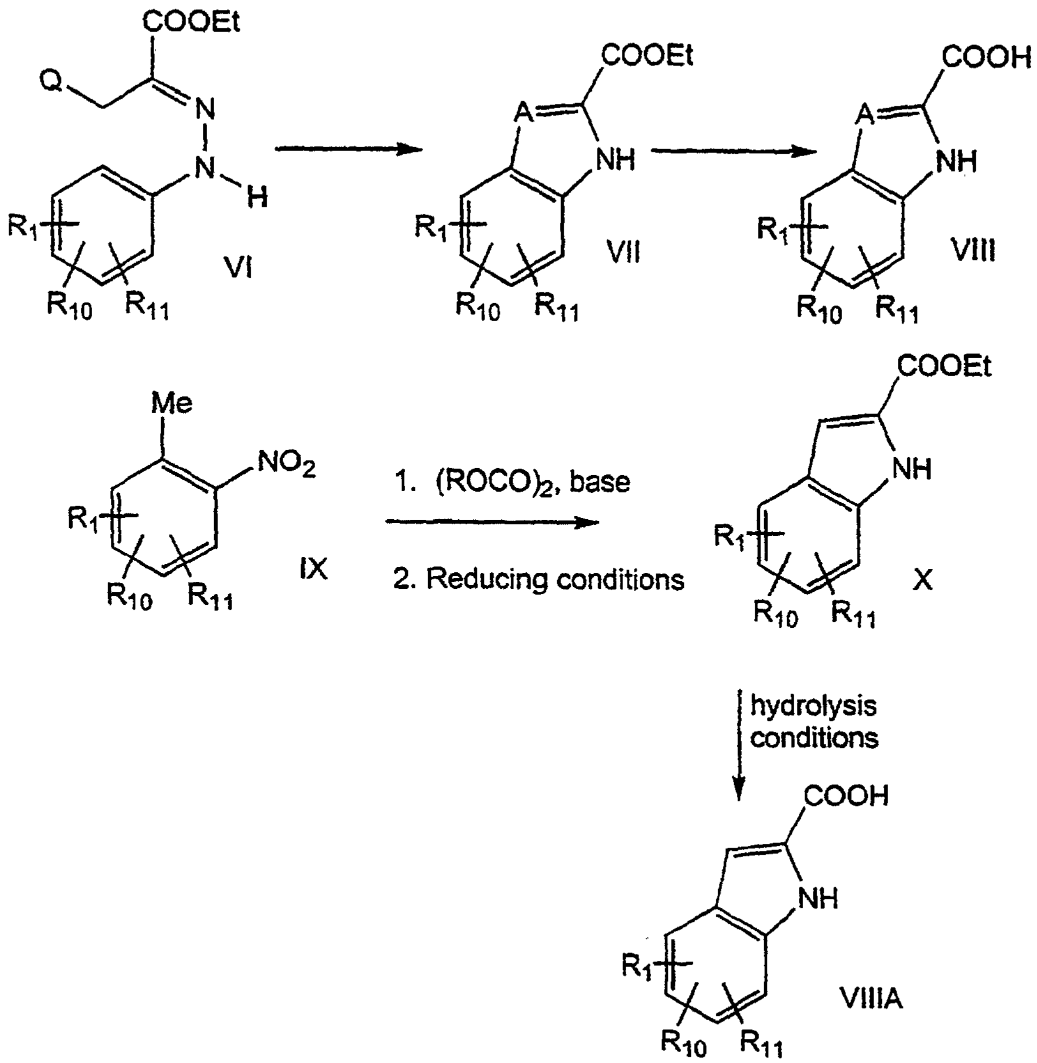
VI
VII
VIII
IX
1. (ROCO)2, base
2. Reducing conditions
X
hydrolysis conditions
VIIIA

## SCHEME III

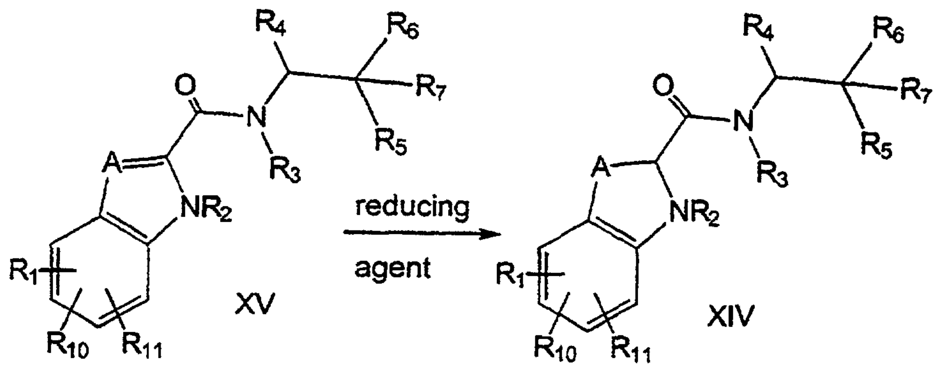


XV XIV

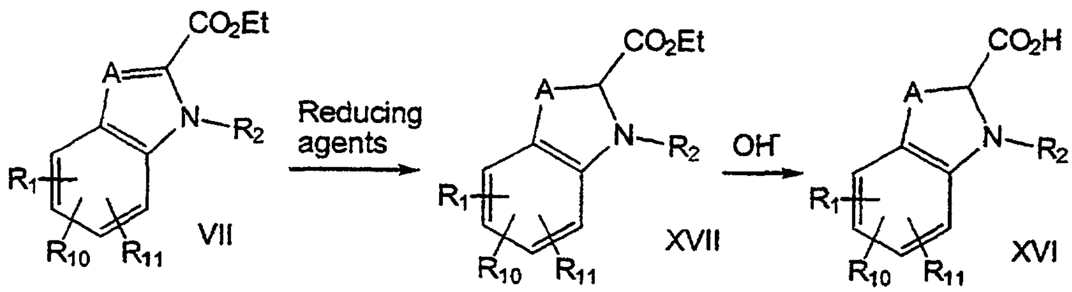


VII XVII XVI

## SCHEME IV

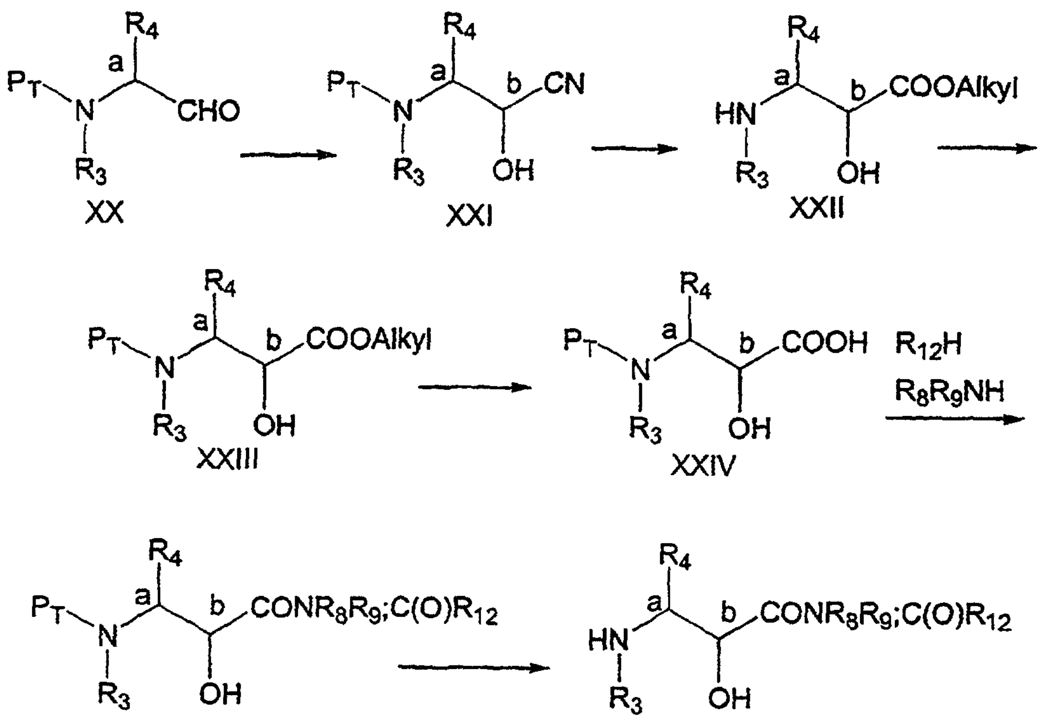


XX XXI XXII XXIII XXIV XXV XXVI

## SCHEME V

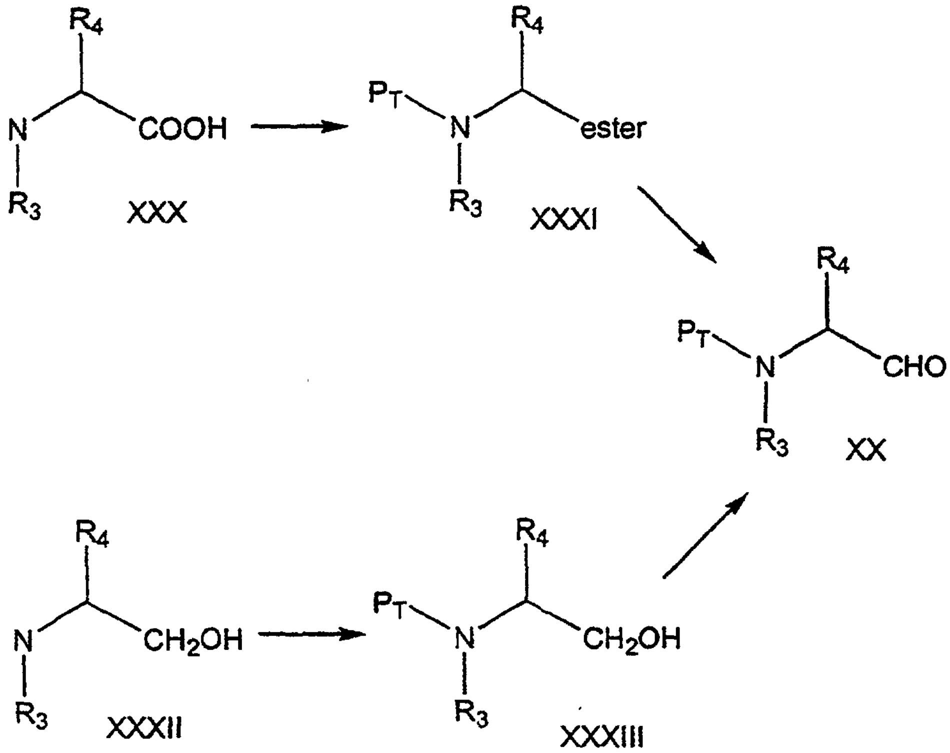

XXX XXXI XX XXXII XXXIII

# SCHEME VI

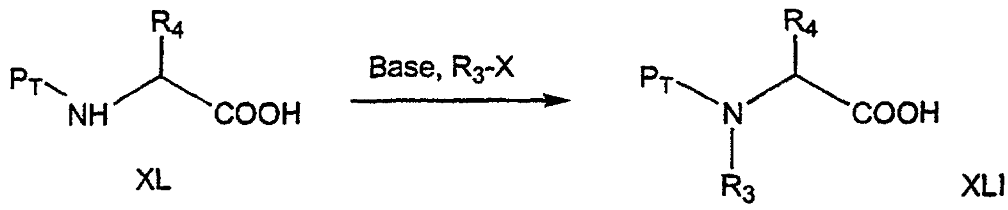


XL

XLI

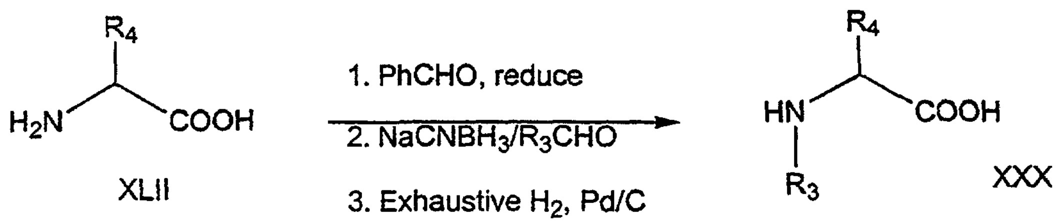


XLII

XXX

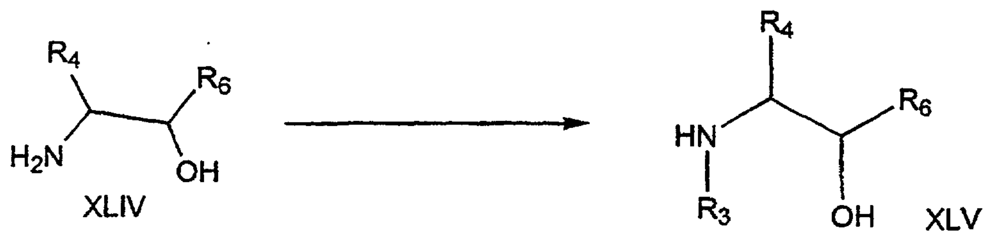

XLIV

XLV

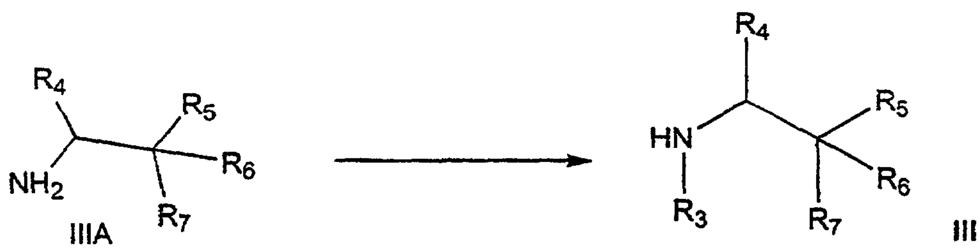

IIIA

III

# SCHEME VII

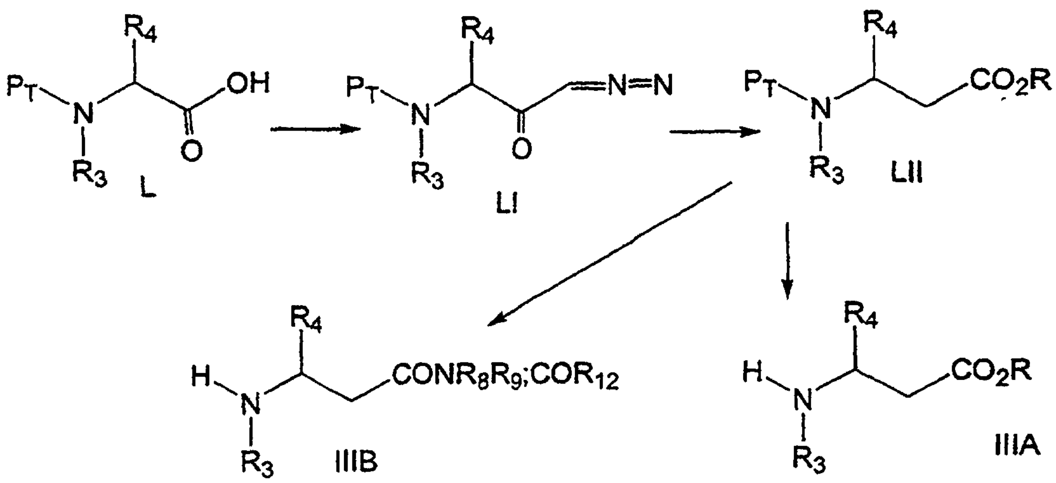

## SCHEME VIII

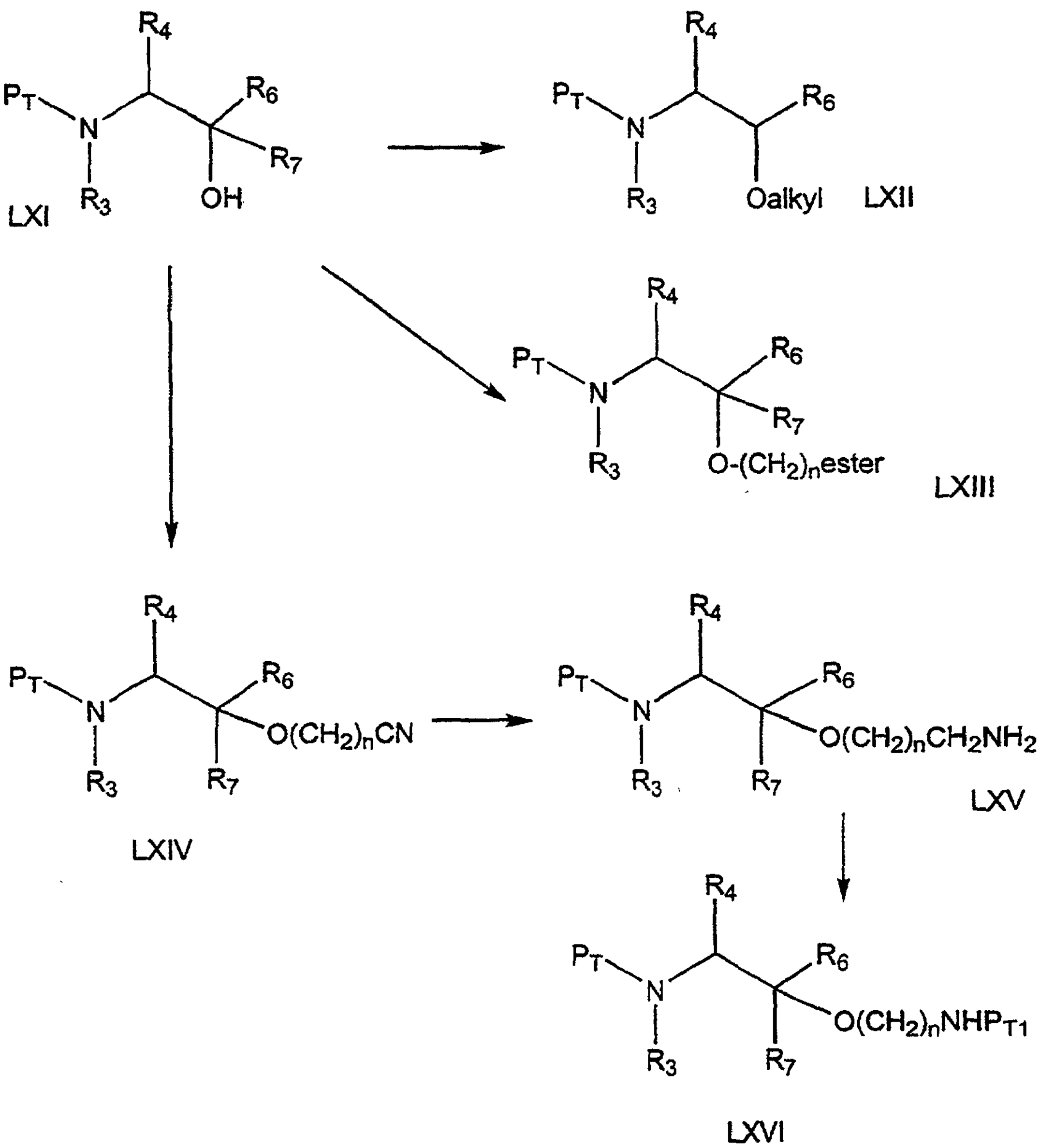

LXI LXII LXIII LXIV LXV LXVI

## SCHEME IX

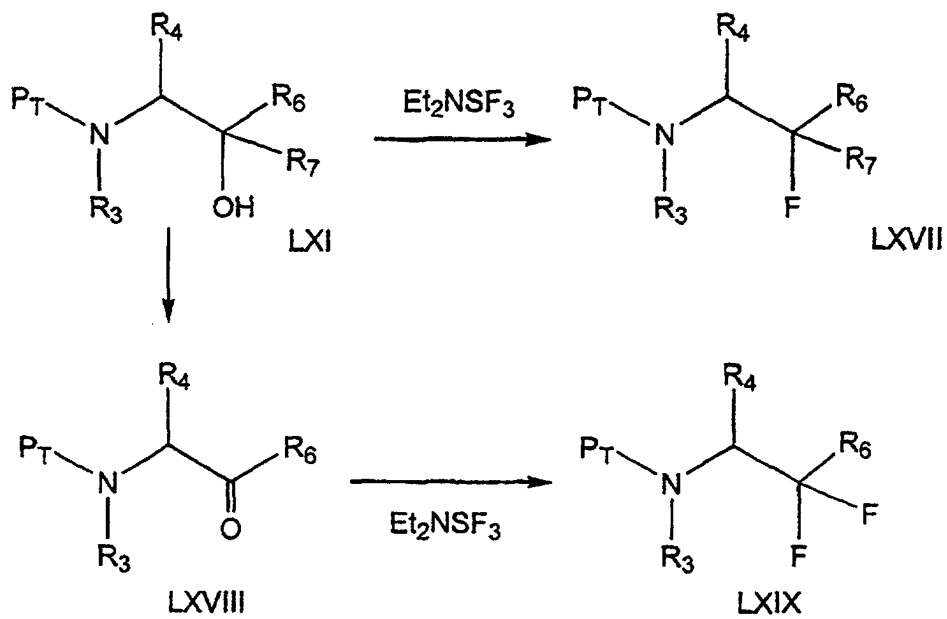


## SCHEME X

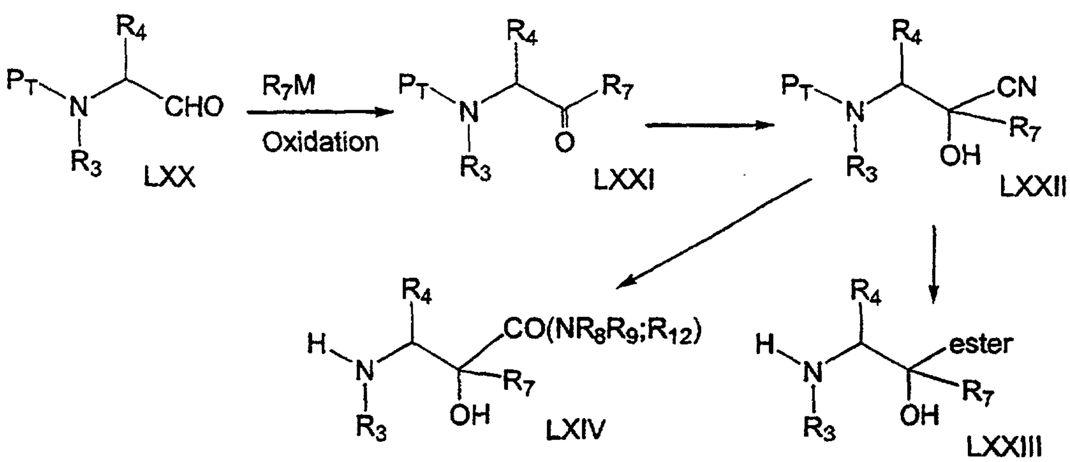


According to Reaction Scheme I the Formula I compounds, wherein $R_1$, $R_{10}$, $R_{11}$, A, $R_2$, $R_3$, $R_4$, $R_5$, $R_6$ and $R_7$ are as defined above may be prepared by either of two general processes. In the first process the desired Formula I compound may be prepared by coupling the appropriate Formula I indofe-2-carboxylic acid or indoline-2-carboxylic acid with the appropriate Formula III amine (i.e., acylating the amine). In the second process the desired Formula I compound may be prepared by coupling the appropriate Formula IV compound (i.e., a Formula I compound wherein $R_6$ is carboxy) with the appropriate alcohol or formula $R_8R_9NH$ or $R_{12}H$ amine or alcohol, wherein $R_8$, $R_9$ and $R_{12}$ are as defined above (i.e., acylating the amine or alcohol).

Typically, the Formula II compound is combined with the Formula III compound (or Formula IV compound is combined with the appropriate amine (e.g., $R_{12}H$ or $R_8R_9NH$)) or alcohol in the presence of a suitable coupling agent. A suitable coupling agent is one which transforms a carboxylic acid into a reactive species which forms an amide or ester linkage on reaction with an amine or alcohol, respectively.

The coupling agent may be a reagent which effects this condensation in a one pot process when mixed together with the carboxylic acid and amine or alcohol. If the acid is to be condensed with an alcohol it is preferable to

employ a large excess of the alcohol as the reaction solvent, with or without 1.0 to 1.5 equivalent added dimethylaminopyridine. Exemplary coupling reagents are 1-(3-dimethylaminopropyl)-3-ethylcarbodiimide hydrochloride-hydroxybenzotriazole (DEC/HBT), carbonyldiimidazole, dicyclohexylcarbodiimide/hydroxybenzotriazole (HBT), 2-ethoxy1-ethoxycarbonyl-1,2-dihydroquinoline (EEDQ), carbonyldiimidazole/HBT, and diethylphosphorylcyanide. The coupling is performed in an inert solvent, preferably an aprotic solvent at a temperature of about -20°C to about 50°C for about 1 to about 48 hours. Exemplary solvents include acetonitrile, dichloromethane, dimethylformamide and chloroform.

The coupling agent may also be that agent which converts the carboxylic acid to an activated intermediate which is isolated and/or formed in a first step and allowed to react with the amine or alcohol in a second step. Examples of such coupling agents and activated intermediates are thionyl chloride or oxalyl chloride to form the acid chloride, cyanuric fluoride to form an acid fluoride or an alkyl chloroformate such as isobutyl or isopropenyl chloroformate (with a tertiary amine base) to form a mixed anhydride of the carboxylic acid. If the coupling agent is oxalyl chloride it is advantageous to employ a small amount of dimethylformamide as cosolvent with another solvent (such as dichloromethane) to catalyze the formation of the acid chloride. Use of these coupling agents and appropriate selection of solvents and temperatures are known to those skilled in the art or can be readily determined from the literature. These and other exemplary conditions useful for coupling carboxylic acids are described in Houben-Weyl, Vol XV, part II, E. Wunsch, Ed., G. Theime Verlag, 1974, Stuttgart, and M. Bodansky, Principles of Peptide Synthesis, Springer-Verlag Berlin 1984, and The Peptides. Analysis , Synthesis and Biology (ed. E. Gross and J. Meienhofer), vols 1-5 (Academic Press NY 1979-1983).

The Formula IV compounds wherein $R_1$, $R_{10}$, $R_{11}$, A, $R_2$, $R_3$, $R_4$, $R_5$, and $R_7$ are as defined above may be prepared from the corresponding Formula V ester (i.e., Formula I compounds wherein $R_6$ is ($C_1$-$C_5$)alkoxycarbonyl or benzyloxycarbonyl) by hydrolysis with aqueous alkali at a temperature of about -20°C to about 100°C, typically at about 20°C, for about 30 minutes to about 24 hours.

Alternatively, Formula IV compounds are prepared by activation of a Formula II indole carboxylic add with a coupling agent (as described above) which gives an activated intermediate (such as an acid chloride, acid fluoride, or mixed anhydride) which is then allowed to react with a compound of Formula III wherein $R_3$, $R_4$, $R_5$, and $R_7$ are as described above and $R_6$ is carboxy, in a suitable solvent in the presence of a suitable base. Suitable solvents include water or methanol or a mixture thereof, together with a cosolvent such as dichloromethane, tetrahydrofuran, or dioxane. Suitable bases include sodium, potassium or lithium hydroxides, sodium or potassium bicarbonate, sodium or potassium carbonate, or potassium carbonate together with tetrabutyl ammonium bromide (1 equivalent) in sufficient quantity to consume the add liberated in the reaction (generally that quantity sufficient to maintain the pH of the reaction at greater than 8). The base may be added incrementally together with the activated intermediate to effect proper pH control of the reaction. The reaction is conducted generally between -20°C and 50°C. Isolation procedures are tailored by one skilled in the art to remove impurities, but typically consist of removal of water-miscible cosolvents by evaporation, extraction of impurities at high pH with an organic solvent, acidification to low pH (1-2) and filtration or extraction of the desired product with a suitable solvent such as ethyl acetate or dichloromethane.

The Formula V compound may be prepared by coupling the appropriate Formula III compound wherein $R_6$ is alkoxycarbonyl and the appropriate Formula II compound in an analogous procedure to that described above (e.g., Procedure A).

Alternatively, Formula I compounds which contain sulfur atoms in the sulfoxide or sulfone oxidation state may be prepared from the corresponding Formula I compounds having the sulfur atom in the unoxidized form, by treatment with a suitable oxidizing agent, such as with m-chloroperoxybenzoic acid in dichloromethane at a temperature of about 0°C to about 25°C for about 1 to about 48 hours using about 1 to about 1.3 equivalent for conversion to the sulfoxide oxidation state and greater than about 2 equivalents for conversion to the sulfone oxidation state.

Alternatively, the Formula I compounds that are mono- or di-alkylated on $R_5$ aminoalkoxy may be prepared from the corresponding Formula I compound wherein $R_5$ is aminoalkoxy by monoalkylation or dialkylation on the $R_5$ amine to prepare the desired Formula I compound. Such a mono- or di-alkylation may be conducted by treatment of the $R_5$ aminoalkoxy compound with 1 equivalent of the appropriate carbonyl compound (for monoalkylation) or greater than 2 equivalents of the appropriate carbonyl compound (for dialkylation) and a suitable reducing agent in a suitable solvent. Suitable reducing conditions include sodium cyanoborohydride or sodium borohydride in methanol or ethanol, or hydrogen/hydrogenation catalyst (such as palladium on carbon) in a polar solvent such as water, methanol, or ethanol at about 0°C to 60°C for 1 to 48 hours.

Alternatively, the Formula I compounds, wherein $R_5$ is alkanoyloxy (RCOO-), are prepared by O-acylation of the appropriate Formula I compound with an appropriate acid chloride or other activated acid derivative in the presence, if necessary, of a suitable base, (e.g., tertiary amine base such as trialkylamine or pyridine), preferably in an aprotic solvent such as tetrahydrofuran or dichloromethane, at a temperature of about 0°C to about 50°C, for about 0.5 to about 48 hours.

Altematively, the Formula I compounds wherein $R_5$ and $R_7$ are taken together to be oxo are prepared by

oxidizing a corresponding Formula I compound, for example, wherein $R_5$ is hydroxy and $R_7$ is H, with a suitable oxidizing agent. Exemplary oxidizing agents include the Dess-Martin reagent in dichloromethane, a carbodiimide and dimethylsulfoxide and add catalyst (Pfitzner-Moffatt conditions or modifications thereof, such as employing a water-soluble carbodiimide) or Swem-type reactions (e.g., oxalyl chloride/DMSO/triethylamine). The Formula I compounds having other oxidation sensitive functionality may benefit from appropriate protection and deprotection of such functionality.

For example, in Reaction Scheme I certain Formula I compounds contain primary amine, secondary amine or carboxylic add functionality in the part of the molecule defined by $R_5$ or $R_6$ which may interfere with the intended coupling reaction of Reaction Scheme I if the Formula III intermediate, or $R_{12}H$ or $R_8R_9NH$ amine is left unprotected. Accordingly, the primary or secondary amine functionality may be protected, where it is present in the $R_5$ or $R_6$ moieties of the Formula III intermediate or amine ($R_8R_9NH$ or $R_{12}H$) by an appropriate protecting group during the coupling reaction of Reaction Scheme I. The product of such coupling reaction is a Formula I compound containing the protecting group. This protecting group is removed in a subsequent step to provide the Formula I compound. Suitable protecting groups for amine and carboxylic acid protection include those protecting groups commonly used in peptide synthesis (such as N-t-butoxycarbonyl, N-carbobenzyloxy, and 9-fluorenylmethylenoxycarbonyl for amines and lower alkyl or benzyl esters for carboxylic acids) which are not chemically reactive under the coupling conditions described above (and immediately preceding the Examples herein as Procedure A) and can be removed without chemically altering other functionality in the Formula I compound.

The starting indole-2-carboxylic adds and indoline-2-carboxylic acids used in Reaction Scheme I, when not commercially available or known in the prior art (such art is extensively published), are available by conventional synthetic methods. For example, according to Reaction Scheme II the Formula VII indole ester may be prepared from the Formula VI compound (wherein Q is selected to achieve the desired A as defined above) via a Fischer Indole synthesis (see The Fischer Indole Synthesis Robinson, B. (Wiley, New York, 1982)) followed by saponification of the resulting Formula VII indole ester to yield the corresponding Formula VIII add. The starting aryl hydrazone may be prepared by condensation of a readily available hydrazine with the appropriate carbonyl derivative or via the Japp-Klingeman reaction (see Organic Reactions, Phillips, R. R., 1959, 10, 143).

Alternatively, the Formula VIIIA indole 2-carboxylic acid may be prepared by condensation of a Formula IX ortho methyl nitro compound with an oxalate ester to yield the Formula X indole ester followed by reduction of the nitro group and subsequent hydrolysis.

This three step process is known as the Reissert indole synthesis (Reissert, Chemische Berichte 1897, 30, 1030). Conditions for accomplishing this sequence, and references thereto, are described in the literature (Kermack, et al., J. Chem. Soc. 1921, 119, 1602; Cannon et al., J. Med. Chem. 1981, 24, 238; Julian, et al in Heterocyclic Compounds, vol 3 (Wiley, New York, NY, 1962, R.C. Elderfield, ed.) p 18). An example of the specific implementation of this sequence is Examples 10A-10C herein.

3-Halo-5-chloro-1H-indole-2-carboxylic adds may also be prepared by halogenation of 5-chloro-1H-indole-2-carboxylic acids.

Alternatively, (to Reaction Scheme II) the Formula XIV substituted indolines may be prepared by reduction of the corresponding Formula XV indoles with a reducing agent such as magnesium in methanol at a temperature of about 25°C to about 65°C for about 1 to about 48 hours (Reaction Scheme III).

Formula XVI indoline carboxylic acids are prepared by saponification of the corresponding Formula XVII ester (Reaction Scheme III). The Formula XVII compound is prepared by reduction of the corresponding Formula VII indole ester with a reducing agent such as magnesium in methanol as described for the conversion of the Formula XV compound to the Formula XIV compound above.

The following paragraphs describe how to prepare the various amines which are used in the above Reaction Schemes.

According to Reaction Scheme IV the Formula XXII compounds (the Formula III amines of Reaction Scheme I wherein $R_5$ is OH, $R_7$ is H and $R_6$ is an ester) or Formula XXVI compounds ($R_6$ is $C(O)NR_8R_9$ or $C(O)R_{12}$) are prepared starting from a Formula XX N-protected (denoted by $P_T$) aldehyde. The Formula XX aldehyde or the sodium bisulfite adduct of a Formula XX aldehyde is treated with potassium or sodium cyanide in aqueous solution with a cosolvent such as dioxane or ethyl acetate at a temperature of about 0°C to about 50°C to provide a Formula XXI cyanohydrin. The Formula XXI cyanohydrin is treated with an alcohol (e.g., ($C_1$-$C_6$)alkanol such as methanol) and a strong acid catalyst such as hydrogen chloride at a temperature of about 0°C to about 50°C, followed by addition of water, if necessary. The protecting group ($P_T$) is then removed, if still present, by an appropriate deprotection method yielding a Formula XXII compound. For example, if the Formula XX N-protecting group $P_T$ is tert-butoxycarbonyl (t-Boc), the Formula XXIII compound is directly formed from the Formula XXI compound, and addition of water is not necessary. The Formula XXII compound may be protected on nitrogen with an appropriate protecting group to form a Formula XXIII compound followed by hydrolysis of the ester with aqueous alkali at a temperature of about 0°C to about 50°C in a reaction-inert solvent resulting in the corresponding Formula XXIV hydroxy acid. The Formula XXIV compound is coupled (in an analogous procedure to the coupling process described in Reaction Scheme I) with an appropriate

$R_8R_9NH$ or $HR_{12}$ amine to form a Formula XXV compound, which is then deprotected resulting in the Formula XXVI compound (i.e., Formula III compound wherein $R_5$ is OH, $R_7$ is H and $R_6$ is $C(O)R_{12}$ or $C(O)NR_8R_9$. An example of the conversion of a Formula XXI cyanohydrin to the corresponding Formula XXII methyl ester with removal of the t-boc protecting group is provided in PCT publication WO/9325574, Example 1a. Other examples wherein a cyanohydrin is converted to Formula XXIII lower alkyl esters may be found in U.S. patent no. 4,814,342, and EPO publication 0438233.

Certain Formula I compounds are stereoisomeric by virtue of the stereochemical configuration at the carbons labeled a and b. One skilled in the art may prepare Formula XXII and XXVI intermediates with the desired stereochemistry according to Reaction Scheme IV. For example, the Formula XX aldehyde is available in either enantiomeric form (stereochemistry at a) by literature procedures outlined below (see Reaction Scheme V). The Formula XXI cyanohydrin may be prepared from the Formula XX compound by treatment with sodium or potassium cyanide as described above while maintaining the stereochemistry at carbon a resulting in a mixture of stereoisomers at carbon b.

The skilled chemist may employ crystallization at this stage to separate isomers or purify one isomer.

For example, the preparation of the Formula XXI compound wherein $P_T$ is Boc, $R_3$ is H, $R_4$ is benzyl and the stereochemistry of carbons a and b is (S) and (R) respectively, employing this route together with purification by recrystallization is described in Biochemistry 1992, 31, 8125-8141.

Alternatively, isomer separation may be effected by chromatography or recrystallization techniques after conversion of a compound of formula XXI (mixture of isomers) to a compound of formula XXII, XXIII, XXIV, XXV, XXVI, V, IV, or I by the procedures and/or sequences described herein. Formula XXI intermediates of a specific stereochemistry at carbons a and b are converted to Formula XXII intermediates with retention of this stereochemistry by treatment with an alcohol and a strong acid catalyst, followed by addition of water, if necessary, as described above.

Alternatively, the desired isomer of the Formula XXI compound may also be obtained by derivatization of the Formula XXI intermediate and chromatographic separation of the diastereomeric derivatives (for example with trimethylsilyl chloride (TMS) or t-butyldimethylsilyl chloride (TBDMS) to give O-TMS or O-TBDMS derivatives). A silyl derivative of a Formula XXI intermediate having a single stereoisomeric form at carbons a and b is converted with retention of stereochemistry, to a Formula XXII intermediate (if the silyl group is not removed in this step it is removed subsequently by an appropriate method, such as treatment with tetrabutylammonium fluoride in tetrahydrofuran), by the method described above for the conversion of the Formula XXI compound to the Formula XXII compound.

According to Reaction Scheme V the Formula XX aldehydes (starting materials for Reaction Scheme IV) are prepared from the corresponding Formula XXX amino acids. The Formula XXX amino acid is protected on nitrogen with a protecting group ($P_T$) (such as Boc). The protected compound is esterified with an alcohol and converted to an ester, preferably the methyl or ethyl ester of the Formula XXXI compound. This may be accomplished by treating the Formula XXX compound with methyl or ethyl iodide in the presence of a suitable base (e.g., $K_2CO_3$) in a polar solvent such as dimethylformamide. The Formula XXXI compound is reduced, for example, with diisobutylaluminum hydride in hexane or toluene, or a mixture thereof, at a temperature of about -78°C to about -50°C followed by quenching with methanol at -78°C as described in J. Med. Chem., 1985, 28, 1779-1790 to form the Formula XX aldehyde. Alternatively (not depicted in Reaction Scheme V), analogous N-methoxymethylamides corresponding to the Formula XXXI compound, wherein the alcohol substituent of the ester is replaced by N(OMe)Me, are formed from a Formula XXX compound, N,O-dimethylhydroxylamine and a suitable coupling agent (e.g., 1-(3-dimethylaminopropyl)3-ethylcarbodiimide hydrochloride (DEC). The resulting compound is reduced, for example, with lithium aluminum hydride in a reaction-inert solvent such as ether or tetrahydrofuran at a temperature of about 0°C to about 25°C to form the Formula XX aldehyde. This two-step method is general for the conversion of N-protected a-amino acids to Formula XX aldehydes (Fehrentz and Castro, Synthesis 1983, 676-678).

Alternatively Formula XX aldehydes may be prepared by oxidation of Formula XXXIII protected aminoalcohols, for example, with pyridine-$SO_3$ at a temperature of about -10°C to about 40°C in a reaction-inert solvent, preferably dimethylsulfoxide . Formula XXXIII protected aminoalcohols, if not commercially available, may be prepared by protection of Formula XXXII aminoalcohols. The Formula XXXII aminoalcohols are prepared by reduction of Formula XXX amino acids. This reduction is accomplished by treatment of Formula XXX compounds with lithium aluminum hydride according to the procedure described by Dickman et at., Organic Syntheses; Wiley: New York, 1990; Collect. Vol. VII, p 530, or with sulfuric acid-sodium borohydride by the procedure of Abiko and Masamune, Tetrahedron Lett. 1992 333, 5517-5518, or with sodium borohydride-iodine according to the procedure of McKennon and Meyers, J. Org. Chem. 1993, 58, 3568-3571, who also reviewed other suitable procedures for converting Formula XXX amino acids to Formula XXXII amino alcohols.

According to Reaction Scheme VI the Formula XXX compounds utilized in Reaction Scheme V may be prepared as follows. The Formula XLI amino acids may be prepared by N-alkylation of the Formula XL protected ($P_T$) amino acids by treatment with an appropriate base and alkylating agent. Specific procedures for this alkylation are described by Benoiton, Can. J. Chem 1977, 55, 906-910, and Hansen, J. Org. Chem. 1985, 50 945-950. For example, when $R_3$ is methyl, sodium hydride and methyl iodide in tetrahydrofuran are utilized. Deprotection of the Formula XLI

compound yields the desired Formula XXX compound.

Alternatively, a Formula XLII amino acid may be N-alkylated by a three-step sequence involving reductive benzylation (such as with benzaldehyde, Pd/C-catalyzed hydrogenation) to give the mono-N-benzyl derivative and reductive amination with the appropriate acyl compound (for example with formaldehyde and sodium cyanoborohydride to introduce $R_3$ as methyl) to give the N-Benzyl, N-$R_3$-substituted amino acid. The N-benzyl protecting group is conveniently removed (for example by hydrogenation with an appropriate catalyst) to yield the Formula XXX compound. Specific conditions for this three step alkylation procedure are described by Reinhold et al., J. Med. Chem., 1968, 11, 258-260.

The immediately preceding preparation may also be used to introduce an $R_3$ moiety into the Formula XLIV intermediate to form the Formula XLV intermediate (which is a Formula III intermediate wherein $R_7$ is OH). The immediately preceding preparation may also be used to introduce an $R_3$ moiety into a Formula IIIa intermediate (which is a Formula III intermediate wherein $R_3$ is H).

The amino acids used in the schemes herein (e.g., XL, XLII), if not commercially available, or reported in the literature, may be prepared by a variety of methods known to those skilled in the art. For example, the Strecker synthesis or variations thereof may be used. Accordingly, an aldehyde ($R_4CHO$), sodium or potassium cyanide and ammonium chloride react to form the corresponding aminonitrile. The aminonitrile is hydrolyzed with mineral acid to form the desired Formula XLII $R_4C(NH_2)COOH$ amino acid. Alternatively, the Bucherer-Berg method may be used wherein a hydantoin is formed by heating an aldehyde ($R_4CHO$) with ammonium carbonate and potassium cyanide followed by hydrolysis (for example, with barium hydroxide in refluxing dioxane) with acid or base to form the desired Formula XLII $R_4C(NH_2)COOH$ amino acid.

Other methods for synthesis of $\alpha$-amino acids are also reported in the literature which would permit one skilled in the art to prepare the desired Formula XLII $R_4C(NH_2)COOH$ intermediate necessary for the synthesis of Formula I compounds.

Suitable methods for the synthesis or resolution of Formula XLII compounds are found in reviews by Duthaler (Tetrahedron 1994, 50, 1539-1650), or by Williams (R. M. Williams, Synthesis of optically active amino acids. Pergamon: Oxford, U.K., 1989).

A specific method for the synthesis of a Formula XLII intermediate in either enantiomeric form from the corresponding $R_4X$ (X = Cl, Br, or I) intermediate is the procedure of Pirrung and Krishnamurthy (J. Org. Chem. 1993, 58, 957-958), or by the procedure of O'Donnell, et al. (J. Am. Chem. Soc. 1989, 111, 2353-2355). The required $R_4X$ intermediates are readily prepared by many methods familiar to the chemist skilled in the art. For example, those compounds when $R_4X$ is $ArCH_2X$ may be prepared by radical halogenation of the compound $ArCH_3$ or by formulation of the arene Ar-H and conversion of the alcohol to the bromide.

Another specific method for the synthesis of Formula XLII intermediates in either enantiomeric form is that of Corey and Link (J. Am. Chem. Soc. 1992, 114, 1906-1908). Thus, an intermediate of formula $R_4COCCl_3$ is reduced enantiospecifically to intermediate $R_4CH(OH)CCl_3$, which is converted on treatment with azide and base to an intermediate $R_4CH(N_3)COOH$, which is reduced by catalytic hydrogenation to the desired Formula XLII compound. The requisite trichloromethyl ketone $R_4COCCl_3$ is obtained by reaction of the aldehyde $R_4CHO$ with trichloromethide anion followed by oxidation (Gallina and Giordano, Synthesis 1989, 466-468).

Formula III intermediate amines (used in Reaction Scheme I), wherein $R_5$ and $R_7$ are H may be prepared according to Reaction Scheme VII. A Formula L amino acid (suitably protected ($P_T$) is activated by conversion to the acid chloride, fluoride or mixed anhydride (e.g., with isobutyl chloroformate and triethylamine in an inert solvent such as tetrahydrofuran or dioxane at about -0°C to about -40°C) and the activated intermediate treated with diazomethane to give the Formula LI diazoketone. The Formula LI diazoketone is treated with an alcohol (ROH) (e.g., ($C_1$-$C_6$)alkanol such as methanol), and a suitable catalyst such as heat, silver oxide or silver benzoate to prepare the Formula LII ester. The Formula LII ester is deprotected to form the Formula IIIA compound (via Wolff rearrangement). Alternatively the Formula LII ester is hydrolyzed, with for example alkali, and coupled with the appropriate $R_{12}H$ or $HNR_8R_9$ amine to prepare the Formula IIIB compound as described previously.

According to Reaction Scheme VIII the Formula III intermediate amines wherein $R_5$ is an oxygen linked substituent (e.g., alkoxy) (used in Reaction Scheme I) may be prepared as follows. The Formula LXI compound is alkylated on oxygen by treatment with an appropriate alkylating agent (e.g., alkyliodide, alkylbromide, alkylchloride or alkyltosylate) and sufficient base to form the alkoxide (sodium or potassium hydride) in a suitable polar aprotic solvent (e.g., dimethylformamide or tetrahydrofuran) at a temperature of about 0°C to about 150°C resulting in a Formula LXII compound. The Formula LXII compound is deprotected to afford the desired amine intermediate.

The Formula III intermediate amines wherein $R_5$ is ($C_1$-$C_6$) alkoxycarbonylalkoxy (used in Reaction Scheme I) may be prepared as follows. The Formula LXI compound is alkylated with a halo-alkanoate ester to form a Formula LXIII compound which is then deprotected to form the desired amine. The corresponding acid may be prepared by hydrolysis of the ester using aqueous alkali in an appropriate solvent Those Formula III amines wherein $R_6$ contains an ester and $R_5$ contains a carboxy may be prepared from the Formula LXIII amine (as prepared above in this para-

graph), wherein $R_5$ contains the carboxylic acid functionality protected as the t-butyl ester by treatment with anhydrous acid to provide the corresponding acid at $R_5$ without hydrolyzing the ester at the $R_6$ position. The Formula LXVI compounds (Formula III intermediate amines wherein $R_5$ is protected aminoalkoxy) may be prepared from the Formula LXI compound. The Formula LXI compound is alkylated with a halo-alkane-nitrile to form the Formula LXIV compound. The Formula LXIV compound is reduced to the primary amine by treatment with hydrogen and an appropriate catalyst (e.g., rhodium-on-carbon) in the presence of ammonia in preferably a polar, protic solvent such as water, methanol or ethanol to give the Formula LXV primary amine. The Formula LXV compound is protected on nitrogen with a protecting group ($P_{T1}$), which is orthogonal to the other protecting group ($P_T$), followed by deprotection of the $P_T$ protecting group to yield the desired Formula III compound. The protected Formula III compound is coupled with the appropriate Formula II compound and the resulting protected Formula I compound is deprotected.

The Formula LXIII and LXIV compounds wherein n is two are preferably prepared by treatment of the Formula LXI compound with an excess of acrylate ester or acrylonitrile, respectively, in the presence of a suitable base, such as potassium or sodium hydroxide, in a suitable solvent, preferably a polar protic solvent.

According to Reaction Scheme IX the Formula LXVII and Formula LXIX compounds (Formula III compounds wherein $R_5$ is F or $R_5$ and $R_7$ are both F) may be prepared from the Formula LXI compound. The Formula LXI compound is treated with a suitable fluorinating agent such as diethylaminosulfur trifluoride in a reaction-inert solvent such as an aprotic solvent, preferably dichloromethane, to form the Formula LXVII compound. The Formula LXVII compound is conveniently deprotected.

The Formula LXI compound is oxidized to the Formula LXVIII compound utilizing the conditions described above for the preparation of the Formula I compounds wherein $R_5$ and $R_7$ together form oxo. The Formula LXVIII compound is difluorinated under suitable conditions (e.g., diethylaminosulfur trifluoride in dichloromethane).

According to Reaction Scheme X the Formula LXXIII compound or Formula LXIV compound wherein $R_7$ is alkyl (i.e., Formula III compound wherein $R_7$ is alkyl) are prepared from the Formula LXX compound (also see Reaction Scheme V for analogous amine preparation). The Formula LXX compound is treated with an organometallic reagent $R_7M$ and the resulting secondary alcohol oxidized as in the directly preceding paragraph to form the Formula LXXI compound. The Formula LXXI compound is converted via the Formula LXXII cyanohydrin to the Formula LXXIII compound using the same conditions that are used to convert the Formula XXI compound to the Formula XXII compound in Reaction Scheme IV.

Alternatively, the Formula LXXII compound is converted to the Formula LXIV compound as described for the conversion of the cyano intermediate to the amide in Reaction Scheme V.

A compound of the formula $R_8NH_2$ or $R_9NH_2$ is monoalkylated with a carbonyl compound corresponding to $R_8$ or $R_9$, respectively, under appropriate reductive amination conditions, to give a formula $R_8R_9NH$ amine. To avoid dialkylation, it may be preferable to protect the amines ($R_8NH_2$ or $R_9NH_2$) with a suitable protecting group $P_T$ to give $R_8(P_T)NH$ or $R_9(P_T)NH$, for example by reaction with benzaldehyde and a reducing agent. The protected amines are monoalkylated with a carbonyl compound corresponding to $R_9$ or $R_8$ respectively, under suitable reductive amination conditions, to give $R_8R_9N(P_T)$. The protecting group ($P_T$) is removed (e.g. by exhaustive catalytic hydrogenation when $P_T$ is benzyl) to give a compound of formula $R_8R_9NH$. Appropriate reductive amination conditions are available from the literature to one skilled in the art. These conditions include those reported by Borch et al. (J. Am. Chem. Soc. 1971, 2897-2904) and those reviewed by Emerson (Organic Reactions, Wiley: New York, 1948 (14), 174), Hutchins et al. (Org. Prep. Proced. Int 1979 (11), 20, and Lane et al. (Synthesis 1975, 135). Reductive amination conditions favoring N-monoalkylation include those reported by Morales, et al. (Synthetic Communications 1984, 1213-1220) and Verardo et al. (Synthesis 1992 121-125). The $R_8NH_2$ or $R_9NH_2$ amines may also be monoalkylated with $R_9X$ or $R_8X$, respectively, where X is chloride, bromide, tosylate or mesylate. Alternatively, an intermediate of formula $R_8(P_T)NH$ or $R_9(P_T)NH$ may be alkylated with $R_9X$ or $R_8X$, and the protecting group removed to give a compound of formula $R_8R_9NH$.

Additional methods may be used to prepare formula $R_8R_9NH$ amines wherein $R_8$-NH or $R_9$-NH are oxygen-nitrogen linked. Thus a readily available compound of formula ($C_1$-$C_4$)alkoxycarbonyl-NHOH or $NH_2$CONHOH is dialkylated on nitrogen and oxygen by treatment with base and excess suitable alkylating agent (R-X) to give the corresponding ($C_1$-$C_4$)alkoxycarbonyl-N(R)OR which is then hydrolyzed to give a compound of formula $R_8R_9NH$ (wherein $R_8$=$R_9$=R). Suitable conditions, base, and alkylating agent include those described by Goel and Krolls (Org. Prep. Proced. Int. 1987, 19, 75-78) and Major and Fleck (J. Am. Chem. Soc. 1928, 50, 1479). Alternatively, a formula $NH_2$CONH(OH) amine may be sequentially alkylated, first on oxygen to give $NH_2$CONH(OR'), then on nitrogen to give $NH_2$CON(R")(OR'), by successive treatment with the alkylating agents R'X and R"X, respectively, in the presence of a suitable base. Suitable base and alkylating agents include those described by Kreutzkamp and Messinger (Chem. Ber. 100, 3463-3465 (1967) and Danen et al (J. Am. Chem. Soc. 1973, 95, 5716-5724). Hydrolysis of these alkylated hydroxyurea derivatives yields the amines R'O$NH_2$ and R'ONHR", which correspond to certain formula $R_8R_9NH$ amines. The chemist skilled in the art can adapt the procedures described in this paragraph to other alkylating agents R, R' and R"-X to prepare other amines of formula $R_8R_9NH$ wherein $R_8$-N or $R_9$-N are oxygen-nitrogen linked. Uno et al (SynLett 1991, 559-560) describe the $BF_3$-catalyzed addition of an organometallic reagent R-Li to an O-alkyl oxime

of formula R'CH=N-OR", to give compounds of formula R'RCH-NH(OR"). This route may also be used to give compounds of formula $R_8R_9NH$ wherein one of $R_8$-NH or $R_9$-NH are oxygen-nitrogen linked.

Prodrugs of this invention where a carboxyl group in a carboxylic add of Formula I is replaced by an ester may be prepared by combining the carboxylic acid with the appropriate alkyl halide in the presence of a base such as potassium carbonate in an inert solvent such as dimethylformamide at a temperature of about 0°C to 100°C for about 1 to about 24 hours. Altematively the acid is combined with appropriate alcohol as solvent in the presence of a catalytic amount of acid such as concentrated sulfuric acid at a temperature of about 20°C to 120°C, preferably at reflux, for about 1 hour to about 24 hours. Another method is the reaction of the acid with a stoichiometric amount of the alcohol in the presence of a catalytic amount of acid in an inert solvent such as tetrahydrofuran, with concomitant removal of the water being produced by physical (e.g. Dean-Stark trap) or chemical (e.g. molecular sieves) means.

Prodrugs of this invention where an alcohol function has been derivatized as an ether may be prepared by combining the alcohol with the appropriate alkyl bromide or iodide in the presence of a base such as potassium carbonate in an inert solvent such as dimethylformamide at a temperature of about 0°C to 100°C for about 1 to about 24 hours. Alkanoylaminomethyl ethers may be obtained by reaction of the alcohol with a bis-(alkanoylamino)methane in the presence of a catalytic amount of acid in an inert solvent such as tetrahydrofuran, according to a method described in US 4,997, 984. Alternatively, these compounds may be prepared by the methods described by Hoffman et al. in J. Org. Chem. 1994, 59, 3530.

The dialkylphosphate esters may be prepared by reaction of the alcohol with a dialkyl chlorophosphate in the presence of a base in an inert solvent such as tetrahydrofuran. The dihydrogen phosphates may be prepared by reaction of the alcohol with a diaryl or dibenzyl chlorophosphate as described above, followed by hydrolysis or hydrogenation in the presence of a noble metal catalyst, respectively.

Glycosides are prepared by reaction of the alcohol and a carbohydrate in an inert solvent such as toluene in the presence of acid. Typically the water formed in the reaction is removed as it is being formed as described above. An alternate procedure is the reaction of the alcohol with a suitably protected glycosyl halide in the presence of base followed by deprotection.

N-(1-hydroxyalkyl) amides, N-(1-hydroxy-1-(alkoxycarbonyl)methyl) amides or compounds where $R_2$ has been replaced by C(OH)C(O)OY may be prepared by the reaction of the parent amide or indole with the appropriate aldehyde under neutral or basic conditions (e.g. sodium ethoxide in ethanol) at temperatures between 25 and 70°C. N-alkoxymethyl indoles or N-1-(alkoxy)alkyl indoles can be obtained by reaction of the N-unsubstituted indole with the necessary alkyl halide in the presence of a base in an inert solvent. 1-(N,N-dialkylaminomethyl) indole, 1-(1-(N,N-dialkylamino)ethyl) indole and N,N-dialkylaminomethyl amides (e.g. $R_3 = CH_2N(CH_3)_2$) may be prepared by the reaction of the parent N-H compound with the appropriate aldehyde and amine in an alcoholic solvent at 25 to 70°C.

Cyclic prodrugs (e.g., the prodrugs of this invention where $R_2$ and $R_3$ are a common carbon) may be prepared by reaction of the parent compound (drug) with an aldehyde or ketone or its dimethyl acetal in an inert solvent in the presence of a catalytic amount of add with concomitant water or methanol removal. Altematively, these compounds may be prepared by reaction of the amino alcohol or hydroxy amide with a gem-dibromo alkane in the presence of base (e.g. potassium carbonate) in an inert solvent (e.g. dimethylformamide).

The Formula IA compounds may be prepared as described below.

The scheme numbers and formula numbers mentioned after this point of the text refer to scheme numbers and formula numbers appearing after this point in the text (i.e., they are not to be confused with the previous discussion).

## SCHEME XI

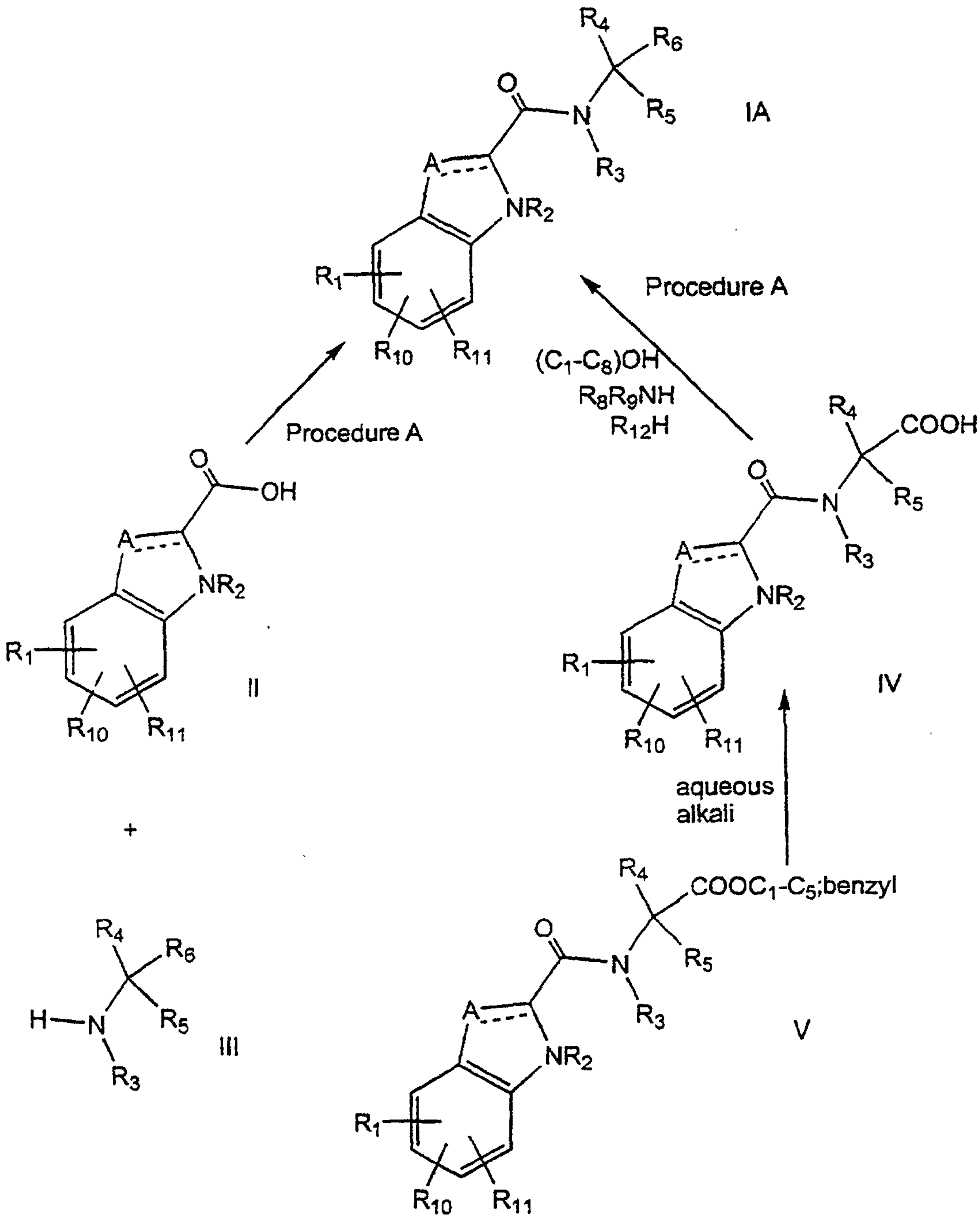

## SCHEME XII

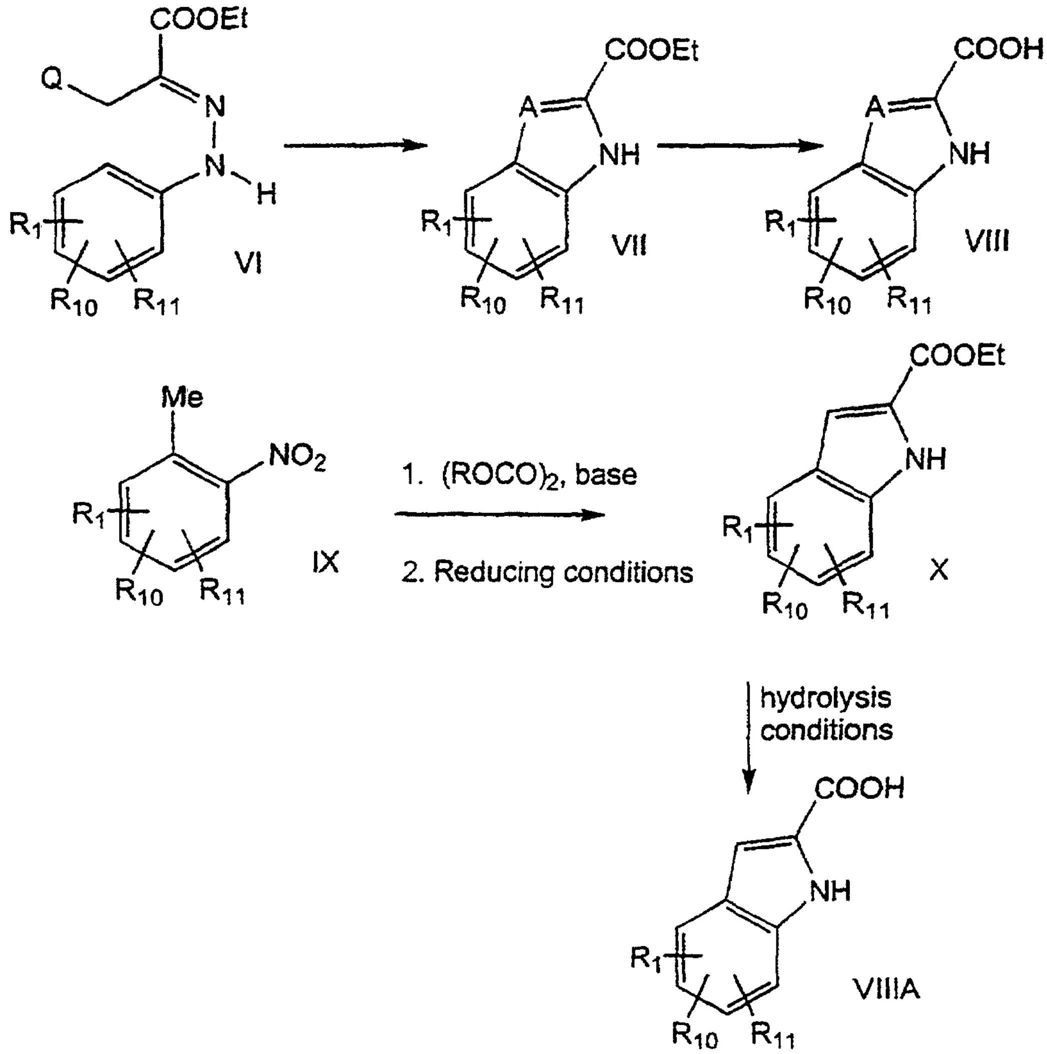


VI, VII, VIII, IX, X, VIIIA

## SCHEME XIII

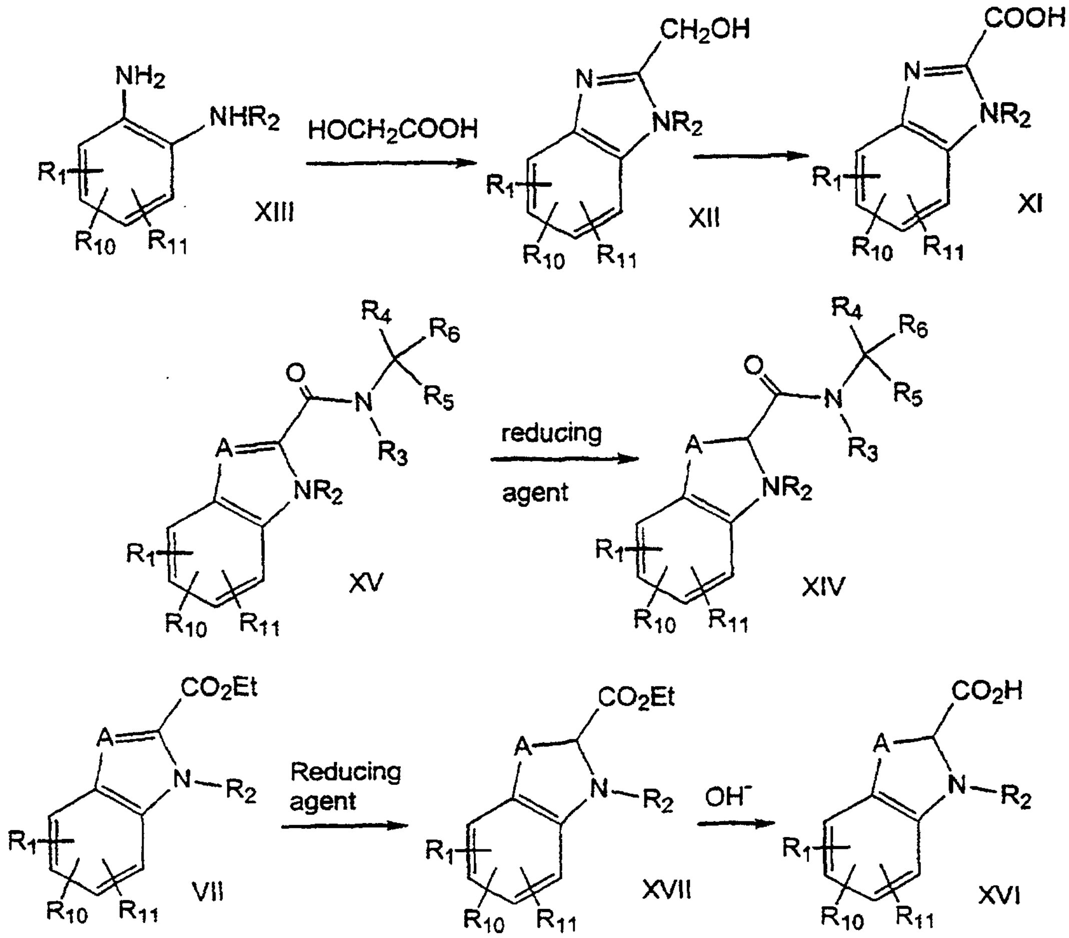


XIII XII XI

XV XIV

VII XVII XVI

## SCHEME XIV

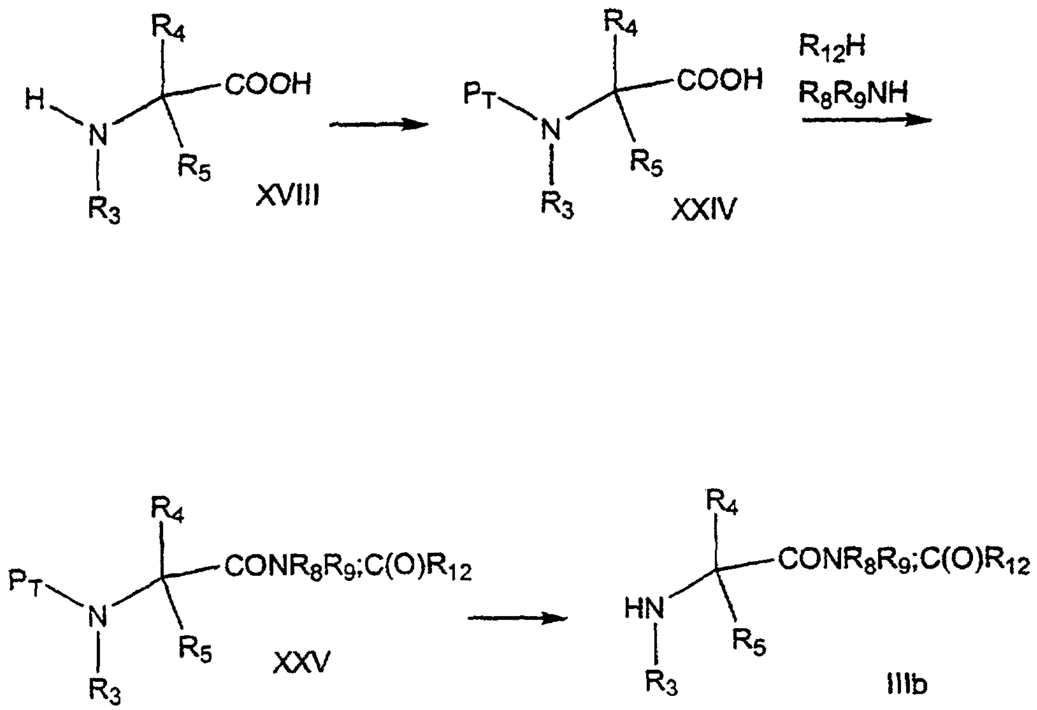


XVIII

XXIV

XXV

IIIb

## SCHEME XV

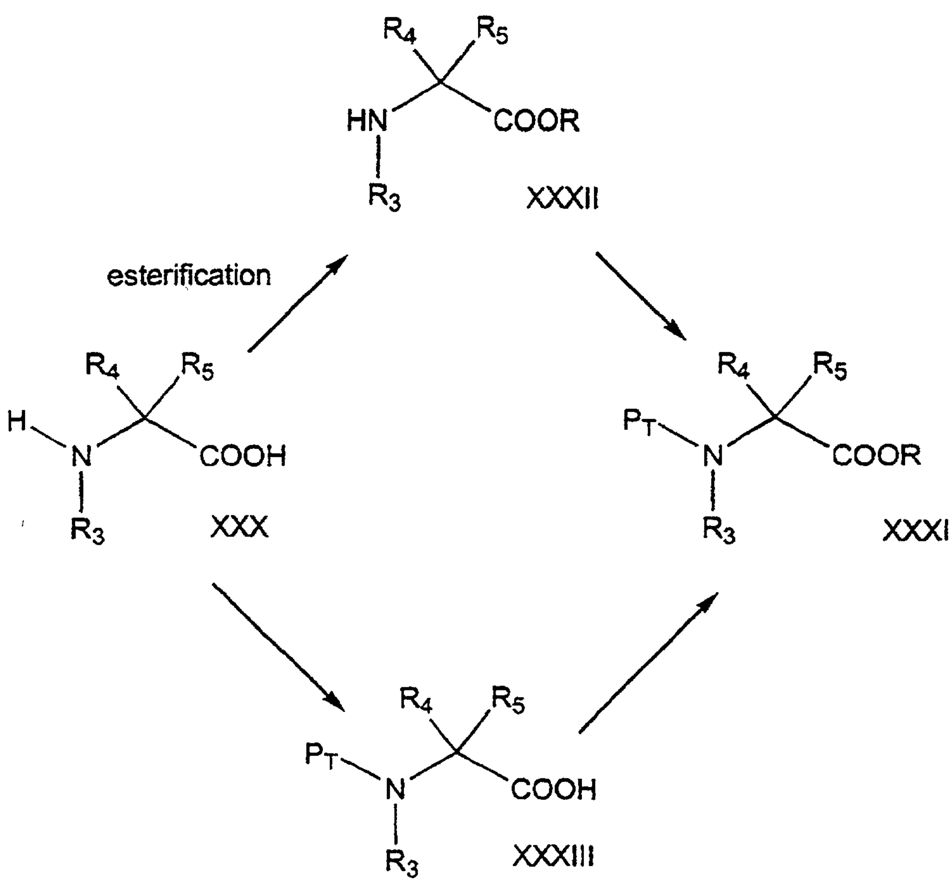

SCHEME XVI

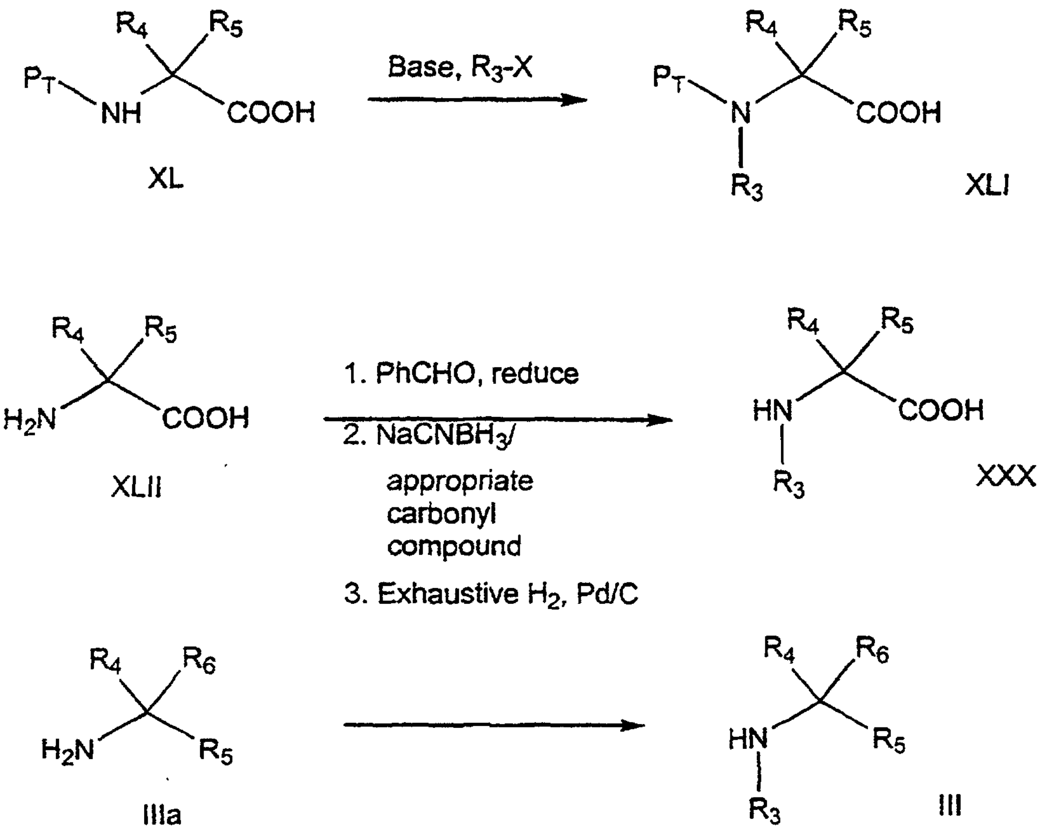


According to Reaction Scheme XI the Formula IA compounds, wherein $R_1$, $R_{10}$, $R_{11}$, A, $R_2$, $R_3$, $R_4$, $R_5$ and $R_6$ are as defined above may be prepared by either of two general processes. In the first process the desired Formula IA compound may be prepared by coupling the appropriate Formula II indole-2-carboxylic acid, indoline-2-carboxylic acid or benzimidazole-2-carboxylic acid with the appropriate Formula III amine (i.e., acylating the amine). In the second process the desired Formula IA compound may be prepared by coupling the appropriate Formula IV compound (i.e., a Formula IA compound wherein $R_6$ is carboxy) with the appropriate alcohol or formula $R_8R_9NH$ or $R_{12}H$ amine, wherein $R_8$, $R_9$ and $R_{12}$ are as defined above (i.e., acylating the amine or alcohol). The first process (coupling Formula II compounds with Formula III compounds is typically preferred when $R_4$ is not H and $R_5$ is H.

Typically, the Formula II compound is combined with the Formula III compound (or Formula IV compound is combined with the appropriate amine (e.g., $R_{12}H$ or $R_8R_9NH$)) or alcohol in the presence of a suitable coupling agent. A suitable coupling agent is one which transforms a carboxylic add into a reactive species which forms an amide or ester linkage on reaction with an amine or alcohol, respectively.

The coupling agent may be a reagent which effects this condensation in a one pot process when mixed together with the carboxylic acid and amine or alcohol. If the add is to be condensed with an alcohol it is preferable to employ a large excess of the alcohol as the reaction solvent, with or without 1.0 to 1.5 equivalent added dimethylaminopyridine. Exemplary coupling reagents are 1-(3-dimethylaminopropyl)-3-ethylcarbodiimide hydrochloride-hydroxybenzotriazole (DEC/HBT), carbonyldiimidazole, dicydohexylcarbodiimide/hydroxybenzotriazole (HBT), 2-ethoxy1-ethoxycarbonyl-1,2-dihydroquinoline (EEDQ), carbonyldiimidazole/HBT, propanephosphonic anhydride (propanphosphonic acid anhydride, PPA) and diethylphosphorylcyanide. The coupling is performed in an inert solvent, preferably an aprotic solvent at a temperature of about -20°C to about 50°C for about 1 to about 48 hours, in the optional presence of a tertiary amine base such as triethylamine. Exemplary solvents include acetonitrile, dichloromethane, ethyl acetate, dimethylformamide and chloroform or mixtures thereof.

The coupling agent may also be that agent which converts the carboxylic acid to an activated intermediate which is isolated and/or formed in a first step and allowed to react with the amine or alcohol in a second step. Examples of such coupling agents and activated intermediates are thionyl chloride or oxalyl chloride to form the acid chloride,

cyanuric fluoride to form an add fluoride or an alkyl chloroformate such as isobutyl or isopropenyl chloroformate (with a tertiary amine base) to form a mixed anhydride of the carboxylic acid. If the coupling agent is oxalyl chloride it is advantageous to employ a small amount of dimethylformamide as cosolvent with another solvent (such as dichloromethane) to catalyze the formation of the add chloride. This acid chloride may be coupled by mixing with the Formula III intermediate in an appropriate solvent together with an appropriate base. Appropriate solvent/base combinations are for example, dichloromethane, dimethylformamide or acetonitrile or mixtures thereof in the presence of a tertiary amine base e.g., triethylamine. Other appropriate solvent/base combinations include water or a ($C_1$-$C_5$)alcohol or a mixture thereof together with a cosolvent such as dichloromethane, tetrahydrofuran or dioxane and a base such as sodium or potassium carbonate, sodium potassium of lithium hydroxide or sodium bicarbonate in sufficient quantity to consume the acid liberated in the reaction. Use of a phase transfer catalyst (typically 1 to 10 mole %) such as a quaternary ammonium halide (e.g. tetrabutylammonium bromide or methyl trioctylammonium chloride) is advantageous when a mixture of only partially miscible cosolvents is employed (e.g. dichloromethane-water or dichloromethane-rnethanol). Use of these coupling agents and appropriate selection of solvents and temperatures are known to those skilled in the art or can be readily determined from the literature. These and other exemplary conditions useful for coupling carboxylic acids are described in Houben-Weyl, Vol XV, part II, E. Wunsch, Ed., G. Theime Verlag, 1974, Stuttgart, and M. Bodansky, Principles of Peptide Synthesis, Springer-Verlag Berlin 1984, and The Peptides. Analysis, Synthesis and Biology (ed. E. Gross and J. Meienhofer), vols 1-5 (Academic Press NY 1979-1983).

The Formula IV compounds wherein $R_1$, $R_{10}$, $R_{11}$, A, $R_2$, $R_3$, $R_4$ and $R_5$ are as defined above may be prepared from the corresponding Formula V ester (i.e., Formula IA compounds wherein $R_6$ is ($C_1$-$C_5$)alkoxycarbonyl or benzyloxycarbonyl) by hydrolysis with aqueous alkali at a temperature of about -20°C to about 100°C, typically at about 20°C, for about 30 minutes to about 24 hours.

Alternatively, Formula IV compounds are prepared by activation of a Formula II indole carboxylic acid with a coupling agent (as described above) which gives an activated intermediate (such as an acid chloride, acid fluoride, or mixed anhydride) which is then allowed to react with a compound of Formula III wherein $R_3$, $R_4$ and $R_5$, are as described above and $R_6$ is carboxy, in a suitable solvent in the presence of a suitable base. Suitable solvents include water, or methanol or a mixture thereof, together with a cosolvent such as dichloromethane, tetrahydrofuran, or dioxane. Suitable bases include sodium, potassium or lithium hydroxides, sodium or potassium bicarbonate, sodium or potassium carbonate, or potassium carbonate together with tetrabutyl ammonium bromide (1 equivalent) in sufficient quantity to consume the acid liberated in the reaction (generally that quantity sufficient to maintain the pH of the reaction at greater than 8). The base may be added incrementally together with the activated intermediate to effect proper pH control of the reaction. The reaction is conducted generally between -20°C and 50°C. Isolation procedures are tailored by one skilled in the art to remove impurities, but typically consist of removal of water-miscible cosolvents by evaporation, extraction of impurities at high pH with an organic solvent, acidification to low pH (1-2) and filtration, or extraction of the desired product with a suitable solvent such as ethyl acetate or dichloromethane.

The Formula V compound may be prepared by coupling the appropriate Formula III compound wherein $R_6$ is alkoxycarbonyl and the appropriate Formula II compound in an analogous procedure to that described above.

Alternatively, Formula IA compounds which contain sulfur atoms in the sulfoxide or sulfone oxidation state may be prepared from the corresponding Formula IA compounds having the sulfur atom in the unoxidized form, by treatment with a suitable oxidizing agent, such as m-chloroperoxybenzoic acid in dichloromethane at a temperature of about 0°C to about 25°C for about 1 to about 48 hours using about 1 to about 1.3 equivalent for conversion to the sulfoxide oxidation state and greater than about 2 equivalents for conversion to the sulfone oxidation state.

For example, in Reaction Scheme XI certain Formula IA compounds contain primary amine, secondary amine or carboxylic acid functionality in the part of the molecule defined by $R_6$ which may interfere with the intended coupling reaction of Reaction Scheme XI, if the Formula III intermediate or $R_{12}$H or $R_8R_9$NH amine is left unprotected. Accordingly, the primary amine, secondary amine or carboxylic acid functionality may be protected, where it is present in the $R_6$ moieties of the Formula III intermediate $R_8R_9$NH or $R_{12}$H amine by an appropriate protecting group during the coupling reaction of Reaction Scheme XI. The product of such coupling reaction in such a case is a Formula IA compound containing the protecting group. This protecting group is removed in a subsequent step to provide the Formula IA compound. Suitable protecting groups for amine and carboxylic acid protection indude those protecting groups commonly used in peptide synthesis (such as N-t-butoxycarbonyl, N-carbobenzyloxy, and 9-fluorenylmethylenoxycarbonyl for amines and lower alkyl or benzyl esters for carboxylic acids) which are not chemically reactive under the coupling conditions described above and can be removed without chemically altering other functionality in the Formula IA compound.

The starting indole-2-carboxylic acids and indoline-2-carboxylic acids used in Reaction Scheme XI, when not commercially available or known in the prior art (such art is extensively published), are available by conventional synthetic methods. For example, according to Reaction Scheme XII the Formula VII indole ester (wherein A is not nitrogen) may be prepared from the Formula VI compound (wherein Q is selected to achieve the desired A as defined above, except for N) via a Fischer Indole synthesis (see The Fischer Indole Synthesis Robinson, B. (Wiley, New York, 1982))

followed by saponification of the resulting Formula VII indole ester to yield the corresponding Formula VIII add. The starting aryl hydrazone may be prepared by condensation of a readily available hydrazine with the appropriate carbonyl derivative or via the Japp-Klingeman reaction (see Organic Reactions, Phillips, R. R., 1959, 10, 143).

Alternatively, the Formula VIIIA indole 2-carboxylic acid may be prepared by condensation of a Formula IX ortho methyl nitro compound with an oxalate ester to yield the Formula X indole ester followed by reduction of the nitro group and subsequent hydrolysis.

This three step process is known as the Reissert indole synthesis (Reissert, Chemische Berichte 1897, 30, 1030). Conditions for accomplishing this sequence, and references thereto, are described in the literature (Kermack, et al., J. Chem. Soc. 1921, 119, 1602; Cannon et al., J. Med. Chem. 1981, 24, 238; Julian, et al in Heterocyclic Compounds, vol 3 (Wiley, New York, NY, 1962, R.C. Elderfield, ed.) p 18).

3-Halo-5-chloro-1H-indole-2-carboxylic acids may also be prepared by halogenation of 5-chloro-1 H-indole-2-carboxylic acids.

According to Reaction Scheme XIII the Formula XI benzimidazole-2-carboxylic acid intermediates may be prepared by condensation of a Formula XIII ortho-diamino compound with glycolic acid, followed by oxidation of the resulting Formula XII benzimidazole-2-methanol (Bistrzycki, A. and Przeworski, G. Ber. 1912, 45, 3483). Alternatively, (to Reaction Scheme XII) the Formula XIV substituted indolines may be prepared by reduction of the corresponding Formula XV indoles with a reducing agent such as magnesium in methanol at a temperature of about 25°C to about 65°C for about 1 to about 48 hours (Reaction Scheme III).

Formula XVI indoline carboxylic acids are prepared by saponification of the corresponding Formula XVII ester (Reaction Scheme XIII). The Formula XVII ester is prepared by reduction of the corresponding Formula VII indole ester with a reducing agent such as magnesium in methanol as described for the conversion of the Formula XV compound to the Formula XIV compound above.

The following paragraphs describe ways to prepare the various amines which are used in the above Reaction Schemes.

According to Reaction Scheme XIV a Formula XXIII alpha-amino add may be protected on nitrogen with an appropriate protecting group ($P_t$) (e.g., t-Boc) to form a Formula XXIV compound. One skilled in the art can readily select an appropriate protecting group and a method for its introduction. For example, two common protecting groups are t-Boc (introduced by treating the amino acid with di-t-butyldicarbonate in a preferably protic suitable solvent or solvent mixture at high pH) and CBZ (introduced by treating the amino acid with benzylchloroformate in a suitable, preferably protic solvent or solvent mixture and base). The Formula XXIV compound is coupled (in an analogous procedure to the coupling process described in Reaction Scheme XI) with an appropriate $R_8R_9NH$ or $HR_{12}$ amine to form a Formula XXV compound, which is then deprotected resulting in the Formula IIIb compound (i.e., Formula III compound wherein $R_6$ is $C(O)R_{12}$ or $C(O)NR_8R_9$). If the protecting group is t-Boc by treatment of the Formula XXV compound with an acid in a suitable, preferably aprotic, solvent. Acids for this deprotection include HCl, $MeSO_3H$ or trifluoracetic acid.

According to Reaction Scheme XV a Formula XXXI compound (N-protected Formula III amine where $R_6$ is ($C_1$-$C_8$)alkoxycarbonyl or benzyloxycarbonyl) may be prepared from the corresponding Formula XXX unprotected amino acid via N-protection (yielding a Formula XXXIII protected amino acid) followed by esterification. For example, the Formula XXXIII compound may be esterified with the appropriate alcohol and an add catalyst such as hydrogen chloride or thionyl chloride, or in the case of tert-butanol by treatment of the amino acid with isobutylene and an acid catalyst such as concentrated sulfuric acid or by treatment with an alkyl halide (e.g., methyl iodide) and base (e.g., potassium carbonate). Alternatively, the esterification may precede the protection step.

According to Reaction Scheme XVI the Formula XXX compounds wherein $R_3$ is not H utilized in Reaction Scheme V may be prepared as follows. The Formula XLI amino acids may be prepared by N-alkylation of the Formula XL protected ($P_T$) amino acids by treatment with an appropriate base and alkylating agent. Specific procedures for this alkylation are described by Benoiton, Can. J. Chem 1977, 55, 906-910, and Hansen, J. Org. Chem. 1985, 50 945-950. For example, when $R_3$ is methyl, and $P_T$ is Boc, sodium hydride and methyl iodide in tetrahydrofuran are utilized. Deprotection of the Formula XLI compound yields the desired Formula XXX compound.

Alternatively, a Formula XLII amino add may be N-alkylated by a three-step sequence involving reductive benzylation (such as with benzaldehyde, Pd/C-catalyzed hydrogenation) to give the mono-N-benzyl derivative and reductive amination with the appropriate carbonyl compound (for example with formaldehyde and sodium cyanoborohydride to introduce $R_3$ as methyl) to give the N-Benzyl, N-$R_3$-substituted amino acid. The N-benzyl protecting group is conveniently removed (for example by hydrogenation with an appropriate catalyst) to yield the Formula XXX compound. Specific conditions for this three step alkylation procedure are described by Reinhold et al., J. Med. Chem., 1968, 11, 258-260.

The immediately preceding preparation may also be used to introduce an $R_3$ moiety into a Formula IIIa intermediate (which is a Formula III intermediate wherein $R_3$ is H).

The amino acids used in the schemes herein (e.g., XL, XLII), if not commercially available, or reported in the

literature, may be prepared by a variety of methods known to those skilled in the art. For example, the Strecker synthesis or variations thereof may be used. Accordingly, an aldehyde ($R_4CHO$), sodium or potassium cyanide and ammonium chloride react to form the corresponding aminonitrile. The aminonitrile is hydrolyzed with mineral add to form the desired Formula XLII $R_4C(NH_2)COOH$ amino acid. Alternatively, the Bucherer-Berg method may be used wherein a hydantoin is formed by heating an aldehyde ($R_4CHO$) with ammonium carbonate and potassium cyanide followed by hydrolysis (for example, with barium hydroxide in refluxing dioxane) with acid or base to form the desired Formula XLII $R_4C(NH_2)COOH$ amino add.

Other methods for synthesis of-α-amino adds are also reported in the literature which would permit one skilled in the art to prepare the desired Formula XLII $R_4C(NH_2)COOH$ intermediate necessary for the synthesis of Formula IA compounds.

Suitable methods for the synthesis and/or resolution of Formula XLII compounds are found in reviews by Duthaler (Tetrahedron 1994, 50, 1539-1650), or by Williams (R. M. Williams, Synthesis of optically active amino acids. Pergamon: Oxford, U.K., 1989).

A specific method for the synthesis of a Formula XLII intermediate in either enantiomeric form from the corresponding $R_4X$ (X = Cl, Br, or I) intermediate is the procedure of Pirrung and Krishnamurthy (J. Org. Chem. 1993, 58, 957-958), or by the procedure of O'Donnell, et al. (J. Am. Chem. Soc. 1989, 111, 2353-2355). The required $R_4X$ intermediates are readily prepared by many methods familiar to the chemist skilled in the art For example, those compounds when $R_4X$ is $ArCH_2X$ may be prepared by radical halogenation of the compound $ArCH_3$ or by formulation of the arene Ar-H and conversion of the alcohol to the bromide.

Another specific method for the synthesis of Formula XLII intermediates in either enantiomeric form is that of Corey and Link (J. Am. Chem. Soc. 1992, 114, 1906-1908). Thus, an intermediate of formula $R_4COCCl_3$ is reduced enantiospecifically to intermediate $R_4CH(OH)CCl_3$, which is converted on treatment with azide and base to an intermediate $R_4CH(N_3)COOH$, which is reduced by catalytic hydrogenation to the desired Formula XLII compound. The requisite trichloromethyl ketone $R_4COCCl_3$ is obtained by reaction of the aldehyde $R_4CHO$ with trichloromethide anion followed by oxidation (Gallina and Giordano, Synthesis 1989, 466-468).

A compound of the formula $R_8NH_2$ or $R_9NH_2$ is monoalkylated with a carbonyl compound corresponding to $R_8$ or $R_9$, respectively, under appropriate reductive amination conditions, to give a formula $R_8R_9NH$ amine. To avoid dialkylation, it may be preferable to protect the amines ($R_8NH_2$ or $R_9NH_2$) with a suitable protecting group $P_T$ to give $R_8(P_T)NH$ or $R_9(P_T)NH$, for example by reaction with benzaldehyde and a reducing agent. The protected amines are monoalkylated with a carbonyl compound corresponding to $R_9$ or $R_8$ respectively, under suitable reductive amination conditions, to give $R_8R_9N(P_T)$. The protecting group ($P_T$) is removed (e.g. by exhaustive catalytic hydrogenation when $P_T$ is benzyl) to give a compound of formula $R_8R_9NH$. Appropriate reductive amination conditions are available from the literature to one skilled in the art. These conditions include those reported by Borch et al. (J. Am. Chem. Soc. 1971,2897-2904) and those reviewed by Emerson (Organic Reactions, Wiley: New York, 1948 (14), 174), Hutchins et al. (Org. Prep. Proced. Int 1979 (11), 20, and Lane et al. (Synthesis 1975, 135). Reductive amination conditions favoring N-monoalkylation include those reported by Morales, et al. (Synthetic Communications 1984, 1213-1220) and Verardo et al. (Synthesis 1992 121-125). The $R_8NH_2$ or $R_9NH_2$ amines may also be monoalkylated with $R_9X$ or $R_8X$, respectively, where X is chloride, bromide, tosylate or mesylate. Alternatively, an intermediate of formula $R_8(P_T)NH$ or $R_9(P_T)NH$ may be alkylated with $R_9X$ or $R_8X$, and the protecting group removed to give a compound of formula $R_8R_9NH$.

Additional methods may be used to prepare formula $R_8R_9NH$ amines wherein $R_8$-NH or $R_9$-NH are oxygen-nitrogen linked. Thus a readily available compound of formula ($C_1$-$C_4$)alkoxycarbonyl-NHOH or $NH_2CONHOH$ is dialkylated on nitrogen and oxygen by treatment with base and excess suitable alkylating agent (R-X) to give the corresponding ($C_1$-$C_4$)alkoxycarbonyl-N(R)OR which is then hydrolyzed to give a compound of formula $R_8R_9NH$ (wherein $R_8$=$R_9$=R). Suitable conditions, base, and alkylating agent include those described by Goel and Krolls (Org. Prep. Proced. Int. 1987, 19, 75-78) and Major and Fleck (J. Am. Chem. Soc. 1928, 50, 1479). Alternatively, N-hydroxyurea ($NH_2CONH(OH)$) may be sequentially alkylated, first on oxygen to give $NH_2CONH(OR')$, then on nitrogen to give $NH_2CON(R'')(OR')$, by successive treatment with the alkylating agents R'X and R"X, respectively, in the presence of a suitable base. Suitable base and alkylating agents include those described by Kreutzkamp and Messinger (Chem. Ber. 100, 3463-3465 (1967) and Danen et al (J. Am. Chem. Soc. 1973, 95, 5716-5724). Hydrolysis of these alkylated hydroxyurea derivatives yields the amines $R'ONH_2$ and R'ONHR", which correspond to certain formula $R_8R_9NH$ amines. The chemist skilled in the art can adapt the procedures described in this paragraph to other alkylating agents R, R' and R"-X to prepare other amines of formula $R_8R_9NH$ wherein $R_8$-N or $R_9$-N are oxygen-nitrogen linked. Uno et al (SynLett 1991, 559-560) describe the $BF_3$-catalyzed addition of an organometallic reagent R-Li to an O-alkyl oxime of formula R'CH=N-OR", to give compounds of formula R'RCH-NH(OR"). This route may also be used to give compounds of formula $R_8R_9NH$ wherein one of $R_8$-NH or $R_9$-NH are oxygen-nitrogen linked.

Prodrugs of this invention where a carboxyl group in a carboxylic acid of Formula IA is replaced by an ester may be prepared by combining the carboxylic acid with the appropriate alkyl halide in the presence of a base such as potassium carbonate in an inert solvent such as dimethylformamide at a temperature of about 0 to 100°C for about 1

to about 24 hours. Alternatively the acid is combined with appropriate alcohol as solvent in the presence of a catalytic amount of acid such as concentrated sulfuric add at a temperature of about 20 to 120°C, preferably at reflux, for about 1 hour to about 24 hours. Another method is the reaction of the acid with a stoichiometric amount of the alcohol in the presence of a catalytic amount of acid in an inert solvent such as tetrahydrofuran, with concomitant removal of the water being produced by physical (e.g. Dean-Stark trap) or chemical (e.g. molecular sieves) means.

Prodrugs of this invention where an alcohol function has been derivatized as an ether may be prepared by combining the alcohol with the appropriate alkyl bromide or iodide in the presence of a base such as potassium carbonate in an inert solvent such as dimethylformamide at a temperature of about 0 to 100°C for about 1 to about 24 hours. Alkanoylaminomethyl ethers may be obtained by reaction of the alcohol with a bis-(alkanoylamino)methane in the presence of a catalytic amount of acid in an inert solvent such as tetrahydrofuran, according to a method described in US 4,997, 984. Alternatively, these compounds may be prepared by the methods described by Hoffman et al. in J. Org. Chem. 1994, 59, 3530.

The dialkylphosphate esters may be prepared by reaction of the alcohol with a dialkyl chlorophosphate in the presence of a base in an inert solvent such as tetrahydrofuran. The dihydrogen phosphates may be prepared by reaction of the alcohol with a diaryl or dibenzyl chlorophosphate as described above, followed by hydrolysis or hydrogenation in the presence of a noble metal catalyst, respectively.

Glycosides are prepared by reaction of the alcohol and a carbohydrate in an inert solvent such as toluene in the presence of acid. Typically the water formed in the reaction is removed as it is being formed as described above. An alternate procedure is the reaction of the alcohol with a suitably protected glycosyl halide in the presence of base followed by deprotection.

N-(1-hydroxyalkyl) amides, N-(1-hydroxy-1-(alkoxycarbonyl)methyl) amides or compounds where $R_2$ has been replaced by C(OH)C(O)OY may be prepared by the reaction of the parent amide or indole with the appropriate aldehyde under neutral or basic conditions (e.g. sodium ethoxide in ethanol) at temperatures between 25 and 70°C. N-alkoxymethyl indoles or N-1-(alkoxy)alkyl indoles can be obtained by reaction of the N-unsubstituted indole with the necessary alkyl halide in the presence of a base in an inert solvent. 1-(N,N-dialkylaminomethyl) indole, 1-(1-(N,N-dialkylamino)ethyl) indole and N,N-dialkylaminomethyl amides (e.g. $R_3 = CH_2N(CH_3)_2$) may be prepared by the reaction of the parent N-H compound with the appropriate aldehyde and amine in an alcoholic solvent at 25 to 70°C.

The prodrugs of this invention where $R_2$ and $R_3$ are a common carbon may be prepared by reaction of the parent compound (drug) with benzaldehyde or a ketone or its dimethyl acetal in an inert solvent in the presence of a catalytic amount of acid with concomitant water or methanol removal.

The starting materials and reagents for the above described reaction schemes (e.g., amines, substituted indole carboxylic acids, substituted indoline carboxylic acids, amino acids), although the preparation of most of which are described above, are also readily available or can be easily synthesized by those skilled in the art using conventional methods of organic synthesis. For example, many of the intermediates used herein to prepare compounds of Formula I and IA are, related to, or are derived from amino acids found in nature, in which there is a large scientific interest and commercial need, and accordingly many such intermediates are commercially available or are reported in the literature or are easily prepared from other commonly available substances by methods which are reported in the literature.

Some of the preparation methods useful for the preparation of the compounds described herein (e.g., Formula I compounds, Formula IA compounds) may require protection of remote functionality. The need for such protection will vary depending on the nature of the remote functionality and the conditions of the preparation methods. The need for such protection is readily determined by one skilled in the art. The use of such protection/deprotection methods is also within the skill in the art. For a general description of protecting groups and their use, see T.W. Greene, Protective Groups in Organic Synthesis, John Wiley & Sons, New York, 1991.

Some of the compounds of this invention have asymmetric carbon atoms and therefore are enantiomers or diastereomers. Diasteromeric mixtures can be separated into their individual diastereomers on the basis of their physical chemical differences by methods known per se., for example, by chromatography and/or fractional crystallization. Enantiomers can be separated by converting the enantiomeric mixture into a diasteromeric mixture by reaction with an appropriate optically active compound (e.g., alcohol), separating the diastereomers and converting (e.g., hydrolyzing) the individual diastereomers to the corresponding pure enantiomers. All such isomers, including diastereomers, enantiomers and mixtures thereof are considered as part of this invention. Also, some of the compounds of this invention are atropisomers (e.g., substituted biaryls) and are considered as part of this invention.

Many of the compounds of this invention are acidic and they form a salt with a pharmaceutically acceptable cation. Some of the compounds of this invention are basic and they form a salt with a pharmaceutically acceptable anion. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, when the compounds of this invention form hydrates or solvates they are also within the scope of the invention.

The utility of the combinations of the present invention as medical agents in the treatment of diseases such as are detailed herein in mammals (e.g. humans) is demonstrated by the activity of the compounds of this invention in conventional assays and the in vitro and in vivo assays described below. Such assays also provide a means whereby the activities of the compounds of this invention can be compared with the activities of other known compounds. The results of these comparisons are useful for determining dosage levels in mammals, including humans, for the treatment of such diseases.

**ALDOSE REDUCTASE INHIBITOR ASSAYS**

The activity of an aldose reductase inhibitor can be determined by the amount of aldose reductase inhibitor that is required to lower tissue sorbitol and thus lower tissue fructose according to the following assay.

Male Sprague-Dawley rats are rendered diabetic by injection of streptozocin at 55 mg/kg, i.v., in pH 4.5 citrate buffer. They are fed ad libitum in controlled conditions of housing, temperature and lighting. After five weeks of diabetes, the rats are anesthetized with an overdose of pentobarbital, and tissues are rapidly removed and analyzed for sorbitol and fructose.

Sorbitol levels are analyzed according to the method of Donald M. Eades et al., "Rapid Analysis of Sorbitol, Galactitol, Mannitol and Myoinositol Mixtures From Biological Sources", Journal of Chromatography, 490, 1-8, (1989).

Fructose in rat tissues is enzymatically measured using a modification of the method of Ameyama (Methods in Enzymology, 89.20-29, 1982), in which ferricyanide was replaced by resazurin, a dye that is reduced to the highly fluorescent resorufin. The amount of resorufin fluorescence is stoichiometric with the amount of fructose oxidized by fructose dehydrogenase. The assay contains 0.1 ml neutralized 6% perchloric acid nerve extract in a final volume of 1.5 ml. Following incubation for 60 minutes at room temperature in a dosed drawer, sample fluorescence is determined at excitation = 560 nm, emission =580 nm with slits of 5 mm each in a Perkin-Elmer model 650-40 fluorescence spectrophotometer. Fructose concentrations are calculated by comparison with a series of known fructose standards.

**GLYCOGEN PHOSPHORYLASE INHIBITOR ASSAYS**

The three different purified glycogen phosphorylase (GP) isoenzymes, wherein glycogen phosphorylase is in the activated "a" state (referred to as glycogen phosphorylase a, or the abbreviation GPa), and referred to here as human liver glycogen phosphorylase a (HLGPa), human musde glycogen phosphorylase a (HMGPa), and human brain glycogen phosphorylase a (HBGPa), can be obtained by the following procedures.

Expression and fermentation

The HLGP and HMGP cDNAs are expressed from plasmid pKK233-2 (Pharmacia Biotech. Inc., Piscataway, New Jersey) in *E. coli* strain XL-1 Blue (Stratagene Cloning Systems, LaJolla, CA). The strain is inoculated into LB medium (consisting of 10 g tryptone, 5 g yeast extract, 5 g NaCl, and 1 ml 1N NaOH per liter) plus 100 mg/L ampicillin, 100 mg/L pyridoxine and 600 mg/L $MnCl_2$ and grown at 37°C to a cell density of $OD_{550}$=1.0. At this point, the cells are induced with 1 mM isopropyl-1-thio-ß-D-galactoside (IPTG). Three hours after induction the cells are harvested by centrifugation and cell pellets are frozen at -70°C until needed for purification.

The HBGP cDNA can be expressed by several methodologies, for example, by the method described by Crerar, et al. (J. Biol. Chem. 270:13748-13756). The method described by Crerar, et al. (J. Biol. Chem. 270: 13748-13756) for the expression of HBGP is as follows: the HBGP cDNA can be expressed from plasmid pTACTAC in *E. Coli* strain 25A6. The strain is inoculated into LB medium (consisting of 10 g tryptone, 5 g yeast extract, 5 g NaCl, and 1 ml 1N NaOH per liter) plus 50 mg/L ampicillin and grown overnight, then resuspended in fresh LB medium plus 50 mg/L ampicillin, and reinoculated into a 40X volume of LB/amp media containing 250 µM isopropyl-1-thio-ß-D-galactoside (IPTG), 0.5 mM pyridoxine and 3 mM $MgCl_2$ and grown at 22°C for 48-50 hours. The cells can then be harvested by centrifugation and cell pellets are frozen at -70°C until needed for purification.

The HLGP cDNA is expressed from plasmid pBlueBac III (Invitrogen Corp., San Diego, CA) which is cotransfected with BaculoGold Linear Viral DNA (Pharmingen, San Diego, CA) into Sf9 cells. Recombinant virus is subsequently plaque-purified. For production of protein, Sf9 cells grown in serum-free medium are infected at a multiplicity of infection (moi) of 0.5 and at a cell density of $2x10^6$ cells/ml. After growth for 72 hours at 27°C, cells are centrifuged, and the cell pellets frozen at -70°C until needed for purification. Purification of Glycogen Phosphorylase expressed in *E. coli*

The *E. coli* cells in pellets described above are resuspended in 25 mM ß-glycerophosphate (pH 7.0) with 0.2 mM DTT, 1 mM $MgCl_2$, plus the following protease inhibitors:

| | |
|---|---|
| 0.7 µg/mL | Pepstatin A |
| 0.5 µg/mL | Leupeptin |
| 0.2 mM | phenylmethylsulfonyl fluoride (PMSF), and |
| 0.5 mM | EDTA, |

lysed by pretreatment with 200 µg/mL lysozyme and 3 µg/mL DNAase followed by sonication in 250 mL batches for 5 x 1.5 minutes on ice using a Branson Model 450 ultrasonic cell disrupter (Branson Sonic Power Co., Danbury CT). The *E. coli* cell lysates are then cleared by centrifugation at 35,000 X g for one hour followed by filtration through 0.45 micron filters. GP in the soluble fraction of the lysates (estimated to be less than 1% of the total protein) is purified by monitoring the enzyme activity (as described in GPa Activity Assay section, below) from a series of chromatographic steps detailed below.

Immobilized Metal Affinity Chromatography (IMAC)

This step is based on the method of Luong et al (Luong et al. Journal of Chromatography (1992) 584, 77-84.). 500 mL of the filtered soluble fraction of cell lysates (prepared from approximately 160 - 250 g of original cell pellet) are loaded onto a 130 mL column of IMAC Chelating-Sepharose (Pharmacia LKB Biotechnology, Piscataway, New Jersey) which has been charged with 50 mM $CuCl_2$ and 25 mM ß-glycerophosphate, 250 mM NaCl and 1 mM imidazole at pH 7 equilibration buffer. The column is washed with equilibration buffer until the $A_{280}$ returns to baseline. The sample is then eluted from the column with the same buffer containing 100 mM imidazole to remove the bound GP and other bound proteins. Fractions containing the GP activity are pooled (approximately 600 mL), and ethylenediaminetetraacetic acid (EDTA), DL-dithiothreitol (DTT), phenylmethylsulfonyl fluoride (PMSF), leupeptin and pepstatin A are added to obtain 0.3 mM, 0.2 mM, 0.2 mM, 0.5 µg/mL and 0.7 µg/mL concentrations respectively. The pooled GP is desalted over a Sephadex G-25 column (Sigma Chemical Co., St. Louis, Missouri) equilibrated with 25 mM Tris-HCl (pH 7.3), 3 mM DTT buffer (Buffer A) to remove imidazole and is stored on ice until the second chromatographic step.

5'-AMP-Sepharose Chromatography

The desalted pooled GP sample (approximately 600mL) is next mixed with 70 mL of 5'-AMP Sepharose (Pharmacia LKB Biotechnology, Piscataway, New Jersey) which has been equilibrated with Buffer A (see above). The mixture is gently agitated for one hour at 22°C then packed into a column and washed with Buffer A until the $A_{280}$ returns to baseline. GP and other proteins are eluted from the column with 25 mM Tris-HCl, 0.2 mM DTT and 10 mM adenosine 5'-monophosphate (AMP) at pH 7.3 (Buffer B). GP-containing fractions are pooled following identification by determining enzyme activity (described below) and visualizing the $M_r$ approximately 97 kdal GP protein band by sodium dodecyl sulfate polyacrylamide gel electrophoresis (SDS-PAGE) followed by silver staining (2D-silver Stain II "Daiichi Kit", Daiichi Pure Chemicals Co., LTD., Tokyo, Japan) and then pooled. The pooled GP is dialyzed into 25 mM ß-glycerophosphate, 0.2 mM DTT, 0.3 mM EDTA, 200 mM NaCl, pH 7.0 buffer (Buffer C) and stored on ice until use.

Prior to use of the GP enzyme, the enzyme is converted from the inactive form as expressed in *E. coli* strain XL-1 Blue (designated GPb) (Stragene Cloning Systems, La Jolla, California), to the active form (designated GPa) by the procedure described in Section (A) Activation of GP below.

Purification of Glycogen Phosphorylase expressed in Sf9 cells

The Sf9 cells in pellets described above are resuspended in 25 mM ß-glycerophosphate (pH 7.0) with 0.2 mM DTT, 1 mM $MgCl_2$, plus the following protease inhibitors:

| | |
|---|---|
| 0.7 µg/mL | Pepstatin A |
| 0.5 µg/mL | Leupeptin |
| 0.2 mM | phenylmethylsulfonyl fluoride (PMSF), and |
| 0.5 mM | EDTA, |

lysed by pretreatment with 3 µg/mL DNAase followed by sonication in batches for 3 x 1 minutes on ice using a Branson Model 450 ultrasonic cell disrupter (Branson Sonic Power Co., Danbury CT). The Sf9 cell lysates are then cleared by centrifugation at 35,000 X g for one hour followed by filtration through 0.45 micron filters. GP in the soluble fraction of the lysates (estimated to be 1.5% of the total protein) is purified by monitoring the enzyme activity (as described in

GPa Activity Assay section, below) from a series of chromatographic steps detailed below.

Immobilized Metal Affinity Chromatography (IMAC)

Immobilized Metal Affinity Chromatography is performed as described in the section above. The pooled, desalted GP is then stored on ice until further processed.

Activation of GP

Before further chromatography, the fraction of inactive enzyme as expressed in Sf9 cells (designated GPb) is converted to the active form (designated GPa) by the following procedure described in Section (A) Activation of GP below.

Anion Exchange Chromatography

Following activation of the IMAC purified GPb to GPa by reaction with the immobilized phosphorylase kinase, the pooled GPa fractions are dialyzed against 25 mM Tris-HCl, pH 7.5, containing 0.5 mM DTT, 0.2 mM EDTA, 1.0 mM phenylmethylsulfonyl fluoride (PMSF), 1.0 μg/mL leupeptin and 1.0 μg/mL pepstatin A. The sample is then loaded onto a MonoQ Anion Exchange Chromatography column (Pharmacia Biotech. Inc., Piscataway, New Jersey). The column is washed with equilibration buffer until the $A_{280}$ returns to baseline. The sample is then eluted from the column with a linear gradient of 0-0.25 M NaCl to remove the bound GP and other bound proteins. GP-containing fractions elute between 0.1-0.2 M NaCl range, as detected by monitoring the eluant for peak protein absorbance at $A_{280}$. The GP protein is then identified by visualizing the $M_r$ approximately 97 kdal GP protein band by sodium dodecyl sulfate polyacrylamide gel electrophoresis (SDS-PAGE) followed by silver staining (2D-silver Stain II "Daiichi Kit", Daiichi Pure Chemicals Co., LTD., Tokyo, Japan) and then pooled. The pooled GP is dialyzed into 25 mM N,N-bis[2-Hydroxyethyl]-2-aminoethanesulfonic add, 1.0 mM DTT, 0.5 mM EDTA, 5 mM NaCl, pH 6.8 buffer and stored on ice until use.

Determination of GP Enzyme Activity

A) Activation of GP: Conversion of GPb to GPa

Prior to the determination of GP enzyme activity, the enzyme is converted from the inactive form as expressed in *E. coli* strain XL-1 Blue (designated GPb) (Stragene Cloning Systems, La Jolla, California), to the active form (designated GPa) by phosphorylation of GP using phosphorylase kinase as follows. The fraction of inactive enzyme as expressed in Sf9 cells (designated GPb) is also converted to the active form (designated GPa) by the following procedure.

GP reaction with Immobilized Phosphorylase Kinase

Phosphorylase kinase (Sigma Chemical Company, St. Louis, MO) is immobilized on Affi-Gel 10 (BioRad Corp., Melvile, NY) as per the manufacturer's instructions. In brief, the phosphorylase kinase enzyme (10 mg) is incubated with washed Affi-Gel beads (1 mL) in 2.5 mL of 100 mM HEPES and 80 mM $CaCl_2$ at pH 7.4 for 4 hours at 4°C. The Affi-Gel beads are then washed once with the same buffer prior to blocking with 50 mM HEPES and 1 M glycine methyl ester at pH 8.0 for one hour at room temperature. Blocking buffer is removed and replaced with 50 mM HEPES (pH 7.4), 1 mM β-mercaptoethanol and 0.2% $NaN_3$ for storage. Prior to use to convert GPb to GPa, the Affi-Gel immobilized phosphorylase kinase beads are equilibrated by washing in the buffer used to perform the kinase reaction, consisting of 25 mM ß-glycerophosphate, 0.3 mM DTT, and 0.3mM EDTA at pH 7.8 (kinase assay buffer).

The partially purified, inactive GPb obtained from 5'-AMP-Sepharose chromatography above (from *E. coli*) or the mixture of GPa and GPb obtained from IMAC above (from Sf9 cells) is diluted 1:10 with the kinase assay buffer then mixed with the aforementioned phosphorylase kinase enzyme immobilized on the Affi-Gel beads. NaATP is added to 5 mM and $MgCl_2$ to 6 mM. The resulting mixture is mixed gently at 25°C for 30 to 60 minutes. The sample is removed from the beads and the percent activation of GPb by conversion to GPa is estimated by determining GP enzyme activity in the presence and absence of 3.3 mM AMP. The percentage of total GP enzyme activity due to GPa enzyme activity (AMP-independent) is then calculated as follows:

$$\text{\% of total HLGPa} = \frac{\text{HLGP activity - AMP}}{\text{HLGP activity + AMP}}$$

Altemately, the conversion of GPb to GPa can be monitored by isoelectric focusing, based on the shift in electrophoretic mobility that is noted following conversion of GPb to GPa. GP samples are analyzed by isoelectric focusing (IEF) utilizing the Pharmacia PfastGel System (Pharmacia Biotech. Inc., Piscataway, New Jersey) using precast gels (pl range 4-6.5) and the manufacturer's recommended method. The resolved GPa and GPb bands are then visualized on the gels by silver staining (2D-silver Stain II "Daiichi Kit", Daiichi Pure Chemicals Co., LTD., Tokyo, Japan). Identification of GPa and GPb is made by comparison to *E. coli* derived GPa and GPb standards that are run in parallel on the same gels as the experimental samples.

B) GPa Activity Assay

The disease/condition treating/preventing activities described herein of the glycogen phosphorylase inhibitor compounds of this invention can be indirectly determined by assessing the effect of the compounds of this invention on the activity of the activated form of glycogen phosphorylase (GPa) by one of two methods; glycogen phosphorylase a activity is measured in the forward direction by monitoring the production of glucose-1-phosphate from glycogen or by following the reverse reaction, measuring glycogen synthesis from glucose-1-phosphate by the release of inorganic phosphate. All reactions can be run in triplicate in 96-well microtiter plates and the change in absorbance due to formation of the reaction product is measured at the wavelength specified below in a MCC/340 MKII Elisa Reader (Lab Systems, Finland), connected to a Titertech Microplate Stacker (ICN Biomedical Co, Huntsville, Alabama).
To measure the GPa enzyme activity in the forward direction, the production of glucose-1-phosphate from glycogen is monitored by the multienzyme coupled general method of Pesce et al. [Pesce, M.A., Bodourian, S.H., Harris, R.C. and Nicholson, J.F. (1977) Clinical Chemistry 23, 1711-1717] modified as follows: 1 to 100 μg GPa, 10 units phosphoglucomutase and 15 units glucose-6-phosphate dehydrogenase (Boehringer Mannheim Biochemicals, Indianapolis, IN) are diluted to 1 mL in Buffer A (described hereinafter). Buffer A is at pH 7.2 and contains 50 mM HEPES, 100 mM KCl, 2.5 mM ethyleneglycoltetraacetic acid (EGTA), 2.5 mM $MgCl_2$, 3.5 mM $KH_2PO_4$ and 0.5 mM dithiothreitol. 20 μl of this stock is added to 80 μl of Buffer A containing 0.47 mg/mL glycogen, 9.4 mM glucose, 0.63 mM of the oxidized form of nicotinamide adenine dinucleotide phosphate (NADP+). The compounds to be tested are added as 5 μL of solution in 14% dimethylsulfoxide (DMSO) prior to the addition of the enzymes. The basal rate of GPa enzyme activity in the absence of inhibitors is determined by adding 5 μL of 14% DMSO and a fully-inhibited rate of GPa enzyme activity is obtained by adding 20 μL of 50 mM of the positive control test substance, caffeine. The reaction is followed at room temperature by measuring the conversion of oxidized NADP+ to reduced NADPH at 340 nm.
To measure the GPa enzyme activity in the reverse direction, the conversion of glucose-1-phosphate into glycogen plus inorganic phosphate is measured by the general method described by Engers et al. [Engers, H.D., Shechosky, S. and Madsen, N.B. (1970) Can. J. Biochem. 48, 746-754] modified as follows: 1 to 100 μg GPa is diluted to 1 mL in Buffer B (described hereinafter). Buffer B is at pH 7.2 and contains 50 mM HEPES, 100 mM KCl, 2.5 mM EGTA, 2.5 mM $MgCl_2$ and 0.5 mM dithiothreitol. 20 μL of this stock is added to 80 μL of Buffer B with 1.25 mg/mL glycogen, 9.4 mM glucose, and 0.63 mM glucose-1-phosphate. The compounds to be tested are added as 5 μL of solution in 14% DMSO prior to the addition of the enzyme. The basal rate of GPa enzyme activity in the absence of added inhibitors is determined by adding 5 μL of 14% DMSO and a fully-inhibited rate of GPa enzyme activity is obtained by adding 20 μL of 50 mM caffeine. This mixture is incubated at room temperature for 1 hour and the inorganic phosphate released from the glucose-1-phosphate is measured by the general method of Lanzetta et al. [Lanzetta, P.A., Alvarez, L.J., Reinach, P.S. and Candia, O.A. (1979) Anal. Biochem. 100, 95-97] modified as follows: 150 μL of 10 mg/mL ammonium molybdate, 0.38 mg/mL malachite green in 1 N HCl is added to 100 μL of the enzyme mix. After a 20 minute incubation at room temperature, the absorbance is measured at 620 nm.
The above assays carried out with a range of concentrations of test compound allows the determination of an $IC_{50}$ value (concentration of test compound required for 50% inhibition) for the *in vitro* inhibition of GPa enzyme activity by that test compound.

**INSULIN RESISTANT INDICATION ASSAYS**

The combinations of this invention are readily adapted to clinical use as hypoglycemic agents. The hypoglycemic activity of the combinations of this invention can be determined by the amount of test compound that reduces glucose levels relative to a vehicle without test compound in male ob/ob mice. The test also allows the determination of an approximate minimal effective dose (MED) value for the in vivo reduction of plasma glucose concentration in such mice for such test compounds.
Since the concentration of glucose in blood is closely related to the development of diabetic disorders, these combinations by virtue of their hypoglycemic action, prevent, arrest and/or regress diabetic disorders.
Five to eight week old male C57BL/6J-ob/ob mice (obtained from Jackson Laboratory, Bar Harbor, ME) are housed five per cage under standard animal care practices. After a one week acclimation period, the animals are

weighed and 25 microliters of blood are collected from the retro-orbital sinus prior to any treatment. The blood sample is immediately diluted 1:5 with saline containing 0.025% sodium heparin, and held on ice for metabolite analysis. Animals are assigned to treatment groups so that each group has a similar mean for plasma glucose concentration. After group assignment, animals are dosed orally each day for four days with the vehicle consisting of either: 1)0.25% w/v methyl cellulose in water without pH adjustment; or 2) 0.1% Pluronic® P105 Block Copolymer Surfactant (BASF Corporation, Parsippany, NJ) in 0.1% saline without pH adjustment. On day 5, the animals are weighed again and then dosed orally with the test compound or the vehicle alone. All drugs are administered in vehicle consisting of either: 1) 0.25% w/v methyl cellulose in water without pH adjustment; or 2) 10% DMSO/0.1% Pluronic® P105 (BASF Corporation, Parsippany, NJ) in 0.1% saline without pH adjustment The animals are then bled from the retro-orbital sinus three hours later for determination of blood metabolite levels. The freshly collected samples are centrifuged for two minutes at 10,000 x g at room temperature. The supernatant is analyzed for glucose, for example, by the Abbott VP™ (Abbott Laboratories, Diagnostics Division, Irving, TX) and VP Super System® Autoanalyzer (Abbott Laboratories, Irving, TX), or the Abbott Spectrum CCX ™ Autoanalyzer (Abbott Laboratories, Irving, TX) using the A-Gent™ Glucose-UV Test reagent system (Abbott Laboratories, Irving, TX) (a modification of the method of Richterich and Dauwalder, Schweizerische Medizinische Wochenschrift, 101, 860 (1971)) (hexokinase method) using a 100 mg/dL standard. Plasma glucose is then calculated by the equation:

$$\text{Plasma glucose (mg/dL)} = \text{Sample value} \times 8.14$$

where 8.14 is the dilution factor, adjusted for plasma hematocrit (assuming the hematocrit is 44%).

The animals dosed with vehicle maintain substantially unchanged hyperglycemic glucose levels (e.g., greater than or equal to 250 mg/dL), animals treated with test compounds at suitable doses have significantly depressed glucose levels. Hypoglycemic activity of the test compounds is determined by statistical analysis (unpaired t-test) of the mean plasma glucose concentration between the test compound group and vehicle-treated group on day 5. The above assay carried out with a range of doses of test compounds allows the determination of an approximate minimal effective dose (MED) value for the in vivo reduction of plasma glucose concentration.

The compounds of this invention are readily adapted to clinical use as hyperinsulinemia reversing agents, triglyceride lowering agents and hypocholesterolemic agents. Such activity can be determined by the amount of test compound that reduces insulin, triglycerides or cholesterol levels relative to a control vehicle without test compound in male ob/ob mice.

Since the concentration of cholesterol in blood is closely related to the development of cardiovascular, cerebral vascular or peripheral vascular disorders, the combinations of this invention by virtue of their hypocholesterolemic action, prevent, arrest and/or regress atherosclerosis.

Since the concentration of insulin in blood is related to the promotion of vascular cell growth and increased renal sodium retention, (in addition to the other actions e.g., promotion of glucose utilization) and these functions are known causes of hypertension, the combinations of this invention by virtue of their hypoinsulinemic action, prevent, arrest and/or regress hypertension.

Since the concentration of triglycerides and free fatty acids in blood contributes to the overall levels of blood lipids, the combinations of this invention by virtue of their triglyceride and free fatty add lowering activity prevent, arrest and/or regress hyperlipidemia.

Free fatty acids contribute to the overall level of blood lipids and independently have been negatively correlated with insulin sensitivity in a variety of physiologic and pathologic states.

Five to eight week old male C57BL/6J-ob/ob mice (obtained from Jackson Laboratory, Bar Harbor, ME) are housed five per cage under standard animal care practices and fed standard rodent diet ad libitum. After a one week acclimation period, the animals are weighed and 25 microliters of blood are collected from the retro-orbital sinus prior to any treatment. The blood sample is immediately diluted 1:5 with saline containing 0.025% sodium heparin, and held on ice for plasma glucose analysis. Animals are assigned to treatment groups so that each group has a similar mean for plasma glucose concentration. The compound to be tested is administered by oral gavage as an about 0.02% to 2.0% solution (weight/volume (w/v)) in either 1) 10% DMSO/0.1% Pluronic® P105 Block Copolymer Surfactant (BASF Corporation, Parsippany, NJ) in 0.1% saline without pH adjustment or 2) 0.25% w/v methylcellulose in water without pH adjustment. Single daily dosing (s.i.d.) or twice daily dosing (b.i.d.) is maintained for 1 to for example 15 days. Control mice receive the 10% DMSO/0.1% Pluronic® P105 in 0.1% saline without pH adjustment or the 0.25% w/v methylcellulose in water without pH adjustment only.

Three hours after the last dose is administered, the animals are sacrificed by decapitation and trunk blood is collected into 0.5 mL serum separator tubes containing 3.6 mg of a 1:1 weight/weight sodium fluoride: potassium oxalate mixture. The freshly collected samples are centrifuged for two minutes at 10,000 x g at room temperature, and the serum supernatant is transferred and diluted 1:1 volume/volume with a 1TIU/mL aprotinin solution in 0.1% saline

without pH adjustment

The diluted serum samples are then stored at -80°C until analysis. The thawed, diluted serum samples are analyzed for insulin, triglycerides, free fatty acids, and cholesterol levels. Serum insulin concentration is determined using Equate® RIA INSULIN kits (double antibody method; as specified by the manufacturer) purchased from Binax, South Portland, ME. The inter assay coefficient of variation is ≤ 10%. Serum triglycerides are determined using the Abbott VP™ and VP Super System® Autoanalyzer (Abbott Laboratories, Irving, TX), or the Abbott Spectrum CCX™ (Abbott Laboratories, Irving, TX) using the A-Gent™ Triglycerides Test reagent system (Abbott Laboratories, Diagnostics Division,Irving, TX) (lipase-coupled enzyme method; a modification of the method of Sampson, et al., Clinical Chemistry 21, 1983 (1975)). Serum total cholesterol levels are determined using the Abbott VP™ and VP Super System® Autoanalyzer (Abbott Laboratories, Irving, TX), or the Abbott Spectrum CCX™ (Abbott Laboratories, Irving, TX) and A-Gent™ Cholesterol Test reagent system (cholesterol esterase-coupled enzyme method; a modification of the method of Allain, et al. Clinical Chemistry 20, 470 (1974)) using 100 and 300 mg/dL standards. Serum free fatty add concentration is determined utilizing a kit from Amano International Enzyme Co., Inc., as adapted for use with the Abbott VP™ and VP Super System® Autoanalyzer (Abbott Laboratories, Irving, TX), or the Abbott Spectrum CCX™ (Abbott Laboratories, Irving, TX). Serum insulin, triglycerides, free fatty acids and total cholesterol levels are then calculated by the equations,

$$\text{Serum insulin } (\mu U/mL) = \text{Sample value} \times 2$$

$$\text{Serum triglycerides } (mg/dL) = \text{Sample value} \times 2$$

$$\text{Serum total cholesterol } (mg/dL) = \text{Sample value} \times 2$$

$$\text{Serum free fatty acid } (\mu Eq/L) = \text{Sample value} \times 2$$

where 2 is the dilution factor.

The animals dosed with vehicle maintain substantially unchanged, elevated serum insulin (e.g. 275 μU/mL), serum triglycerides (e.g. 235 mg/dl), serum free fatty acid (e.g., 1500 μEq/mL) and serum total cholesterol (e.g. 190 mg/dL) levels, while animals treated with compounds of this invention generally display reduced serum insulin, triglycerides, free fatty acids and total cholesterol levels. The serum insulin, triglycerides, free fatty acids and total cholesterol lowering activity of the test compounds are determined by statistical analysis (unpaired t-test) of the mean serum insulin, triglycerides, free fatty adds or total cholesterol concentration between the test compound group and the vehicle-treated control group.

Activity in providing protection from ischemia (e.g., damage to heart tissue) for the compounds of this invention can be demonstrated *in vitro* along the lines presented in Butwell et al., Am. J. Physiol., 264, H1884-H1889, 1993 and Allard et al., Am. J. Physio., 1994, 267, H66-H74. Experiments are performed using an isovolumic isolated rat heart preparation, essentially as described in the above-referenced article. Normal male Sprague-Dawley rats, male Sprague-Dawley rats treated to possess cardiac hypertrophy by an aortic banding operation, acutely diabetic male BB/W rats, or non-diabetic BB/W age matched control rats are pretreated with heparin (1000 u, i.p.), followed by pentobarbital (65 mg/kg, i.p.). After deep anesthesia is achieved as determined by the absence of a foot reflex, the heart is rapidly excised and placed into iced saline. The heart is retrogradely perfused through the aorta within 2 minutes. Heart rate and ventricular pressure are determined using a latex balloon in the left ventricle with high pressure tubing connected to a pressure transducer. The heart is perfused with a perfusate solution consisting of (mM) NaCl 118, KCl 4.7, $CaCl_2$ 1.2, $MgCl_2$ 1.2, $NaHCO_3$ 25, glucose 11. The perfusion apparatus is tightly temperature-controlled with heated baths used for the perfusate and for the water jacketing around the perfusion tubing to maintain heart temperature at 37°C. Oxygenation of the perfusate is provided by a pediatric hollow fiber oxygenator (Capiax, Terumo Corp., Tokyo, Japan) immediately proximal to the heart. Hearts are exposed to perfusion solution ± test compound for about 10 minutes or more, followed by 20 minutes of global ischemia and 60 minutes of reperfusion in the absence of the test compound. The heart beats of the control and test compound treated hearts are compared in the period following ischemia. The left ventricular pressure of the control and test compound treated hearts are compared in the period following ischemia. At the end of the experiment, hearts are also perfused and stained to determine the ratio of infarct area relative to the area at risk (%IA/AAR) as described below.

The therapeutic effects of the compounds of this invention in preventing heart tissue damage otherwise resulting from an ischemic insult can also be demonstrated *in vivo* along lines presented in Liu et al., Circulation, Vol.

84, No. 1, (July 1991), as described specifically herein. The *in vivo* assay tests the cardioprotection of the test compound relative to the control group which receives saline vehicle. As background information, it is noted that brief periods of myocardial ischemia followed by coronary artery reperfusion protects the heart from subsequent severe myocardial ischemia (Murry et al., Circulation 74:1124-1136, 1986). Cardioprotection, as indicated by a reduction in infarcted myocardium, can be induced pharmacologically using intravenously administered adenosine receptor agonists in intact, anesthetized rabbits studied as an *in situ* model of myocardial ischemic preconditioning (Liu et al., Circulation 84: 350-356, 1991). The *in vivo* assay tests whether compounds can pharmacologically induce cardioprotection, i.e., reduced myocardial infarct size, when parenterally administered to intact, anesthetized rabbits. The effects of the compounds of this invention can be compared to ischemic preconditioning using the A1 adenosine agonist, $N^6$-1-(phenyl-2R-isopropyl) adenosine (PIA) that has been shown to pharmacologically induce cardioprotection in intact anesthetized rabbits studied *in situ* (Liu et al., Circulation 84:350-356, 1991). The exact methodology is described below.

Surgery: New Zealand White male rabbits (3-4 kg) are anesthetized with sodium pentobarbital (30 mg/kg, i.v.). A tracheotomy is performed via a ventral midline cervical incision and the rabbits are ventilated with 100% oxygen using a positive pressure ventilator. Catheters are placed in the left jugular vein for drug administration and in the left carotid artery for blood pressure measurements. The hearts are then exposed through a left thoracotomy and a snare (00 silk) placed around a prominent branch of the left coronary artery. Ischemia is induced by pulling the snare tight and clamping it in place. Releasing the snare allows the affected area to reperfuse. Myocardial ischemia is evidenced by regional cyanosis; reperfusion is evidenced by reactive hyperemia.

Protocol: Once arterial pressure and heart rate have been stable for at least 30 minutes the experiment is started. Ischemic preconditioning is induced by twice occluding the coronary artery for 5 min followed by a 10 min reperfusion. Pharmacological preconditioning is induced by twice infusing test compound over, for example 5 minutes and allowing 10 minutes before further intervention or by infusing the adenosine agonist, PIA (0.25 mg/kg). Following ischemic preconditioning, pharmacological preconditioning or no conditioning (unconditioned, vehicle control) the artery is occluded for 30 minutes and then reperfused for two hours to induce myocardial infarction. The test compound and PIA are dissolved in saline or other suitable vehicle and delivered at 1 to 5 ml/kg, respectively.

Staining (Liu et al., *Circulation* 84:350-356, 1991): At the end of the 2 hour reperfusion period, the hearts are quickly removed, hung on a Langendorff apparatus, and flushed for 1 minute with normal saline heated to body temperature (38°C). The silk suture used as the snare is then tied tightly to reocclude the artery and a 0.5% suspension of fluorescent particles (1-10 μm) Duke Scientific Corp. (Palo Alto, CA) is infused with the perfusate to stain all of the myocardium except the area at risk (nonfluorescent ventricle). The hearts are then quickly frozen and stored overnight at -20°c. On the following day, the hearts are cut into 2 mm slices and stained with 1% triphenyl tetazolium chloride (TTC). Since TTC reacts with living tissue, this stain differentiates between living (red stained) tissue, and dead tissue (unstained infarcted tissue). The infarcted area (no stain) and the area at risk (no fluorescent particles) are calculated for each slice of left ventricle using a pre-calibrated image analyzer. To normalize the ischemic injury for differences in the area at risk between hearts, the data is expressed as the ratio of infarct area vs. area at risk (%IA/AAR). All data are expressed as Mean±SEM and compared statistically using single factor ANOVA or unpaired t-test. Significance is considered as $p<0.05$.

The invention can be tested for its utility in reducing or preventing ischemic injury in non-cardiac tissues, for example, the brain, or the liver, utilizing procedures reported in the scientific literature. The compounds of this invention can be administered by the preferred route and vehicle of administration and at the preferred time of administration either prior to the ischemic episode, during the ischemic episode, following the ischemic episode (reperfusion period) if included, or during any of the below-mentioned experimental stages.

The benefit of the invention to reduce ischemic brain damage can be demonstrated, for example, in mammals using the method of Park, et al (Ann. Neurol. 1988;24:543-551). In brief, in the procedure of Park, et al. adult male Sprague Dawley rats are anesthetized initially with 2% halothane, and thereafter by mechanical ventilation with a nitrous oxide-oxygen mixture (70%:30%) containing 0.5-1% halothane. A tracheostomy is then performed. The stroke volume of the ventilator is adjusted to maintain arterial carbon dioxide tension at approximately 35 mm Hg and adequate arterial oxygenation ($PaO_2$>90 mm Hg). Body temperature can be monitored by a rectal thermometer, and the animals can be maintained normothermic, if necessary, by external heating. The animals next undergo subtemporal craniectomy to expose the main trunk of the left middle cerebral artery (MCA) under an operating microscope, and the exposed artery is occluded with microbipolar coagulation to generate large ischemic lesions in the cerebral cortex and basal ganglia. After three hours of MCA occlusion, the rats are deeply anesthetized with 2% halothane and a thoracotomy is performed to infuse heparinized saline into the left ventricle. The effluent is collected via an incision of the right atrium. The saline washout is followed by approximately 200 ml of a 40% formaldehyde, glacial acetic acid and absolute methanol solution (FAM; 1:1:8, v/v/v), then the animals are decapitated and the head is stored in the fixative for 24 hours. The brain is then removed, dissected, processed, embedded in paraffin wax, and sectioned (approximately 100 sections per brain). The sections are then stained with hematoxylin-eosin or with a combination of cresyl violet and Luxol fast blue, and examined by light microscopy to identify and quantitate the ischemic damage using an image

analyzer (e.g. the Quantimet 720). The ischemic volumes and areas are expressed in absolute units ($mm^3$ and $mm^2$) and as a percentage of the total region examined. The effect of the compositions and methods of this invention to reduce ischemic brain damage induced by MCA occlusion is noted based on a reduction in the area or volume of relative or absolute ischemic damage in the brain sections from the rats in the treatment group compared to brain sections from rats in a placebo-treated control group.

Other methods which could alternatively be utilized to demonstrate the benefit of the invention to reduce ischemic brain damage include those described by Nakayama, et al. in Neurology 1988;38:1667-1673, Memezawa, et al. in Stroke 1992;23:552-559, Folbergrova, et al. in Proc. Natl. Acad. Sci 1995;92:5057-5059, and Gotti, et al. in Brain Res. 1990;522:290-307.

The benefit of the compositions and methods of this invention to reduce ischemic liver damage can be demonstrated, for example, in mammals using the method of Yokoyama, et al. (Am. J. Physiol. 1990;258:G564-G570). In brief, in the procedure of Yokoyama, et al., fasted adult male Sprague Dawley rats are anesthetized with sodium pentobarbital (40 mg/kg i.p.), then the animals are tracheotomized and mechanically ventilated with room air. The liver is extirpated and placed in an environmental chamber maintained at constant temperature (37 °C), then perfused through the portal vein at a constant pressure of 15 cm $H_2O$ with a modified, hemoglobin-free Krebs-Henseleit buffer (in mM: 118 NaCl, 4.7 KCl, 27 $NaHCO_3$, 2.5 $CaCl_2$, 1.2 $MgSO_4$, 1.2 $KH_2PO_4$, 0.05 EDTA, and 11 mM glucose, plus 300 U heparin). The pH of the perfusate is maintained at 7.4 by gassing the buffer with 95% $O_2$ - 5% $CO_2$. Each liver is perfused at a flow rate of 20 ml/min in a single-pass manner for a 30 min washout and equilibration period (preischemic period), followed by a 2 hour period of global ischemia, and then a 2 hour period of reperfusion under conditions identical to the preischemic period. Aliquots (20 ml) of the perfusate are collected during the preischemic period, immediately after the occlusive ischemic period, and every 30 min of the 2 hour reperfusion period. The perfusate samples are assayed for the appearance of hepatocellular enzymes, for example, aspartate amino-transferase (AST), alanine amino-transferase (ALT), and lactate dehydrogenase (LDH), which are taken to quantitatively reflect the degree of ischemic liver tissue damage during the procedure. AST, ALT, and LDH activities in the perfusate can be determined by several methods, for example, by the reflectometry method using an automatic Kodak Ektachem 500 analyzer reported by Nakano, et al. (Hepatology 1995;22:539-545). The effect of the compositions and methods of this invention in reducing ischemic liver damage induced by occlusion is noted based on a reduction in the release of hepatocellular enzymes immediately following the occlusive period and/or during the postischemic-reperfusion period in the perfused livers from the rats in the treatment group compared to perfused livers from rats in a placebo-treated control group.

Other methods and parameters which could alternatively be utilized to demonstrate the benefit of the compositions and methods of this invention in reducing ischemic liver damage include those described by Nakano, et al. (Hepatology 1995;22:539-545).

Administration of the compounds of this invention can be via any method which delivers a compound of this invention preferentially to the desired tissue (e.g., liver and/or cardiac tissues). These methods include oral routes, parenteral, intraduodenal routes, etc. Generally, the compounds of the present invention are administered in single (e. g., once daily) or multiple doses.

The combinations of this invention are useful in reducing or minimizing damage effected directly to any tissue that may be susceptible to ischemia/reperfusion injury (e.g., heart, brain, lung, kidney, liver, gut, skeletal muscle, retina) as the result of an ischemic event (e.g., myocardial infarction). The active compounds are therefore usefully employed prophylactically to prevent, i.e. (prospectively or prophylactically) to blunt or stem, tissue damage (e.g., myocardial tissue) in patients who are at risk for ischemia (e.g., myocardial ischemia).

Generally, the compounds of this invention are administered orally, but parenteral administration (e.g., intravenous, intramuscular, subcutaneous or intramedullary) may be utilized, for example, where oral administration is inappropriate for the instant target or where the patient is unable to ingest the drug. Topical administration may also be indicated, for example, where the patient is suffering from gastrointestinal disorders or whenever the medication is best applied to the surface of a tissue or organ as determined by the attending physician.

The two different compounds of this invention can be co-administered simultaneously or sequentially in any order, or a single pharmaceutical composition comprising an aldose reductase inhibitor as described above and a glycogen phosphorylase inhibitor as described above in a pharmaceutically acceptable carrier can be administered.

In any event the amount and timing of compounds administered will, of course, be dependent on the subject being treated, on the severity of the affliction, on the manner of administration and on the judgement of the prescribing physician. Thus, because of patient to patient variability, the dosages given below are a guideline and the physician may titrate doses of the compounds to achieve the treatment (e.g., glucose lowering activity; insulin levels) that the physician considers appropriate for the patient. In considering the degree of treatment desired, the physician must balance a variety of factors such as age of the patient, presence of preexisting disease, as well as presence of other diseases (e.g., cardiovascular disease).

Thus, for example, in one mode of administration the combination of this invention may be administered just prior to cardiac surgery (e.g., within twenty-four hours of surgery) where there is risk of myocardial ischemia. In an

alternative exemplary mode, the compounds may be administered subsequent to cardiac surgery (e.g., within twenty-four hours after surgery) where there is risk of myocardial ischemia. The compounds of this invention may also be administered in a chronic daily mode.

In general an amount of a combination of this invention is used that is sufficient to attain an appropriate insulin sensitizing effect.

Alternatively stated, an amount of a combinatin of this invention is used that is sufficient to achieve normal biologic actions of insulin at "normal concentration" which would be evident by maintaining euglycemia, normoglycemia, and normal lipidemia (e.g., triglycerides, cholesterol, free fatty acids) in addition to normotensive and normal glucose tolerance.

An amount of the combination is also used that is effective for ischemic protection.

An amount of the aldose reductase inhibitor of this invention that is effective for the activities of this invention, for example the, triglycerides, and cholesterol lowering activities and hyperinsulinemia reversing activities is used. Typically, an effective dosage for the aldose reductase inhibitors of this invention is in the range of about 0.1 mg/kg/day to 100 mg/kg/day in single or divided doses, preferably 0.1 mg/kg/day to 20 mg/kg/day in single or divided doses.

In general an effective dosage for the activities of this invention, for example the blood glucose, triglycerides, free fatty acids and cholesterol lowering activities and hyperinsulinemia reversing activities of the glycogen phosphorylase inhibitor compounds of this invention is in the range of 0.005 to 50 mg/kg/day, preferably 0.01 to 25 mg/kg/day and most preferably 0.1 to 15 mg/kg/day.

The compounds of the present invention are generally administered in the form of a pharmaceutical composition comprising at least one of the compounds of this invention together with a pharmaceutically acceptable vehicle or diluent. Thus, the compounds of this invention can be administered individually or together in any conventional oral, parenteral, rectal or transdermal dosage form.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easter, Pa., 15th Edition (1975).

Pharmaceutical compositions according to this invention may contain 0.1%-95% of the compound(s) of this invention, preferably 1%-70%. In any event, the composition or formulation to be administered will contain a quantity of a compound(s) according to the invention in an amount effective to treat the disease/condition of the subject being treated.

Since the present invention has an aspect that relates to the treatment of for example, an insulin resistant condition by treatment with a combination of active ingredients which may be administered separately, the invention also relates to combining separate pharmaceutical compositions in kit form. The kit comprises two separate pharmaceutical compositions: an aldose reductase inhibitor and a glycogen phosphorylase inhibitor as described above. The kit comprises a container for containing the separate compositions such as a divided bottle or a divided foil packet. Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the tablets or capsules so specified should be ingested. Another example of such a memory aid is a calendar printed on the card e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also a daily dose of the first compound can consist of one tablet or capsule while a daily dose of the second compound can consist of several tablets or capsules and vice versa. The memory aid should reflect this.

In another specific embodiment of this invention a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The compounds of this invention either alone or in combination with each other or other compounds generally will be administered in a convenient formulation. The following formulation examples only are illustrative and are not intended to limit the scope of the present invention.

In the formulations which follow, "active ingredient" means compound(s) of this invention and thus may refer to an aldose reductase inhibitor, a glycogen phosphorylase inhibitor or a combination of the two.

| Formulation 1: Gelatin Capsules | |
|---|---|
| Hard gelatin capsules are prepared using the following: | |
| Ingredient | Quantity (mg/capsule) |
| Active ingredient | 0.25-100 |
| Starch, NF | 0-650 |
| Starch flowable powder | 0-50 |
| Silicone fluid 350 centistokes | 0-15 |

A tablet formulation is prepared using the ingredients below:

| Formulation 2: Tablets | |
|---|---|
| Ingredient | Quantity (mg/tablet) |
| Active ingredient | 0.25-100 |
| Cellulose, microcrystalline | 200-650 |
| Silicon dioxide, fumed | 10-650 |
| Stearate acid | 5-15 |

The components are blended and compressed to form tablets.

Alternatively, tablets each containing 0.25-100 mg of active ingredients are made up as follows:

| Formulation 3: Tablets | |
|---|---|
| Ingredient | Quantity (mg/tablet) |
| Active ingredient | 0.25-100 |

(continued)

| Formulation 3: Tablets | |
|---|---|
| Ingredient | Quantity (mg/tablet) |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredients, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50° - 60°C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.25-100 mg of active ingredient per 5 ml dose are made as follows:

| Formulation 4: Suspensions | |
|---|---|
| Ingredient | Quantity (mg/5 ml) |
| Active ingredient | 0.25-100 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified Water to | 5 mL |

The active ingredient are passed through a No. 45 mesh U.S. sieve and mixed with the sodium carboxymethyl cellulose and syrup to form smooth paste. The benzoic acid solution, flavor, and color are diluted with some of the water and added, with stirring. Sufficient water is then added to produce the required volume.

An aerosol solution is prepared containing the following ingredients:

| Formulation 5: Aerosol | |
|---|---|
| Ingredient | Quantity (% by weight) |
| Active ingredient | 0.25 |
| Ethanol | 25.75 |
| Propellant 22 (Chlorodifluoromethane) | 70.00 |

The active ingredient is mixed with ethanol and the mixture added to a portion of the propellant 22, cooled to 30°C, and transferred to a filling device. The required amount is then fed to a stainless steel container and diluted with the remaining propellant. The valve units are then fitted to the container.

Suppositories are prepared as follows:

| Formulation 6: Suppositories | |
|---|---|
| Ingredient | Quantity (mg/suppository) |
| Active ingredient | 250 |
| Saturated fatty acid glycerides | 2,000 |

The active ingredient is passed through a No. 60 mesh U.S. sieve and suspended in the saturated fatty acid glycerides previously melted using the minimal necessary heat. The mixture is then poured into a suppository mold of nominal 2 g capacity and allowed to cool.

An intravenous formulation is prepared as follows:

| Formulation 7: Intravenous Solution | |
|---|---|
| Ingredient | Quantity |
| Active ingredient | 20 mg |
| Isotonic saline | 1,000 mL |

The solution of the above ingredients is intravenously administered to a patient at a rate of about 1 mL per minute.

The active ingredient above may also be a combination of agents.

## Claims

**1.** A pharmaceutical composition comprising a therapeutically effective amount of:

a) an aldose reductase inhibitor;
b) a glycogen phosphorylase inhibitor; and
c) a pharmaceutical carrier.

**2.** A pharmaceutical composition for achieving an insulin sensitizing effect in a mammal comprising:

a) an amount of a first compound, said first compound being an aldose reductase inhibitor;
b) an amount of a second compound, said second compound being a glycogen phosphorylase inhibitor; and
c) a pharmaceutically acceptable diluent or carrier wherein the amount of the first compound alone or the amount of the second compound alone is insufficient to achieve the insulin sensitizing effect, and wherein the combined effect of the amounts of the first and second compounds is greater than the sum of the insulin sensitizing effects achievable with the individual amounts of the first and second compounds.

**3.** A pharmaceutical composition as recited in claim 1 or 2 wherein the aldose reductase inhibitor is 1-phthalazineacetic acid, 1-dihydro-4-oxo-3-[[5-trifluoromethyl)-2-benzothiazolyl]methyl]- or a pharmaceutically acceptable salt thereof.

**4.** A pharmaceutical composition as recited in any one of claims 1 to 3 wherein the glycogen phosphorylase inhibitor is:

5-chloro-1H-indole-2-carboxylic acid [(1S)-benzyl-(2R)-hydroxy-3-((3S)-hydroxypyrrolidin-1-yl)-3-oxopropyl]-amide;
5-chloro-1H-indole-2-carboxylic acid [(1S)-benzyl -3-((3S,4S)-dihydroxypyrrolidin-1-yl) -(2R)-hydroxy- 3-oxo-propyl]-amide;
5-chloro-1H-indole-2-carboxylic acid [(1S)- ((R)-hydroxy-dimethylcarbamoyl-methyl)-2-phenyl-ethyl]-amide;
5-chloro-1H-indole-2-carboxylic acid [(1S)- ((R)-hydroxy-methoxy-methyl-carbamoyl)-methyl)-2-phenyl-ethyl]-amide;
5-chloro-1H-indole-2-carboxylic add [(1S)- ((R)-hydroxy-[(2-hydroxy-ethyl)-methyl-carbamoyl]-methyl)-2-phenyl-ethyl]-amide;
5-chloro-1 H-indole-2-carboxylic acid [(1S)-benzyl-2-(3-hydroxyimino-pyrrolidin-1-yl)-2-oxo-ethyl]-amide;
5-chloro-1H-indole-2-carboxylic add [2-(cis-3,4-dihydroxy-pyrrolidin-1-yl)-2-oxo-ethyl]-amide;
5-chloro-1H-indole-2-carboxylic acid [2-((3S,4S)- dihydroxy-pyrrolidin-1-yl)-2-oxo-ethyl]-amide;
5-chloro-1H-indole-2-carboxylic acid [(1S)-benzyl -3-((cis)-dihydroxypyrrolidin-1-yl) -(2R)-hydroxy- 3-oxopropyl]-amide;
5-chloro-1H-indole-2-carboxylic acid [(1S)-benzyl-2-(cis-3,4-dihydroxy-pyrrolidin-1-yl)-2-oxo-ethyl]-amide;
5-chloro-1H-indole-2-carboxylic acid [2-(1,1-dioxo-thiazolidin-3-yl)-2-oxo-ethyl]-amide;
5-chloro-1 H-indole-2-carboxylic acid [(1S)-(4-fluoro-benzyl)-2-(4-hydroxy-piperidin-1-yl)-2-oxo-ethyl]-amide;
5-chloro-1H-indole-2-carboxylic add [(1S)-benzyl-2-((3RS)-hydroxy-piperidin-1-yl)-2-oxo-ethyl]-amide;
5-chloro-1H-indole-2-carboxylic acid [2-oxo-2-((1RS)-oxo-thiazolidin-3-yl)-ethyl]-amide; or
5-chloro-1H-indole-2-carboxylic acid [(1S)-benzyl-2-(3-hydroxy-azetidin-1-yl)-2-oxo-ethyl]-amide.

**5.** A pharmaceutical composition as recited in any one of claims 1 to 4 wherein the amount of aldose reductase inhibitor is about 0.1 mg/kg to about 20 mg/kg.

**6.** A pharmaceutical composition as recited in any one of claims 1 to 5 wherein the amount of glycogen phosphorylase inhibitor is about 0.1 mg/kg to about 15 mg/kg.

**7.** Use of :

a) an aldose reductase inhibitor; and
b) a glycogen phosphorylase inhibitor in the manufacture of a medicament for treating a mammal having an insulin resistant condition.

**8.** The use as claimed in claim 7 wherein the insulin resistant condition is diabetes, hyperinsulinemia, impaired glucose tolerance, hyperglycemia and/or hyperlipidemia following meals, type II diabetes, altered body composition, reduction of lean body mass, obesity, hypertension, dyslipidemia, atherosclerosis, tissue ischemia, cardiovascular diseases, syndrome X, pregnancy, conditions of infection, uremia, hyperandrogenism, hypercortisolemia or other conditions of adrenocortical hormone excess, acromegaly, growth hormone excess, or polycystic ovarian disease.

**9.** Use of:

a) an aldose reductase inhibitor; and
b) a glycogen phosphorylase inhibitor in the manufacture of a medicament for achieving an insulin sensitizing effect in a mammal having an insulin resistant condition, wherein the amount of the first compound alone and the amount of the second compound alone is insufficient to achieve said insulin sensitizing effect and wherein the combined effect of the amounts of the first and second compounds is greater than the sum of the insulin sensitizing effects achievable with the individual amounts of the first and second compound.

**10.** Use of:

a) an aldose reductase inhibitor; and
b) a glycogen phosphorylase inhibitor in the manufacture of a medicament for reducing tissue damage resulting from ischemia.

**11.** The use as claimed in claim 10 wherein the tissue is cardiac, brain, liver, kidney, lung, gut, skeletal muscle, spleen, pancreas, nerve, spinal cord, retina tissue, the vasculature, or intestinal tissue.

**12.** The use as claimed in any one of claims 7 to 11 wherein the aldose reductase inhibitor is 1-phthalazineacetic acid, 3,4-dihydro-4-oxo-3-[[5-trifluoromethyl)-2-benzothiazolyl]methyl]- or a pharmaceutically acceptable salt thereof.

**13.** The use as claimed in any one of claims 7 to 12 wherein the glycogen phosphorylase inhibitor is:

5-chloro-1H-indole-2-carboxylic acid [(1S)-benzyl-(2R)-hydroxy-3-((3S)-hydroxypyrrolidin-1-yl)-3-oxopropyl]-amide;
5-chloro-1H-indole-2-carboxylic acid [(1S)-benzyl-3-((3S,4S)-dihydroxypyrrolidin-1-yl) -(2R)-hydroxy- 3-oxopropyl]-amide;
5-chloro-1H-indole-2-carboxylic acid [(1S)- ((R)-hydroxy-dimethylcarbamoyl-methyl)-2-phenyl-ethyl]-amide;
5-chloro-1H-indole-2-carboxylic acid [(1S)- ((R)-hydroxy-methoxy-methyl-carbamoyl)-methyl)-2-phenyl-ethyl]-amide;
5-chloro-1H-indole-2-carboxylic acid [(1S)- ((R)-hydroxy-[(2-hydroxy-ethyl)-methyl-carbamoyl]-methyl)-2-phenyl-ethyl]-amide;
5-chloro-1H-indole-2-carboxylic acid [(1S)-berzyl-2-(3-hydroxyimino-pyrrolidin-1-yl)-2-oxo-ethyl]-amide;
5-chloro-1H-indole-2-carboxylic acid [2-(cis-3,4-dihydroxy-pyrrolidin-1-yl)-2-oxo-ethyl]-amide;
5-chloro-1H-indole-2-carboxylic acid [2-((3S,4S)- dihydroxy-pyrrolidin-1-yl)-2-oxo-ethyl]-amide;
5-chloro-1 H-indole-2-carboxylic acid [(1S)-benzyl-3-((cis)-dihydroxypyrrolidin-1-yl) -(2R)-hydroxy- 3-oxopropyl]-amide;
5-chloro-1H-indole-2-carboxylic acid [(1S)-benzyl-2-(cis-3,4-dihydroxy-pyrrolidin-1-yl)-2-oxo-ethyl]-amide;
5-chloro-1H-indole-2-carboxylic acid [2-(1,1-dioxo-thiazolidin-3-yl)-2-oxo-ethyl]-amide;
5-chloro-1H-indole-2-carboxylic acid [(1S)-(4-fluoro-benzyl)-2-(4-hydroxy-piperidin-1-yl)-2-oxo-ethyl]-amide;

5-chloro-1H-indole-2-carboxylic acid [(1S)-benzyl-2-((3RS)-hydroxy-piperidin-1-yl)-2-oxo-ethyl]-amide;
5-chloro-1H-indole-2-carboxylic acid [2-oxo-2-((1RS)-oxo-thiazolidin-3-yl)-ethyl]-amide; or
5-chloro-1H-indole-2-carboxylic acid [(1S)-benzyl-2-(3-hydroxy-azetidin-1-yl)-2-oxo-ethyl]-amide.

**14.** The use as recited in any one of claims 7 to 13 wherein the amount of aldose reductase inhibitor is about 0.1 mg/kg to about 20 mg/kg and the amount of glycogen phosphorylase inhibitor is about 0.1 mg/kg to about 15 mg/kg.

**15.** The use as recited in any one of claims 7 to 13 wherein the dose of aldose reductase inhibitor is about 0.1 mg/kg/day to about 20 mg/kg/day and the dose of glycogen phosphorylase inhibitor is about 0.1 mg/kg/day to about 15 mg/kg/day.

**16.** A kit comprising:

a. a therapeutically effective amount of an aldose reductase inhibitor and a pharmaceutically acceptable carrier in a first unit dosage form;
b. a therapeutically effective amount of a glycogen phosphorylase inhibitor and a pharmaceutically acceptable carrier in a second unit dosage form; and
c. container means for containing said first and second dosage forms.

**Patentansprüche**

**1.** Pharmazeutische Zusammensetzung, umfassend eine therapeutisch wirksame Menge von:

a) einem Aldosereductase-Inhibitor;
b) einem Glycogenphosphorylase-Inhibitor und
c) einem pharmazeutischen Träger.

**2.** Pharmazeutische Zusammensetzung zum Erreichen einer insulinsensibilisierenden Wirkung bei einem Säuger, umfassend:

a) eine Menge einer ersten Verbindung, wobei die erste Verbindung ein Aldosereductase-Inhibitor ist;
b) eine Menge einer zweiten Verbindung, wobei die zweite Verbindung ein Glycogenphosphorylase-Inhibitor ist und
c) ein pharmazeutisch verträgliches Verdünnungsmittel oder einen pharmazeutisch verträglichen Träger,

wobei die Menge der ersten Verbindung allein oder die Menge der zweiten Verbindung allein nicht ausreichend ist, um die insulinsensibilisierende Wirkung zu erreichen, und wobei die kombinierte Wirkung der Mengen der ersten und zweiten Verbindung größer ist als die Summe der insulinsensibilisierenden Wirkungen, die mit den jeweiligen Mengen der ersten und zweiten Verbindung erreichbar ist.

**3.** Pharmazeutische Zusammensetzung nach Anspruch 1 oder 2, wobei der Aldosereductase-Inhibitor 3,4-Dihydro-4-oxo-3-[[5-(trifluormethyl)-2-benzothiazolyl]methyl]-1-phthalazinessigsäure oder ein pharmazeutisch verträgliches Salz davon ist.

**4.** Pharmazeutische Zusammensetzung nach einem der Ansprüche 1 bis 3, wobei der Glycogenphosphorylase-Inhibitor ist:

5-Chlor-1H-indol-2-carbonsäure[(1S)-benzyl-(2R)-hydroxy-3-((3S)-hydroxypyrrolidin-1-yl)-3-oxopropyl]-amid;
5-Chlor-1H-indol-2-carbonsäure[(1S)-benzyl-3-((3S,4S)-dihydroxypyrrolidin-1-yl)-(2R)-hydroxy-3-oxopropyl]-amid;
5-Chlor-1H-indol-2-carbonsäure[(1S)-((R)-hydroxydimethylcarbamoyl-methyl)-2-phenyl-ethyl]-amid;
5-Chlor-1H-indol-2-carbonsäure[(1S)-((R)-hydroxy-methoxy-methyl-carbamoyl)-methyl)-2-phenyl-ethyl]-amid;
5-Chlor-1H-indol-2-carbonsäure[(1S)-((R)-hydroxy-[(2-hydroxy-ethyl)-methyl-carbamoyl]-methyl)-2-phenyl-ethyl]-amid;
5-Chlor-1H-indol-2-carbonsäure[(1S)-benzyl-2-(3-hydroxyimino-pyrrolidin-1-yl)-2-oxo-ethyl]-amid;

5-Chlor-1H-indol-2-carbonsäure[2-(cis-3,4-dihydroxy-pyrrolidin-1-yl)-2-oxo-ethyl]-amid;
5-Chlor-1H-indol-2-carbonsäure[2-((3S,4S)-dihydroxy-pyrrolidin-1-yl)-2-oxo-ethyl]-amid;
5-Chlor-1H-indol-2-carbonsäure[(1S)-benzyl-3-((cis)-dihydroxypyrrolidin-1-yl)-(2R)-hydroxy-3-oxopropyl]-amid;
5-Chlor-1H-indol-2-carbonsäure[(1S)-benzyl-2-(cis-3,4-dihydroxy-pyrrolidin-1-yl)-2-oxo-ethyl]-amid;
5-Chlor-1H-indol-2-carbonsäure[2-(1,1-dioxo-thiazolidin-3-yl)-2-oxo-ethyl]amid;
5-Chlor-1H-indol-2-carbonsäure[(1S)-(4-fluor-benzyl)-2-(4-hydroxy-piperidin-1-yl)-2-oxo-ethyl]-amid;
5-Chlor-1H-indol-2-carbonsäure[(1S)-benzyl-2-((3RS)-hydroxy-piperidin-1-yl)-2-oxo-ethyl]-amid;
5-Chlor-1H-indol-2-carbonsäure[2-oxo-2-((1RS)-oxo-thiazolidin-3-yl)-ethyl]-amid oder
5-Chlor-1H-indol-2-carbonsäure[(1S)-benzyl-2-(3-hydroxy-azetidin-1-yl)-2-oxo-ethyl]amid.

**5.** Pharmazeutische Zusammensetzung nach einem der Ansprüche 1 bis 4, wobei die Menge an Aldosereductase-Inhibitor etwa 0,1 mg/kg bis etwa 20 mg/kg ist.

**6.** Pharmazeutische Zusammensetzung nach einem der Ansprüche 1 bis 5, wobei die Menge an Glycogenphosphorylase-Inhibitor etwa 0,1 mg/kg bis etwa 15 mg/kg ist.

**7.** Verwendung von:

a) einem Aldosereductase-Inhibitor und
b) einem Glycogenphosphorylase-Inhibitor

bei der Herstellung eines Arzneimittels zur Behandlung eines Säugers, der einen insulinresistenten Zustand aufweist.

**8.** Verwendung nach Anspruch 7, wobei der insulinresistente Zustand Diabetes, Hyperinsulinämie, beeinträchtigte Glucosetoleranz, Hyperglycämie und/oder Hyperlipidämie nach Essen, Diabetes Typ-II, veränderte Körperzusammensetzung, Verminderung der Magerkörpermasse, Fettleibigkeit, Hypertension, Dyslipidämie, Arteriosklerose, Gewebsischämie, cardiovaskuläre Erkrankungen, Syndrom X, Schwangerschaft, Infektionszustände, Urämie, Hyperandrogenismus, Hypercortisolämie oder andere Zustände von adrenocorticalem Hormonüberschuss, Acromegalie, Wachstumshormonüberschuss oder polyzystische Eierstockerkrankung ist.

**9.** Verwendung von

a) einem Aldosereductase-Inhibitor und
b) einem Glycogenphosphorylase-Inhibitor

bei der Herstellung eines Arzneimittels zum Erreichen einer insulinsensibilisierenden Wirkung bei einem Säuger mit einem insulinresistenten Zustand,
wobei die Menge der ersten Verbindung allein oder die Menge der zweiten Verbindung allein nicht ausreichend ist, um die insulinsensibilisierende Wirkung zu erreichen, und wobei die kombinierte Wirkung der Mengen der ersten und zweiten Verbindung größer ist als die Summe der insulinsensibilisierenden Wirkungen, die mit den jeweiligen Mengen der ersten und zweiten Verbindung erreichbar ist.

**10.** Verwendung von

a) einem Aldosereductase-Inhibitor und
b) einem Glycogenphosphorylase-Inhibitor

bei der Herstellung eines Arzneimittels zur Verminderung von Gewebsschädigung, die von Ischämie herrührt.

**11.** Verwendung nach Anspruch 10, wobei das Gewebe Herz-, Hirn-, Leber-, Nieren-, Lungen-, Darm-, Skelettmuskel-, Milz-, Pankreas-, Nerven-, Wirbelsäulen-, Netzhautgewebe, die Vaskulatur oder Intestinalgewebe ist.

**12.** Verwendung nach einem der Ansprüche 7 bis 11, wobei der Aldosereductase-Inhibitor 3,4-Dihydro-4-oxo-3-[[5-(trifluormethyl)-2-benzothiazolyl]methyl]-1-phthalazinessigsäure, oder ein pharmazeutisch verträgliches Salz davon ist.

13. Verwendung nach einem der Ansprüche 7 bis 12, wobei der Glycogenphosphorylase-Inhibitor ist:

5-Chlor-1H-indol-2-carbonsäure[(1S)-benzyl-(2R)-hydroxy-3-((3S)-hydroxypyrrolidin-1-yl)-3-oxopropyl]-amid;
5-Chlor-1H-indol-2-carbonsäure[(1S)-benzyl-3-((3S,4S)-dihydroxypyrrolidin-1-yl)-(2R)-hydroxy-3-oxopropyl]-amid;
5-Chlor-lH-indol-2-carbonsäure[(1S)-((R)-hydroxydimethylcarbamoyl-methyl)-2-phenyl-ethyl]-amid;
5-Chlor-lH-indol-2-carbonsäure[(1S)-((R)-hydroxy-methoxy-methyl-carbamoyl)-methyl)-2-phenyl-ethyl]-amid;
5-Chlor-1H-indol-2-carbonsäure[(1S)-((R)-hydroxy-[(2-hydroxy-ethyl)-methyl-carbamoyl]-methyl)-2-phenyl-ethyl]-amid;
5-Chlor-1H-indol-2-carbonsäure[(1S)-benzyl-2-(3-hydroxyimino-pyrrolidin-1-yl)-2-oxo-ethyl]-amid;
5-Chlor-1H-indol-2-carbonsäure[2-(cis-3,4-dihydroxypyrrolidin-1-yl)-2-oxo-ethyl]-amid;
5-Chlor-1H-indol-2-carbonsäure[2-((3S,4S)-dihydroxypyrrolidin-1-yl)-2-oxo-ethyl]-amid;
5-Chlor-1H-indol-2-carbonsäure[(1S)-benzyl-3-((cis)-dihydroxypyrrolidin-1-yl)-(2R)-hydroxy-3-oxopropyl]-amid;
5-Chlor-1H-indol-2-carbonsäure[(1S)-benzyl-2-(cis-3,4-dihydroxy-pyrrolidin-1-yl)-2-oxo-ethyl]-amid;
5-Chlor-1H-indol-2-carbonsäure[2-(1,1-dioxo-thiazolidin-3-yl)-2-oxo-ethyl]amid;
5-Chlor-lH-indol-2-carbonsäure[(1S)-(4-fluor-benzyl)-2-(4-hydroxy-piperidin-1-yl)-2-oxo-ethyl]-amid;
5-Chlor-1H-indol-2-carbonsäure[(1S)-benzyl-2-((3RS)-hydroxy-piperidin-1-yl)-2-oxo-ethyl]-amid;
5-Chlor-1H-indol-2-carbonsäure[2-oxo-2-((1RS)-oxo-thiazolidin-3-yl)-ethyl]-amid oder
5-Chlor-1H-indol-2-carbonsäure[(1S)-benzyl-2-(3-hydroxy-azetidin-1-yl)-2-oxo-ethyl]amid.

14. Verwendung nach einem der Ansprüche 7 bis 13, wobei die Menge an Aldosereductase-Inhibitor etwa 0,1 mg/kg bis etwa 20 mg/kg ist und die Menge an Glycogenphosphorylase-Inhibitor etwa 0,1 mg/kg bis etwa 15 mg/kg ist.

15. Verwendung nach einem der Ansprüche 7 bis 13, wobei die Dosis an Aldosereductase-Inhibitor etwa 0,1 mg/kg/Tag bis etwa 20 mg/kg/Tag ist und die Dosis an Glycogenphosphorylase-Inhibitor etwa 0,1 mg/kg/Tag bis etwa 15 mg/kg/Tag ist.

16. Kit, umfassend:

a. eine therapeutisch wirksame Menge eines Aldosereductase-Inhibitors und einen pharmazeutisch verträglichen Träger in einer ersten Dosierungseinheitsform;
b. eine therapeutisch wirksame Menge eines Glycogenphosphorylase-Inhibitors und einen pharmazeutisch verträglichen Träger in einer zweiten Dosierungseinheitsform und
c. Behältervorrichtung zum Enthalten der ersten und zweiten Dosierungsformen.

## Revendications

1. Composition pharmaceutique, comprenant une quantité thérapeutiquement efficace :

a. d'un inhibiteur d'aldose-réductase ;
b. d'un inhibiteur de glycogène-phosphorylase; et
c. un support pharmaceutique.

2. Composition pharmaceutique destinée à obtenir un effet de sensibilisation à l'insuline chez un mammifère, comprenant :

a. une quantité d'un premier composé, ledit premier composé étant un inihibiteur d'aldose-réductase ;
b. une quantité d'un second composé, ledit second composé étant un inhibiteur de glycogène-phosphorylase; et
c. un diluant ou support pharmaceutiquement acceptable,

dans laquelle la quantité du premier composé seul ou la quantité du second composé seul est insuffisante pour obtenir l'effet de sensibilisation à l'insuline, et l'effet total des quantités des premier et second composés est supérieur à la somme des effets de sensibilisation à l'insuline pouvant être obtenus avec les quantités distinctes des

premier et second composés.

3. Composition pharmaceutique suivant la revendication 1 ou 2, dans laquelle l'inhibiteur d'aldose-réductase est le composé consistant en l'acide 1-phtalazine-acétique, 3,4-dihydro-4-oxo-3-[[5-trifluoro-méthyl)-2-benzothiazolyl]-méthyl]-, ou un de ses sels pharma-ceutiquement acceptables.

4. Composition pharmaceutique suivant l'une quelconque des revendications 1 à 3, dans laquelle l'inhibiteur de glycogène-phosphorylase est :

le [(1S)-benzyl)-(2R)-hydroxy-3-((3S)-hydroxy-pyrrolidine-1-yl)-3-oxo-propyl]-amide d'acide 5-chloro-1H-indole-2-carboxylique.
le [(1S)-benzyl-((3S,4S)-dihydroxy-pyrrolidine-1-yl)-(2R)-hydroxy-3-oxo-propyl]-amide d'acide 5-chloro-1H-indole-2-carboxylique,
le [(lS)-((R)-hydroxydiméthylcarbamoyl-méthyl)-2-phényl-éthyl]-amide d'acide 5-chloro-1H-indole-2-carboxylique,
le [(1S)-((R)-hydroxyméthoxy-méthyl-carbamoyl)-méthyl)-2-phényléthyl]-amide d'acide 5-chloro-1H-indole-2-carboxylique,
le [(1S)-((R)-hydroxy-[(2-hydroxyéthyl)-méthyl-carbamoyl]-méthyl)-2-phényléthyl]-amide d'acide 5-chloro-1H-indole-2-carboxylique,
le [(1S)-benzyl-2-(3-hydroxyimino-pyrrolidine-1-yl)-2-oxo-éthyl]-amide d'acide 5-chloro-lH-indole-2-carboxylique,
le [2-(cis-3,4-dihydroxy-pyrrolidine-1-yl)-2-oxo-éthyl]-amide d'acide 5-chloro-1H-indole-2-carboxylique,
le [2-((3S,4S)-dihydroxy-pyrrolidine-1-yl)-2-oxo-éthyl]-amide d'acide 5-chloro-1H-indole-2-carboxylique,
le [(1S)-benzyl-3-((cis)-dihydroxypyrrolidine-1-yl)-(2R)-hydroxy-3-oxopropyl]-amide d'acide 5-chloro-1H-indole-2-carboxylique,
le [(1S)-benzyl-2-(cis-3,4-dihydroxy-pyrroli-dine-1-yl)-2-oxo-éthyl]-amide d'acide 5-chloro-1H-indole-2-carboxylique,
le [2-(1,1-dioxo-thiazolidine-3-yl)-2-oxo-éthyl]-amide d'acide 5-chloro-1H-indole-2-carboxylique,
le [(1S)-(4-fluorobenzyl)-2-(4-hydroxy-pipéridine-1-yl)-2-oxo-éthyl]-amide d'acide 5-chloro-1H-indole-2-carboxylique,
le [(lS)-benzyl-2((3RS)-hydroxy-pipéridine-1-yl)-2-oxo-éthyl]-amide d'acide 5-chloro-lH-indole-2-carboxy-lique,
le [2-oxo-2-((1RS)-oxo-1-thiazolidine-3-yl)-éthyl]-amide d'acide 5-chloro-1H-indole-2-carboxylique,
le [(lS)-benzyl-2-(3-hydroxy-azétidine-1-yl)-2-oxo-éthyl]-amide d'acide 5-chloro-1H-indole-2-carboxylique.

5. Composition pharmaceutique suivant l'une quelconque des revendications 1 à 4, dans laquelle la quantité d'inhibiteur d'aldose-réductase est comprise dans l'intervalle d'environ 0,1 mg/kg à environ 20 mg/kg.

6. Composition pharmaceutique suivant l'une quelconque des revendications 1 à 5, dans laquelle la quantité d'inhibiteur de glycogène-phosphorylase est comprise dans l'intervalle d'environ 0,1 mg/kg à environ 15 mg/kg.

7. Utilisation :

a. d'un inhibiteur d'aldose-réductase ; et
b. d'un inhibiteur de glycogène-phosphorylase,

dans la préparation d'un médicament pour le traitement d'un mammifère présentant un état de résistance à l'insuline.

8. Utilisation selon la revendication 7, dans laquelle l'état de résistance à l'insuline est le diabète, l'hyperinsulinémie, une tolérance entravée au glucose, l'hyperglycémie et/ou l'hyperlipidémie après absorption de nourriture, le diabète de type II, une modification de la composition de l'organisme, une réduction de la masse maigre de l'organisme, l'obésité, l'hypertension, une dyslipidémie, l'athérosclérose, une ischémie tissulaire, des maladies cardiovasculaires, le syndrome X, la gravidité, des états infectieux, une urémie, un hyperandrogénisme, une hypercortisolémie ou d'autres états comportant un excès d'hormones cortico-surrénales, une acromégalie, un excès d'hormone de croissance ou le syndrome d'ovaires polykystiques.

9. Utilisation :

a. d'un inhibiteur d'aldose-réductase ; et
b. d'un inhibiteur de glycogène-phosphorylase,

dans la préparation d'un médicament pour obtenir un effet de sensibilisation à l'insuline chez un mammifère qui présente un état de résistance à l'insuline, la quantité du premier composé seul et la quantité du second composé seul étant insuffisantes pour obtenir ledit effet de sensibilisation à l'insuline et l'effet total des quantités des premier et second composés étant supérieur à la somme des effets de sensibilisation à l'insuline pouvant être obtenus avec les quantités distinctes des premier et second composés.

**10.** Utilisation :

a. d'un inhibiteur d'aldose-réductase ; et
b. d'un inhibiteur de glycogène-phosphorylase,

dans la préparation d'un médicament pour réduire l'altération tissulaire résultant d'une ischémie.

**11.** Utilisation suivant la revendication 10, dans laquelle le tissu est le tissu du coeur, -du cerveau, du foie, du rein, du poumon, du tube digestif, des muscles squelettiques, de la rate, du pancréas, des nerfs, de la moelle épinière, de la rétine, du système vasculaire ou de l'intestin.

**12.** Utilisation suivant l'une quelconque des revendications 7 à 11, dans laquelle l'inhibiteur d'aldose-réductase est l'acide 1-phtalazine-acétique, 3,4-dihydro-4-oxo-3-[[5-trifluorométhyl)-2-benzothiazolyl]méthyl]-, ou un de ses sels pharmaceutiquement acceptables.

**13.** Utilisation suivant l'une quelconque des revendications 7 à 12, dans laquelle l'inhibiteur de glycogène-phosphorylase est :

le [(1S)-benzyl)-(2R)-hydroxy-3-((3S)-hydroxy-pyrrolidine-1-yl)-3-oxo-propyl]-amide d'acide 5-chloro-1H-indole-2-carboxylique,
le [(1S)-benzyl-((3S,4S)-dihydroxy-pyrrolidine-1-yl)-(2R)-hydroxy-3-oxo-propyl]-amide d'acide 5-chloro-1H-indole-2-carboxylique,
le [(1S)-((R)-hydroxydiméthylcarbamoyl-méthyl)-2-phényl-éthyl]-amide d'acide 5-chloro-1H-indole-2-carboxylique,
le [(IS)-((R)-hydroxyméthoxy-méthyl-carbamoyl)-méthyl)-2-phényléthyl]-amide d'acide 5-chloro-1H-indole-2-carboxylique,
le [(1S)-((R)-hydroxy-[(2-hydroxyéthyl)-méthyl-carbamoyl]-méthyl)-2-phényléthyl]-amide d'acide 5-chloro-1H-indole-2-carboxylique,
le [(1S)-benzyl-2-(3-hydroxyimino-pyrrolidine-1-yl)-2-oxo-éthyl]-amide d'acide 5-chloro-1H-indole-2-carboxylique,
le [2-(cis-3,4-dihydroxy-pyrrolidine-1-yl)-2-oxo-éthyl]-amide d'acide 5-chloro-1H-indole-2-carboxylique,
le [2-((3S,4S)-dihydroxy-pyrrolidine-1-yl)-2-oxo-éthyl]-amide d'acide 5-chloro-1H-indole-2-carboxylique,
le [(IS)-benzyl-2-(cis-3,4-dihydroxy-pyrro-lidine-1-yl)-2-oxo-éthyl]-amide d'acide 5-chloro-lH-indole-2-carboxylique,
le [2-(1,1-dioxo-thiazolidine-3-yl)-2-oxo-éthyl]-amide d'acide 5-chloro-lH-indole-2-carboxylique,
le [(IS)-benzyl-3-((cis)-dihydroxypyrrolidine-1-yl)-(2R)-hydroxy-3-oxopropyl]-amide d'acide 5-chloro-1H-indole-2-carboxylique,
le [(1S)-(4-fluorobenzyl)-2-(4-hydroxy-pipéri-dine-1-yl)-2-oxo-éthyl]-amide d'acide 5-chloro-1H-indole-2-carboxylique,
le [(IS)-benzyl-2-((3RS)-hydroxy-pipéridine-1-yl)-2-oxo-éthyl]-amide d'acide 5-chloro-lH-indole-2-carboxy-lique,
le [2-oxo-2-((1RS)-oxo-thiazolidine-3-yl)-éthyl]-amide d'acide 5-chloro-1H-indole-2-carboxylique, ou
le [(IS)-benzyl-2-(3-hydroxy-azétidine-1-yl)-2-oxo-éthyl]-amide d'acide 5-chloro-1H-indole-2-carboxylique.

**14.** Utilisation suivant l'une quelconque des revendications 7 à 13, dans laquelle la quantité d'inhibiteur d'aldose-réductase est comprise dans l'intervalle d'environ 0,1 mg/kg à environ 20 mg/kg, et la quantité d'inhibiteur de glycogène-phosphorylase est comprise dans l'intervalle d'environ 0,1 mg/kg à environ 15 mg/kg.

**15.** Utilisation suivant l'une quelconque des revendications 7 à 13, dans laquelle la dose d'inhibiteur d'aldose-réductase

est comprise dans l'intervalle d'environ 0,1 mg/kg/jour à environ 20 mg/kg/jour et la dose d'inhibiteur de glycogène-phosphorylase est comprise dans l'intervalle d'environ 0,1 mg/kg/jour à environ 15 mg/kg/jour.

**16.** Kit comprenant :

a. une quantité thérapeutiquement efficace d'un inhibiteur d'aldose-réductase et un support pharmaceutiquement acceptable dans une première forme posologique unitaire ;
b. une quantité thérapeutiquement efficace d'un inhibiteur de glycogène-phosphorylase et un support pharmaceutiquement acceptable dans une seconde forme posologique unitaire ; et
c. un récipient destiné à renfermer lesdites première et seconde formes posologiques.